(19) Europäisches Patentamt / European Patent Office / Office européen des brevets

(11) **EP 3 256 571 B1**

(12) **EUROPEAN PATENT SPECIFICATION**

(45) Date of publication and mention
of the grant of the patent:
**30.03.2022 Bulletin 2022/13**

(21) Application number: **16749976.3**

(22) Date of filing: **12.02.2016**

(51) International Patent Classification (IPC):
***C12N 7/00*** *(2006.01)*

(52) Cooperative Patent Classification (CPC):
**C12N 7/00; A61P 31/14;** C12N 2770/32334;
C12N 2770/32351; C12N 2770/32363

(86) International application number:
**PCT/US2016/017784**

(87) International publication number:
**WO 2016/130940 (18.08.2016 Gazette 2016/33)**

(54) **METHODS FOR PRODUCING VIRUS FOR VACCINE PRODUCTION**

VERFAHREN ZUR ERZEUGUNG EINES VIRUS ZUR HERSTELLUNG EINES IMPFSTOFFS

PROCÉDÉS DE PRODUCTION DE VIRUS POUR PRODUIRE DES VACCINS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **13.02.2015 US 201562116361 P**

(43) Date of publication of application:
**20.12.2017 Bulletin 2017/51**

(73) Proprietor: **Takeda Vaccines, Inc.
Deerfield, IL 60015 (US)**

(72) Inventor: **RAO, Raman
Bozeman, MT 59718 (US)**

(74) Representative: **Mewburn Ellis LLP
Aurora Building
Counterslip
Bristol BS1 6BX (GB)**

(56) References cited:
**WO-A1-2014/112945    US-A1- 2012 045 468
US-A1- 2012 045 468**

- **WU S-C ET AL: "Optimization of microcarrier cell culture process for the inactivated enterovirus type 71 vaccine development", VAC, ELSEVIER, AMSTERDAM, NL, vol. 22, no. 29-30, 28 September 2004 (2004-09-28), pages 3858-3864, XP004567461, ISSN: 0264-410X, DOI: 10.1016/J.VACCINE.2004.05.037**
- **ALEXANDRE LENNAERTZ ET AL: "Viral vector production in the integrity iCELLis single-use fixed-bed bioreactor, from bench-scale to industrial scale", BMC PROCEEDINGS, vol. 7, no. Suppl 6, 1 January 2013 (2013-01-01), page P59, XP055477592, London UK ISSN: 1753-6561, DOI: 10.1186/1753-6561-7-S6-P59**
- **K Lehmann ET AL: "VACCINES GENE THERAPY VIROTHERAPY CASE STUDY: iCELLis Fixed-bed Bioreactor System for the Production of Oncolytic Adenovirus (CGTG-102) with A549 Cells", , 17 October 2012 (2012-10-17), XP055250062, Retrieved from the Internet: URL:https://www.pall.com/pdfs/Biopharmaceu ticals/Case-Study-iCELLis-Fixed-bed-Biorea ctor-System-for-the-Production-of-Oncolyti c-Adenovirus-CGTG-102-with-A549-Cells.pdf [retrieved on 2016-02-15]**
- **LIN Y-C ET AL: "Characterization of a vero cell-adapted virulent strain of enterovirus 71 suitable for use as a vaccine candidate", VAC, ELSEVIER, AMSTERDAM, NL, vol. 20, no. 19-20, 7 June 2002 (2002-06-07), pages 2485-2493, XP004359678, ISSN: 0264-410X, DOI: 10.1016/S0264-410X(02)00182-2**

- **BO SUN ET AL: "Production of influenza H1N1 vaccine from MDCK cells using a novel disposable packed-bed bioreactor", APPLIED MICROBIOLOGY AND BIOTECHNOLOGY, vol. 97, no. 3, 4 September 2012 (2012-09-04), pages 1063-1070, XP055477604, DE ISSN: 0175-7598, DOI: 10.1007/s00253-012-4375-7**
- **WU ET AL.: 'Optimization of microcarrier cell culture process for the inactivated enterovirus type 71 vaccine development.' VACCINE vol. 22, 2004, pages 3858 - 3864, XP004567461**
- **LENNAERTZ, A. ET AL.: "Viral vector production in the integrity iCELLis single-use fixed-bed bioreactor, from bench-scale to industrial scale", BMC PROCEEDINGS, vol. 7, no. Suppl. 6, P59, 4 December 2013 (2013-12-04), pages 1-2,**

**Description**

FIELD

The present disclosure relates to methods for producing Enterovirus A virus for vaccine production.

BACKGROUND

Hand, foot, and mouth disease (HFMD) is caused by several members of the human enterovirus A (HEV-A) group. It is generally a self-limiting infection affecting mostly children and is characterized by ulcers and vesicles on the hands, feet and oral cavity. However, a more severe form of disease may occur with neurological symptoms such as meningitis, encephalitis, polio-like paralysis, and brain stem encephalitis leading to pulmonary edema and death (Huang, CC et al., (1999) N Engl J Med 341: 936-942; Chang, LY et al., (1998) Lancet 352: 367-368; and Ooi, MH et al., (2009) BMC Infect Dis 9: 3). Since the mid-1990s, HFMD infections caused by human enterovirus 71 (EV71) have resulted in significant morbidity and mortality, particularly in the Asia-Pacific region (Solomon, T et al., (2010) Lancet Infect Dis 10(11):778-90; and McMinn, PC (2002) FEMS Microbiol Rev 26: 91-107). China, Viet Nam, and Singapore reported increased activity in January-May 2012 compared to the same period in 2011. Hand, Foot, and Mouth Disease outbreaks disrupt education and economic activities due to school and childcare center closures in efforts to control disease transmission.

Human enterovirus A belongs to the Picornaviridae family of non-enveloped, positive-sense RNA viruses, which also includes polioviruses and rhinoviruses. Members of the HEV-A group which cause HFMD include Enterovirus 71(EV71) and Coxsackieviruses, including serotypes A1, A4, A6, A10, and A16 (Pallansch and Roos, (2007) Knipe DM, Howley PM, Griffin DE, editors. Fields Virology. Philadelphia, PA Lippincott Williams & Wilkins. pp. 839-894; and Kobayashi M et al., Jpn J Infect Dis 2013; 66:260-1). HFMD outbreaks due to EV71 infection have the greatest propensity to cause severe neurological disease. Experimental infection of cynomologus macaques showed that strains isolated across several decades were all neurotropic, as well as showing a broader tissue tropism than polioviruses (Nagata, N et al., (2002) J Med Virol 67: 207-216).

EV71 has four capsid proteins (VP1-VP4) and seven nonstructural proteins. In addition to protecting the viral RNA, the capsid proteins recognize receptors on the surface of host cells and contribute to the antigenic profile of the virus (Pallansch and Roos, (2007) Knipe DM, Howley PM, Griffin DE, editors. Fields Virology. Philadelphia, PA Lippincott Williams & Wilkins. pp. 839-894). Known human cell surface receptors for EV71 are the scavenger receptor B2 (SCARB2), and the P-selectin glycoprotein ligand 1 (PSGL-1) (Yamayoshi, S et al., (2009) Nat Med 15:798-801; and Nishimura, Y et al., (2009) Nat Med 15: 794-797).

Although the classical method of typing enteroviruses by serum neutralization defines EV71 as a single serotype (Oberste, MS et al. (1999) J Clin Microbiol 37: 1288-1293), current molecular typing methods indicate that several genogroups have been circulating in the Asia-Pacific region at least since the 1990s (Lee, MS et al., (2012) PLoS Negl Trop Dis 6: e1737). EV71 isolates were previously classified into genogroups A, B, and C and sub-genogroups based on VP1 nucleotide sequences alone (Brown, BA et al., (1999) J Virol 73: 9969-9975); nucleotide sequence identity of the VP1 gene is >92% within genogroups, whereas nucleotide sequence identity between the genogroups is 78-83% (Solomon, T et al., (2010) Lancet Infect Dis 10(11):778-90). However, whole-genome sequencing resulted in the reclassification of subgenogroup B5 under B4 and addition of genogroup D; the authors suggested that the 3D polymerase sequence together with VP1 better represented whole genomes (Chan, YF et al., (2009) Infect Genet Evol 10(3):404-12). Recombination between genogroups and with other Human enterovirus A serotypes also occurs (Yoke-Fun and Abu-Bakar (2006) BMC Microbiol 6: 74).

At present no specific antiviral therapy or vaccine against EV71 is available. Intravenous immunoglobulin has been used in severe HFMD cases, with some therapeutic benefit suggested by the outcomes but as yet unproven by clinical trials (Ooi, MH et al.,

Wu et al Vaccine 22 (2004) 3858-2864 describes optimization of micocarrier cell culture processes for the inactivated enterovirus type 71 vaccine development. US2012/0045468 A1 also describes production of enterovirus by a microcarrier cell culture process. (2009) BMC Infect Dis 9: 3; and Wang, SM et al., (1999) Clin Infect Dis 29: 184-190). Preventive and control measures during EV71 outbreaks are limited to surveillance, closure of educational and childcare facilities, and isolation of patients.

For production of potential vaccines, viral vaccines are usually produced by anchorage-dependent cell lines (e.g. VERO cells). At industrial scale, these cells are either cultivated in static mode on multi-plate systems (e.g., Cell Factories, Cell Cube, etc.), on roller bottles, or on microcarriers (porous or non-porous) in suspension in bioreactors. Multi-plate systems are bulky and require significant handling operations, whereas microcarrier cultures require numerous operations (sterilization and hydration of carriers, etc.) and many steps from precultures to final process with complex operations (i.e. bead-to-bead transfers). Wu et al Vaccine 22 (2004) 3858-2864 describes optimization of micocarrier cell culture processes for the inactivated enterovirus type 71 vaccine development. US2012/0045468 A1 also describes production of enterovirus by a microcarrier cell culture process.

Like many other enteric viruses, Enterovirus A (e.g., EV71 virus) has been adapted to grow on cell lines (e.g., Vero cells) that produce CPE with complete cell lysis, releasing the virus into the cell culture fluid. Enterovirus A (e.g., EV71 virus) virus culture is more challenging than other viruses due to lower quantum of virus production per cell and rapid cytolysis, resulting in low yields per culture run. As cell lysis occurs very rapidly, the production of large quantities of viral particles is a massive task requiring several large cell culture devices of conventional systems like hundreds of roller bottles or cell factories or several liters of large scale microcarrier culture tanks, thus requiring prolonged preculture steps as well as complex multiple operations. The current regulatory environment demands avoiding reusable culture methods as well as multiple steps of asceptic operations and transfers riddled with the risk of contaminations. However, most of the currently-available single-use reactors are not well-adapted to cultivate animal cells immobilized on micro carriers.

BRIEF SUMMARY

Thus, there is a need to develop methods for Enterovirus A production, e.g., methods that allow for viral production on an industrial scale. As disclosed herein, the methods of the present disclosure, *inter alia,* use a fixed bed culture system to enable enhanced viral production with greater cost efficiency and streamlined processing. Advantageously, these methods may be used, for example, for large- or industrial-scale production of an Enterovirus A, which is useful in the manufacture of vaccines and/or immunogenic compositions.

Accordingly, certain aspects of the present disclosure provide methods for producing an Enterovirus A virus, including (a) culturing an adherent cell in a fixed bed containing a wherein (i) the cell is inoculated with the Enterovirus A virus at an MOI of between 0.025 and 0.0009 optionally 0.001, and/or (ii) between 200,000 cells/cm$^2$ and 350,00 cells/cm$^2$ are inoculated in step (b). macrocarrier, where the cell is cultured in a first cell culture medium; (b) inoculating the cell with the Enterovirus A virus under conditions in which the Enterovirus A virus infects the cell; (c) culturing the infected cell in the fixed bed in a second cell culture medium under conditions in which the infected cell produces the Enterovirus A virus; and (d) harvesting the Enterovirus A virus produced by the cell wherein (i) the cell is inoculated with the Enterovirus A virus at an MOI of between 0.025 and 0.0009, optionally 0.001, and/or (ii) between 200,000 cells/cm$^2$ and 350,000 cells/cm$^2$ are inoculated in step (b). In some embodiments the cell is a mammalian cell. In some embodiments that may be combined with any of the above embodiments, the cell is a Vero cell. In some embodiments that may be combined with any of the above embodiments, the Vero cell line is selected from a WHO Vero 10-87, ATCC CCL-81, Vero 76 (ATCC Accession No. CRL-1587), and Vero C1008 (ATCC Accession No. CRL-1586). In some embodiments that may be combined with any of the above embodiments, the Enterovirus A virus is selected from EV71, CA6, and CA16. In some embodiments that may be combined with any of the above embodiments, the Enterovirus A virus contains at least one non-human cell adaptation mutation. In some embodiments that may be combined with any of the above embodiments, the Enterovirus A virus is EV71, and the at least one non-human cell adaptation mutation includes a VP1 polypeptide mutation. In some embodiments that may be combined with any of the above embodiments, the Enterovirus A virus is CA6, and the at least one non-human cell adaptation mutation includes a mutation selected from a VP1 polypeptide mutation, a VP2 polypeptide mutation, a VP3 polypeptide mutation, and a 5' untranslated region (UTR) mutation. In some embodiments that may be combined with any of the above embodiments, the Enterovirus A virus is CA16, and the at least one non-human cell adaptation mutation includes a mutation selected from a 2A polypeptide mutation, a VP1 polypeptide mutation, a VP2 polypeptide mutation, and a 5' untranslated region (UTR) mutation. In some embodiments that may be combined with any of the above embodiments, between about 4,000 cells/cm$^2$ and about 16,000 cells/cm$^2$ are cultured in the step of culturing an adherent cell in a fixed bed containing a macrocarrier, where the cell is cultured in a first cell culture medium. In some embodiments that may be combined with any of the above embodiments, about 5,000 cells/cm$^2$ are cultured in the step of culturing an adherent cell in a fixed bed containing a macrocarrier, where the cell is cultured in a first cell culture medium.

In some embodiments that may be combined with any of the above embodiments, the first cell culture medium contains serum. In some embodiments that may be combined with any of the above embodiments, the first cell culture medium contains less than 10% fetal bovine serum, and the cell is not adapted to serum free medium. In some embodiments that may be combined with any of the above embodiments, the first cell culture medium contains 5% fetal bovine serum. In some embodiments that may be combined with any of the above embodiments, the first cell culture medium and the second cell culture medium are different. In some embodiments that may be combined with any of the above embodiments, the method further includes, between the steps of culturing an adherent cell in a fixed bed containing a macrocarrier, where the cell is cultured in a first cell culture medium and inoculating the cell with the Enterovirus A virus under conditions in which the Enterovirus A virus infects the cell, removing the first cell culture medium and rinsing the cell with the second culture medium. In some embodiments that may be combined with any of the above embodiments, the second cell culture medium is a serum-free medium.

In some embodiments that may be combined with any of the above embodiments, the cell is inoculated with the Enterovirus A virus at an MOI of about 0.001. In some embodiments that may be combined with any of the above embodiments, the cell is cultured during the steps of culturing an adherent cell in a fixed bed containing a macrocarrier, where the cell is cultured in a first cell culture medium, inoculating the cell with the Enterovirus A virus under conditions in which the Enterovirus A virus infects the cell, and culturing the infected cell in the fixed bed in a second cell culture medium under conditions in which the infected cell produces the Enterovirus A virus, at a volume/surface ratio of about 0.3 mL/cm$^2$. In some embodiments that may be combined with any of the above embodiments, the lactate concentration in the first cell culture medium during the steps of culturing an adherent cell in a fixed bed containing a macrocarrier, where the cell is cultured in a first cell culture medium and/or inoculating the cell with the Enterovirus A virus under conditions in which the Enterovirus A virus infects the cell does not exceed about 25 mM. In some embodiments that may be combined with any of the above embodiments, the lactate concentration in the second cell culture medium during the step of culturing the infected cell in the fixed bed in a second cell culture medium under conditions in which the infected cell produces the Enterovirus A virus does not exceed about 25 mM. In some embodiments that may be combined with any of the above embodiments, the density of oxygen (DO) in the first cell culture medium during the steps of culturing an adherent cell in a fixed bed containing a macrocarrier, where the cell is cultured in a first cell culture medium and/or inoculating the cell with the Enterovirus A virus under conditions in which the Enterovirus A virus infects the cell is maintained above about 50%. In some embodiments that may be combined with any of the above embodiments, the density of oxygen (DO) in the second cell culture medium during the step of culturing the infected cell in the fixed bed in a second cell culture medium under conditions in which the infected cell produces the Enterovirus A virus is maintained above about 50%. In some embodiments that may be combined with any of the above embodiments, the fixed bed has a bed height of about 2 cm. In some embodiments that may be combined with any of the above embodiments, the fixed bed has a bed height of about 10 cm. In some embodiments that may be combined with any of the above embodiments, the macrocarrier is a microfiber matrix. In some embodiments that may be combined with any of the above embodiments, the macrocarrier has a porosity between about 60% and 99%. In some embodiments that may be combined with any of the above embodiments, the porosity is between about 80% and about 90%. In some embodiments that may be combined with any of the above embodiments, the macrocarrier has a surface area accessible to the cell of between about 150 cm$^2$/cm$^3$ and about 1000 cm$^2$/cm$^3$. In some embodiments that may be combined with any of the above embodiments, at least 5.0 x 10$^7$ TCID50/mL of the Enterovirus A virus is harvested in the step of harvesting the Enterovirus A virus produced by the cell. In some embodiments that may be combined with any of the above embodiments, the methods further include inactivating the Enterovirus A with one or more of beta-propiolactone (BPL), formalin, or binary ethylenimine (BEI). In some embodiments that may be combined with any of the above embodiments, the Enterovirus A virus is an attenuated virus.

Also described herein is an Enterovirus A virus produced by the method of any of the above embodiments. In some embodiments that may be combined with any of the above embodiments, the virus contains one or more antigens. In some embodiments that may be combined with any of the above embodiments, the virus has been inactivated with one or more of beta-propiolactone (BPL), formalin, or binary ethylenimine (BEI).

Also described herein is a composition containing an Enterovirus A virus produced by the method of any of the above embodiments. Also described is an immunogenic composition containing an Enterovirus A virus produced by the method of any of the above embodiments. Also described is a vaccine containing an Enterovirus A virus produced by the method of any of the above embodiments.

BRIEF DESCRIPTION OF THE DRAWINGS

FIG. 1 shows an experimental cell culture set-up in NANO. For the recirculation, a MasterFlex pump mounted with EasyLoad heads (not depicted) was used instead of the perfusion module shown in this picture.

FIG. 2A to 2C show pictures of the NANO carriers. Carriers were sampled out at different times and colored with Crystal Violet. FIG. 2A shows the empty carrier. FIG. 2B shows the carrier at Day 3. FIG. 2C shows the carrier at Day 6.

FIG. 3 shows monitoring results for the iCELLis NANO. The measured parameters were cell number, glucose and lactate concentrations, dissolved of oxygen (DO), pH, and temperature. Medium replacement was performed on Days 3 and 6.

FIG. 4 shows a comparison of cell densities in the iCELLis NANO and in the control Cell-Stacks. For the iCELLis NANO, cells were counted using the nucleus count method, whereas for the Cell-Stacks, cells were counted after trypsinization using the Trypan blue method.

**FIG. 5** shows the experimental plan for the EV71 iCELLis NANO culture using a cellular density of 0.52 E6 cells/cm$^2$.

**FIG. 6** shows the experimental plan for the control EV71 Cell-Stack culture using a cellular density of 0.52 E6 cells/cm$^2$.

**FIG. 7** shows monitoring results for the EV71 iCELLis NANO culture using a cellular density of 0.52 E6 cells/cm$^2$. The measured parameters were cells number, glucose and lactate concentrations, dissolved of oxygen (DO), pH, and temperature. Medium replacement was performed on Day 3 and infection on Day 6.

**FIG. 8** shows monitoring results for the control EV71 Cell-Stack culture using a cellular density of 0.52 E6 cells/cm$^2$. The measured parameters were: glucose and lactate concentrations. Infection was performed on Day 4.

**FIG. 9** shows EV71 cell-stack culture cytopathic effect (CPE) using a cellular density of 0.52 E6 cells/cm$^2$.

**FIG. 10** shows the experimental plan for the EV71 iCELLis NANO culture and control cell-stack cultures using a cellular density of 0.25 E6 cells/cm$^2$.

**FIG. 11** shows monitoring results for the EV71 iCELLis NANO culture using a cellular density of 0.25 E6 cells/cm$^2$. The measured parameters were cells number, glucose and lactate concentrations, dissolved of oxygen (DO), pH, and temperature. Infection was performed on Day 4.

**FIG. 12** shows monitoring results for the control EV71 Cell-Stack culture using a cellular density of 0.25 E6 cells/cm$^2$. The measured parameters were glucose and lactate concentrations. Infection was performed on Day 4.

**FIG. 13** shows the experimental plan for the benchmark Cytodex process for Polio S2 production.

**FIG. 14** shows the experimental plan for the Polio S2 iCELLis NANO culture using a cellular density of 0.52 E6 cells/cm$^2$.

**FIG. 15** shows the experimental plan for the control Polio S2 Cell-Stack culture using a cellular density of 0.52 E6 cells/cm$^2$.

**FIG. 16** shows monitoring results for the Polio S2 iCELLis NANO culture using a cellular density of 0.52 E6 cells/cm$^2$. The measured parameters were: cells number, glucose, and lactate concentrations. Medium replacement was performed on Day 3 and infection was performed on Day 6.

**FIG. 17** shows monitoring results for the control Polio S2 Cell-Stack culture using a cellular density of 0.52 E6 cells/cm$^2$. The measured parameters were: glucose and lactate concentrations. Infection was performed on Day 4.

**FIG. 18** shows Polio S2 cell-stack culture cytopathic effect (CPE) using a cellular density of 0.52 E6 cells/cm$^2$.

**FIG. 19** shows the experimental plan for the Polio S2 iCELLis NANO and control cell-stack cultures using a cellular density of 0.25 E6 cells/cm$^2$.

**FIG. 20** shows monitoring results for the Polio S2 iCELLis NANO culture using a cellular density of 0.25 E6 cells/cm$^2$. The measured parameters were: cells number, glucose and lactate concentrations, density of oxygen (DO), pH, and temperature. Infection was performed on Day 4.

**FIG. 21** shows monitoring results for the control Polio S2 Cell-Stack culture using a cellular density of 0.25 E6 cells/cm$^2$. The measured parameters were: glucose and lactate concentrations. Infection was performed on Day 4.

**FIG. 22** shows Polio S2 cell-stack culture cytopathic effect (CPE) using a cellular density of 0.25 E6 cells/cm$^2$.

**FIG. 23A** shows specific (per cell) maximal EV71 productivity according to cell density at infection. **FIG. 23B** shows total (per volume of harvest) maximal EV71 productivity according to cell density at infection. **FIG. 23C** shows virus release kinetics per cell. **FIG. 23D** shows virus release kinetics per volume of harvest.

**FIG. 24** shows an evaluation of decreasing cell density at EV71 inoculation. Two iCELLis NANO were inoculated, one at 15,000 cells/cm2 (upper panel), the other at a 3-fold lower cell density (5,000 cells/cm2; lower panel). Glucose (red curve) and lactate (blue curve) were monitored throughout the culture.

**FIG. 25** shows the impact of volume/surface ratio and FBS concentration on final cell density in T-flasks.

**FIG. 26** shows a schematic view of the EV71 optimized process in iCELLis NANO.

**FIG. 27** shows a summary of maximal viral productivities obtained for EV71 in all iCELLis NANO experiments.

**FIG. 28** shows a schematic of the EV71 process in iCELLis500/100.

**FIG. 29** shows the experimental set-up of the EV71 iCELLis500/100 run.

**FIG. 30A to 30C** show EV71 cell culture parameters in iCELLis500/100. **FIG. 30A** shows cell density. **FIG. 30B** shows dissolved oxygen (set-point is 50%). **FIG. 30C** shows glucose and lactate concentrations. **FIG. 30D to 30F** show EV71 cell culture parameters in control iCELLis NANO. **FIG. 30D** shows cell density. **FIG. 30E** shows dissolved oxygen (set-point is 50%). **FIG. 30F** shows glucose and lactate concentrations.

**FIG. 31A** shows the kinetic release of EV71 virus after infection in iCELLis500/100. Virus was quantified according to the TCID50 method. **FIG. 31B** shows the kinetic release of HCP after infection in iCELLis500/100. HCP was quantified using the Octet and Signus anti Vero-HCP antibodies. **FIG. 31C** shows the kinetic release of and DNA after infection in iCELLis500/100. DNA was quantified using the pico-green method.

**FIG. 32** shows a schematic drawing summarizing the yield of the EV71 iCELLis500/100 run.

**FIG. 33** shows a schematic drawing of EV71 infection in T-flasks using serum-free (SFM, OptiPro) or 10% FBS (M199) media.

**FIG. 34** shows a schematic of the EV71 process development strategy.

**FIG. 35** shows a schematic of the EV71 factory-scale process in iCELLis500/500.

DETAILED DESCRIPTION

## *General Techniques*

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

## *Cell Culture*

Certain aspects of the present disclosure relate to methods for producing an Enterovirus A virus (the term "Enterovirus A" may be used interchangeably herein). Producing an Enterovirus A may be useful, *e.g.,* for vaccines and/or immunogenic compositions including, without limitation, purified viruses, inactivated viruses, attenuated viruses, recombinant viruses, or purified and/or recombinant viral proteins for subunit vaccines. In some embodiments, a method of the present disclosure for producing an Enterovirus A virus includes culturing a cell in a bed containing a matrix, where the cell is cultured in a first cell culture medium; inoculating the cell with the Enterovirus A virus under conditions in which the Enterovirus A virus infects the cell; culturing the infected cell in the fixed bed in a second cell culture medium under conditions in which the infected cell produces the Enterovirus A virus; and harvesting the Enterovirus A virus produced by the cell.

In some embodiments, the cell of the present disclosure is a mammalian cell (*e.g.,* a mammalian cell line). Cell lines suitable for growth of the at least one virus of the present disclosure are preferably of mammalian origin, and include but are not limited to: Vero cells (from monkey kidneys), horse, cow (e.g. MDBK cells), sheep, dog (e.g. MDCK cells from dog kidneys, ATCC CCL34 MDCK (NBL2) or MDCK 33016, deposit number DSM ACC 2219 as described in WO97/37001), cat, and rodent (e.g. hamster cells such as BHK21-F, HKCC cells, or Chinese hamster ovary cells (CHO cells)), and may be obtained from a wide variety of developmental stages, including for example, adult, neonatal, fetal, and embryo. In certain embodiments, the cells are immortalized (e.g. PERC.6 cells, as described in WO01/38362 and WO02/40665, and as deposited under ECACC deposit number 96022940). In preferred embodiments, mammalian cells are utilized, and may be selected from and/or derived from one or more of the following non-limiting cell types: fibroblast cells (e.g. dermal, lung), endothelial cells (e.g. aortic, coronary, pulmonary, vascular, dermal microvascular, umbilical), hepatocytes, keratinocytes, immune cells (e.g. T cell, B cell, macrophage, NK, dendritic), mammary cells (e.g. epithelial), smooth muscle cells (e.g. vascular, aortic, coronary, arterial, uterine, bronchial, cervical, retinal pericytes), melanocytes, neural cells (e.g. astrocytes), prostate cells (e.g. epithelial, smooth muscle), renal cells (e.g., epithelial, mesangial, proximal tubule), skeletal cells (e.g. chondrocyte, osteoclast, osteoblast), muscle cells (e.g. myoblast, skeletal, smooth, bronchial), liver cells, retinoblasts, and stromal cells. WO97/37000 and WO97/37001 describe production of animal cells and cell lines that capable of growth in suspension and in serum free media and are useful in the production and replication of viruses.

In some embodiments, the cell is a Vero cell. Examples of suitable Vero cell lines include, without limitation, WHO Vero 10-87, ATCC CCL-81, Vero 76 (ATCC Accession No. CRL-1587), or Vero C1008 (ATCC Accession No. CRL-1586).

In some embodiments, the cell is an adherent cell. As used herein, an adherent cell may refer to any cell that adheres or anchors to a substrate during culturing.

Culture conditions for the above cell types are known and described in a variety of publications, or alternatively culture medium, supplements, and conditions may be purchased commercially, such as for example, as described in the catalog and additional literature of Cambrex Bioproducts (East Rutherford, N.J.).

Known serum-free media include Iscove's medium, Ultra-CHO medium (BioWhittaker) or EX-CELL (JRH Bioscience). Ordinary serum-containing media include Eagle's Basal Medium (BME) or Minimum Essential Medium (MEM) (Eagle, Science, 130, 432 (1959)) or Dulbecco's Modified Eagle Medium (DMEM or EDM), which are ordinarily used with up to 10% fetal calf serum or similar additives. Optionally, Minimum Essential Medium (MEM) (Eagle, Science, 130, 432 (1959)) or Dulbecco's Modified Eagle Medium (DMEM or EDM) may be used without any serum containing supplement. Protein-free media like PF-CHO (JHR Bioscience), chemically-defined media like ProCHO 4CDM (BioWhittaker) or SMIF 7 (Gibco/BRL Life Technologies) and mitogenic peptides like Primactone, Pepticase or HyPep.TM. (all from Quest International) or lactalbumin hydrolyzate (Gibco and other manufacturers) are also adequately known in the prior art. The media additives based on plant hydrolyzates have the special advantage that contamination with viruses, mycoplasma or unknown infectious agents can be ruled out.

Certain aspects of the methods of the present disclosure relate to the density at which cells are cultured. In some embodiments, the density or absolute number of cells cultured in a first culture medium may refer to the density or absolute number of cells with which the cell culture is seeded, *i.e.,* before viral inoculation. As described herein, and without wishing to be bound to theory, it is thought that culturing cells at a lower density may be advantageous to reduce the cell density at viral inoculation, prolong the cells' growth phase, and/or reduce factors including without limitation the size of the inoculum, labor time, footprint in the pre-culture step, number of incubators, and/or the risk of contamination.

In some embodiments, between about 4,000 cells/cm$^2$ and about 16,000 cells/cm$^2$ are cultured. In some embodiments, about 4,000 cells/cm$^2$; about 5,000 cells/cm$^2$; about 6,000 cells/cm$^2$; about 7,000 cells/cm$^2$; about 8,000 cells/cm$^2$; about 9,000 cells/cm$^2$; about 10,000 cells/cm$^2$; about 11,000 cells/cm$^2$; about 12,000 cells/cm$^2$; about 13,000 cells/cm$^2$; about 14,000 cells/cm$^2$; about 15,000 cells/cm$^2$; or about 16,000 cells/cm$^2$ are cultured, including any value therebetween. In some embodiments, the cell density before inoculation is less than about any of the following densities (in cells/cm$^2$): 16,000; 15,500; 15,000; 14,500; 14,000; 13,500; 13,000; 12,500; 12,000; 11,500; 11,000; 10,500; 9,000; 8,500; 8,000; 7,500; 7,000; 6,500; 6,000; 5,500; 5,000; or 4,500. In some embodiments, the cell density before inoculation is greater than about any of the following densities (in cells/cm$^2$): 4,000; 4,500; 5,000; 5,500; 6,000; 6,500; 7,000; 7,500; 8,000; 8,500; 9,000; 9,500; 10,000; 10,500; 11,000; 11,500; 12,000; 12,500; 13,000; 13,500; 14,000; 14,500; 15,000; or 15,500. That is, the cell density before inoculation can be any of a range of densities having an upper limit of 16,000;

15,500; 15,000; 14,500; 14,000; 13,500; 13,000; 12,500; 12,000; 11,500; 11,000; 10,500; 9,000; 8,500; 8,000; 7,500; 7,000; 6,500; 6,000; 5,500; 5,000; or 4,500 and an independently selected lower limit of 4,000; 4,500; 5,000; 5,500; 6,000; 6,500; 7,000; 7,500; 8,000; 8,500; 9,000; 9,500; 10,000; 10,500; 11,000; 11,500; 12,000; 12,500; 13,000; 13,500; 14,000; 14,500; 15,000; or 15,500; wherein the lower limit is less than the upper limit. In certain embodiments, about 5,000 cells/cm$^2$ are cultured.

In some embodiments, a cell of the present disclosure is inoculated with an Enterovirus A virus of the present disclosure under conditions in which the Enterovirus A virus infects the cell. Conditions under which an Enterovirus A infects a cell are known in the art and may depend on the type of cell, the type of Enterovirus A, the culture medium, temperature, cell density, viral density (*e.g.*, MOI), cell growth rate, number of cell passages, and so forth. Exemplary descriptions of conditions under which an Enterovirus infects a cell are provided *infra*.

Certain aspects of the methods of the present disclosure relate to the cell density at the time of viral inoculation. As described herein, and without wishing to be bound to theory, it is thought that the cell density at infection may impact virus production (*e.g.*, viral productivity). An optimal cell density at viral inoculation may result in increased specific (per cell) productivity, volumetric (per mL of harvest) productivity, and/or stability, as well as reduced media consumption and/or contaminants in the harvest.

In some embodiments, between 200,000 cells/cm$^2$ and 350,000 cells/cm$^2$ are inoculated with Enterovirus A. In some embodiments, the cell density at inoculation is less than about any of the following densities (in cells/cm$^2$): 350,000; 300,000 or 250,000. In some embodiments, the cell density at inoculation is greater than about any of the following densities (in cells/cm$^2$): 200,000; 250,000 or 300,000; That is, the cell density at inoculation can be any of a range of densities having an upper limit of or 350,000; 300,000; or 250,000 and an independently selected or lower limit of 200,000; 250,000 or 300,000 wherein the lower limit is less than the upper limit.

Certain aspects of the methods of the present disclosure relate to the MOI of enterovirus used to inoculate a cell culture of the present disclosure. MOI is used herein consistent with its accepted meaning in the art, *i.e.,* a known or predicted ratio of viral agent (*e.g.,* Enterovirus A virus) to viral target (*e.g.,* a cell of the present disclosure). MOI is known in the art to affect the percentage of cells in a culture that are infected with at least one virus. While a higher MOI may increase viral productivity, at a certain range of MOI infection rate may saturate, and reducing the MOI after reaching a threshold or desired infection rate may lower the volume and costs of the corresponding virus seed bank.

In some embodiments, the cell is inoculated with the Enterovirus A virus at an MOI of between about 0.025 and about 0.0009. In some embodiments, the MOI is less than about any of the following MOIs: 0.025, 0.022, 0.020, 0.018, 0.015, 0.012, 0.010, 0.008, 0.005, 0.002, or 0.0010. In some embodiments, the MOI is greater than about any of the following MOIs: 0.0009, 0.0010, 0.002, 0.005, 0.008, 0.010, 0.012, 0.015, 0.018, 0.020, or 0.022. That is, the MOI can be any of a range of MOIs having an upper limit of 0.025, 0.022, 0.020, 0.018, 0.015, 0.012, 0.010, 0.008, 0.005, 0.002, or 0.0010 and an independently selected lower limit of 0.0009, 0.0010, 0.002, 0.005, 0.008, 0.010, 0.012, 0.015, 0.018, 0.020, or 0.022, wherein the lower limit is less than the upper limit. In certain embodiments, the cell is inoculated with the Enterovirus A virus at an MOI of about 0.001.

Upon infection, an infected cell of the present disclosure may be cultured (*e.g.,* in a fixed bed of the present disclosure) in a second cell culture medium. A cell cultured as described herein may be cultured in a first culture medium before viral inoculation and a second culture medium at and/or after viral inoculation. For example, in some embodiments, a cell of the present disclosure may be cultured in a first cell culture medium, then the first cell culture medium may be removed, the cell may optionally be rinsed (e.g., with an aqueous buffered solution such as PBS), and a second culture medium may be added to the cell. In some embodiments, the second culture medium may contain the Enterovirus A virus for inoculation of the cell. In some embodiments, the first culture medium and the second culture medium are the same culture medium (*e.g.*, having the same composition, which in some embodiments is not necessarily the same physical medium). In other embodiments, the first culture medium and the second culture medium are different (*e.g.*, having a different composition).

In some embodiments, the first cell culture medium contains serum (*e.g.*, fetal bovine serum). Any type of serum suitable for growth of the cultured cell may be used. Examples of sera in the art include without limitation fetal bovine serum, fetal calf serum, horse serum, goat serum, rabbit serum, rat serum, mouse serum, and human serum. In some embodiments, the first cell culture medium contains less than 10% serum (*e.g.*, fetal bovine serum), and the cell is not adapted to serum free medium. In certain embodiments, the first cell culture medium contains 5% serum (*e.g.*, fetal bovine serum).

In some embodiments, the second culture medium is a serum-free medium. In some embodiments, the second culture medium is a protein free medium. A medium (*e.g.*, a culture medium of the present disclosure) is referred to as a serum-free medium in the context of the present disclosure in which there are no additives from serum of human or animal origin. Protein-free is understood to mean cultures in which multiplication of the cells occurs with exclusion of proteins, growth factors, other protein additives and non-serum proteins, but can optionally include proteins such as trypsin or other proteases that may be necessary for viral growth. The cells growing in such cultures naturally contain proteins themselves.

The viral inoculum and the viral culture are preferably free from (*i.e.* will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [WO2006/027698].

Other parameters of cell culturing and the cell culture medium may also affect virus production. In some embodiments, a cell of the present disclosure may be cultured in a first and/or a second culture medium of the present disclosure at a desired volume/surface ratio. Without wishing to be bound to theory, the volume/surface ratio at which a cell is cultured may affect parameters such as cell productivity, cell size, growth rate, and/or access to nutrients and other components in the culture medium. In certain embodiments, the cell is cultured (*e.g.,* before, during, and/or after viral inoculation) at a volume/surface ratio of about 0.3 mL/cm$^2$.

Under some conditions, a cell of the present disclosure may produce lactate during culturing. It is known in the art that cells in culture may produce lactate as a result of glycolytic or anaerobic-type metabolism. Without wishing to be bound to theory, it is thought that excessive lactate in cell culture medium may negatively impact cell growth, metabolism, and/or virus productivity, *e.g.,* by reducing the pH of the culture medium. Further, excessive lactate production by cultured cells may indicate a cellular metabolic state that may not be desired for virus production and/or growth. In some embodiments, the lactate concentration in the first cell culture medium and/or the second cell culture medium does not exceed about 25 mM. Methods for measuring lactate concentration in a cell culture medium are known in the art and include without limitation use of a lactometer, a lactate enzymatic assay (*e.g.*, through using lactate dehydrogenase and a colorimetric or fluorometric detection reagent), a microdialysis sampling device, and the like.

In some embodiments, an infected cell of the present disclosure may be cultured (*e.g.,* in a fixed bed of the present disclosure) in a second cell culture medium under which the infected cell produces the Enterovirus A virus. Conditions under which an infected cell produces an Enterovirus A are known in the art and may depend on the type of cell, the type of Enterovirus A, the culture medium, temperature, cell density, viral density (*e.g.*, MOI), cell growth rate, number of cell passages, and so forth. Exemplary descriptions of conditions under which an Enterovirus infects a cell are provided *infra.*

Other aspects of the methods described herein relate to the density of oxygen (DO) in a cell culture medium. Maintaining suitable oxygen levels in a cell culture medium may promote cell growth and/or virus productivity by providing oxygen for cellular respiration. In addition, oxygen utilization by cells in a cell culture may reflect their state of health, growth, and/or metabolism. In some embodiments, the density of oxygen in the first cell culture medium and/or the second cell culture medium is maintained above about 50%. Methods for maintaining and/or measuring DO in a cell culture medium are known in the art and may include without limitation automatic oxygen injection, exposing the cell culture medium to oxygen (preferably while minimizing the risk of contamination), using an oxygen controller, using an oxygen sensor, and so forth.

Various methods known in the art may be used to measure the titer of infectious virus produced by the methods of the present disclosure. Such methods include without limitation an endpoint dilution assay (*e.g.*, to determine a TCID50 value), a protein assay, quantitative transmission electron microscopy (TEM), a plaque assay, a focus forming assay (FFA), and so forth. In some embodiments, virus titer is measured in TCID50/mL. In some embodiments, at least 5.0 x 10$^7$ TCID50/mL of the Enterovirus A virus is harvested.

## *Devices for Cell Culture*

Certain aspects of the present disclosure relate to methods for culturing a cell in a fixed bed. Exemplary cell culture devices are referenced in US8597939, US8137959, US PG Pub 2008/0248552, and WO2014093444 and are commercially available (*e.g.,* the iCELLis® Bioreactors from Pall® Life Sciences, Port Washington, NY, such as the Nano and 500/100 bioreactors).

In some embodiments, the cell is cultured in a fixed-bed bioreactor. Fixed-bed bioreactors include a carrier in the form of a stationary packing material forming a fixed or packed bed for promoting cell adhesion and growth. The arrangement of the packing material of the fixed bed affects local fluid, heat, and mass transport, and usually is very dense to maximize cell cultivation in a given space. In one embodiment, the reactor includes a wall forming an interior with a packed or fixed bed comprised of a packing material (such as fibers, beads, spheres, or the like) for promoting the adhesion and growth of cells. The material is located in a compartment within the interior of the reactor, which compartment may comprise an upper portion of a hollow, vertically extending tube. A second compartment is provided within the interior of the reactor for conveying fluid to and from the material of compartment at least partially forming the fixed bed. Typically, the packing material should be arranged to maximize the surface area for cell growth, with 1,000 square meters being considered an advantageous amount of surface area (which, for example, may be achieved using medical grade polyester microfibers as the packing material). In one embodiment, evenly-distributed media circulation is achieved by a built-in magnetic drive impeller, ensuring low shear stress and high cell viability. The cell culture medium flows through the fixed-bed from the bottom to the top. At the top, the medium falls as a thin film down the outer wall where it takes up $O_2$ to maintain high $K_La$. in the bioreactor. This waterfall oxygenation, together with a gentle agitation and biomass immobilization, enables the bioreactor to achieve and maintain high-cell densities. In some embodiments, the fixed bed has a bed height of about 2 cm. In other embodiments, the fixed bed has a bed height of about 10 cm.

In some embodiments, the fixed bed contains a macrocarrier (*e.g.*, a matrix). In some embodiments, the macrocarrier is a fiber matrix. In some embodiments, the macrocarrier is a carbon fiber matrix. The macrocarrier may be selected from woven or non-woven microfibers, polyester microfibers (*e.g.*, medical-grade polyester microfibers) porous carbon and matrices of chitosans. The microfibers may optionally be made of PET or any other polymer or biopolymer. In some embodiments, the macrocarriers include beads. The polymers may be treated to be compatible with cell culture, if such treatment is necessary.

Suitable macrocarrier, matrix or "carrying material" are mineral carriers such as silicates, calcium phosphate, organic compounds such porous carbon, natural products such as chitosan, polymers or biopolymers compatible with cells growth. The matrix can have the form of beads with regular or irregular structure, or may comprising woven or non-woven microfibers of a polymer or any other material compatible with cell growth. The packing can also be provided as a single piece with pores and or channels. The packing in the recipients can have a variety of forms and dimensions. In some embodiments the matrix is a particulate material of solid or porous spheres, flakes, polygons. Typically a sufficient amount of matrix is used to avoid movement of the matrix particles within the recipient upon use, as this may damage cells and may have an influence on the circulation of gas and/or medium. Alternatively the matrix consists of an element which fits into the inner recipient or into a compartment of the recipient, and having an adequate porosity and surface. An example hereof is a carbon matrix (Carboscale) manufactured by Cinvention (Germany). In some embodiments, the fiber matrix has a surface area accessible to the cell of between about 150 $cm^2/cm^3$ and about 1000 $cm^2/cm^3$.

In some embodiments, the macrocarrier (*e.g.*, a fiber matrix) has a porosity between about 60 and 99%. In some embodiments the macrocarrier (*e.g.*, a fiber matrix) has a porosity between about 80 and about 90%. Optionally, the packing may have a porosity P in the range of 50% to 98%. The term porosity P is the volume of air present in a given volume of the material, and expressed as percentage of the given volume of the material. The porosity can be measured by measuring the weight Wx per volume of the porous material, and using the formula:

$$P = 100 - (1 - Wx\ Wspec)$$

where Wspec is the specific weight of the material. The porous material may be one solid unit of porous material, or may be a plurality of individual units, such as grains, chips, beads, fibers or fiber agglomerates.

In some embodiments, the fixed bed is a single-use or disposable fixed bed. For example, commercially available bioreactors such as the iCELLis® Nano and 500/100 bioreactors Bioreactors (Pall® Life Sciences, Port Washington, NY) may include a bioreactor system with a removable, disposable, or single use fixed bed that provides a large growth surface area in a compact bioreactor volume. Compared to a standard stirred-tank bioreactor using microcarriers, such systems avoid several delicate and time-consuming procedures, including manual operations, sterilization and hydration of microcarriers and bead-to-bead transfers from preculture to final process. As described herein, such bioreactors may enable process at a large scale (*e.g.,* 500 square meters) culture area equivalent and harvest fluid volumes of up to 1500 to 2000 L, which is advantageous for industrial scale production of virus (*e.g.*, for use in vaccine production). As exemplified herein, such devices may enable further advantages such as low cell inoculums; reaching of optimal cell density for infection at a short preculture period; and/or optimization of MOI, media and serum concentrations during the culture growth phase. such devices may be configured to allow rapid perfusion of the cells in culture, *e.g.*, such that 90% or more of the cells experience the same medium environment. Moreover, and without wishing to be bound to theory, a single-use or disposable fixed bed may allow streamlined downstream processing to maximize the productivity as well as reduce the foot print of the process area even with scale up equivalent to several large scale conventional culture vessels. As such, advantageous productivity and purity may be achieved with minimal steps and costs.

In one embodiment, the recipient for cell cultivation has an inner space, which may be annular but may take other forms. The space contains packing. When the recipient is to be used for cell culture the packing should be compatible with cell growth. In the annular configuration, the inner space has an annular volume delimited by: an outer tubular wall having a first outer end and a second outer end and a longitudinal wall extending in longitudinal direction. The outer tubular wall delimits an outer boundary of the annular volume in a longitudinal direction; a first and a second closure delimiting and closing the annular volume at the first outer end respectively the second outer end of the outer tubular wall; an inner elongate wall having a first outer end oriented towards the first outer end of the outer tubular wall, and a second outer end oriented towards the second outer end of the outer tubular wall. The inner elongate wall is positioned within the outer tubular wall. The inner elongate wall extends in a longitudinal direction and delimits an inner boundary of the annular volume, the inner boundary being encompassed by the outer boundary. The second outer end of the inner elongate wall coincides with the second closure. As an example, the outer tubular wall is provided by a cylindrical outer tubular element. The inner elongate wall may be provided by a solid inner cylindrical element, such as a cylindrical rod. The outer tubular element is a cylindrical tubular element, and has a central axis, parallel to the longitudinal direction. The inner cylindrical element and the outer tubular element may be coaxially mounted.

In some embodiments, the first outer end of the inner cylindrical element may comprise a coupling element to couple the inner cylindrical element, and by means of the closures being fixed to the inner cylindrical element and the outer tubular element, the outer tubular element as well, to a drive mechanism, e.g. a motor of the bioreactor. The second closure is provided with a connector, suitable to couple the recipient to a medium or gas source, for providing and/or extracting medium and/or gas to and/or from the inner space. This connector or alternatively additional connectors may be provided to the first closure or the second closure.

In some embodiments, upon moving the recipient, the packing, in particular the porous material, may rest in a fixed relative position to the recipient, or may move within and relative to the recipient or, as the case may be, within the compartment of the recipient. The recipient is to be rotated about its axis, optionally at a rotational speed of between 0.1 and 25 rotations per minute.

In some embodiments, the inner space is partially filled with cultivation medium, such as cell cultivation medium. As an example, the liquid level at least contacts the inner elongate wall, or the inner elongate wall is partially submerged in the medium. The part of the packing positioned under the liquid level is wetted by the cultivation medium, such as cell cultivation medium. The packing positioned above the liquid level is in contact with the gas or air present in the inner space. When the recipient is rotated in one direction about the axis, e.g. clockwise rotated, the cultivation medium, such as ceil cultivation medium rotates in opposite, say anti-clockwise direction relative to the packing. The cultivation medium, such as cell cultivation medium, is passed through the complete packing according to a plug flow. Upon rotation, e.g. clockwise, of the recipient, the cultivation medium, such as cell cultivation medium forces the gas or air at the leading edge of the plug flow to displace anti-clockwise. At the tailing edge of the medium, an optionally limited depression is created, causing gas or air to be sucked towards the trailing edge. As such the medium and the gas or air passes through the complete packing.

In some embodiments, the inner elongate wall of an alternative recipient may be provided by a cylindrical tubular element. The outer tubular wall is provided by an outer tubular element. The inner elongate wall is provided by an elongate cylindrical tubular element. The outer tubular element and the elongate cylindrical tubular element are fixed to two removable closures. The first closure is provided with a coupling element for coupling the recipient to a driving means for rotating the recipient along an axis in a longitudinal direction. The first closure further comprises a connector for connecting the inner space to a conduit, such as a flexible tube.

In some embodiments, the outer tubular element may be a glass tube, having a length L of e.g. 110 mm and an inner diameter Do of, for example, 135 mm. The inner elongate element may be a polyvinylidenefluoride (PVDF) tube having an outer diameter Di of, for example, 88.9 mm. The outer ends of the inner elongate element, hence of the inner elongate wail, coincide with the closures. The closures may be stainless steel or PVDF annular discs, which may be attached to the inner and outer element using silicone. The first closure, which may be provided with a connector, has a coupling element having an outer diameter Dee of, for example, about 35 mm. The inner space is at least partially filled with packing.

In some embodiments, the recipient further comprises 2 fluid permeable dividers dividing the inner space in 2 compartments. The fluid permeable dividers extend from the inner elongate wall to the outer tubular wall and from the first closure to the second closure in the longitudinal direction parallel to the direction of the tubular axis. One compartment is provided with the packing. One compartment is not provided with the packing. The fluid permeable dividers may be provided with pores, such as by using porous material for providing porous dividers, or are provided with apertures, in any case allowing passage of liquid, i.e. cultivation medium, and gas. The dividers are however provided with pores or apertures small enough to prevent the packing to pass from one side of the divider to the other.

In some embodiments, the outer tubular wall is provided by an outer tubular element having a racial cross-section along a plane perpendicular to the longitudinal direction, which cross-section has the shape of a circle. The inner elongate wall is provided by an inner elongate element having a racial cross-section along a plane perpendicular to the longitudinal direction, which cross- section has the shape of a truncated circle or circular segment. In one embodiment the circle segment has a circle section and a chord. For example the height of the circle segment has the dimensions of 200 mm (Dec), 400mm (Do), 240mm (Di) and 125mm (L). The dividers are in this embodiment coplanar with the chord of the circle segment. The second closure of the two closures is provided with two connectors. The first connector is provided near the outer tubular wall. The second connector is provided near the inner elongate wall. When the recipient is only partially filled with cultivation medium, which medium has a liquid surface, the recipient may be rotated to such a position that the liquid does not contact the inner elongate wail, but remains on a given distance from the inner elongate wall. When the recipient is brought in this position, the first connector may be used to remove or provide medium to the compartment, which is not filled with packing. Gas may be removed or provided above the medium liquid surface by means of connector. By rotation of the recipient either clockwise or counter clockwise, e.g. over an angle of up to 360° or even more, the medium will pass through to one of the two dividers, more particular through the divider which will gradually be submerged in the medium. The medium will slowly flow through the packing, as the packing gradually will pass through the medium because of the rotation. Due to the rotation of the recipient, the medium will pass and flow through the complete packing according to a plug flow. A uniform contact between medium and packing throughout the annular volume will occur. Once a part of the packing has passed through the medium, the medium will gradually seep out of the packing and hence the gas in the recipient may again contact the packing, allowing the cells to grow uniformly throughout the packing.

In an alternate embodiment of the recipient, the annular volume of the inner space is provided by a plurality of annular sections, in this particular case four annular quarters. Each of the sections provides one part of the outer tubular wall by means of an outer tubular wall section. Each of the sections provides one part of the inner elongate wall by means of an inner elongate wall section. Each of the sections has two radially extending section walls. These section walls are liquid and gas impermeable. Each of the section walls is provided with mounting means allowing adjacent sections to couple one to the other. A first and a second section closure delimit and close the volume of the annular sections at the first respectively the second outer end of the outer tubular wall. The section closures together form the first respectively second closure of the annular volume of the recipient.

In some embodiments, each of the sections may further be provided with two fluid permeable dividers, dividing the inner space of each annular section in three compartments. The fluid permeable dividers, e.g. porous dividers extend from the inner elongate wall to the outer tubular wall and from the first closure to the second closure in the longitudinal direction parallel to the direction of the tubular axis. One compartment is provided with the packing. Two compartments are not provided with the packing. For each annular section, a first connector is provided near the outer tubular wall. The second connector is provided near the inner elongate wall. Each of the sections may function as an independent recipient section of the recipient, when the recipient is rotated about the axis. The packing in each of the sections is provided with medium, which is present in this section depending upon the radial position of the section.

By mounting the sections, in this embodiment four sections, a recipient with an outer tubular wall and an inner elongate wall is obtained, of which the annular volume is closed at the two outer ends of the outer tubular wall by means of two closures. Because the coupling of the sections is provided by mounting and coupling two radially extending section walls, the combination of two contiguous section walls form a liquid and gas impermeable divider extending from the inner elongate wall to the outer tubular wall and from the first closure to the second closure in the longitudinal direction parallel to the direction of the tubular axis. This embodiment has the advantage that each of the sections may be provided with different medium for a different cell culture. Also, in case of an incorrect functioning of the packing of reaction in one of the sections, only one section is to be replaced. The rotation of the recipient (or any other recipient disclosed herein) may be provided by a rotator. In one example, this rotator may include contacting the outer surface of the outer tubular wall with at least two supporting wheels of which at least one is driven.

In an alternate embodiment, each of the sections of the recipient has two radially extending section walls. These section walls are liquid and gas permeable. Each section wall comprises a number of apertures, each of these apertures finding a corresponding aperture in a second section wall of an adjacent compartment. The aperture of the first section wall may be provided with an outwardly extending rim which extends through the corresponding aperture of the second section wall. Optionally a seal is provided around the apertures in the adjacent section walls to prevent medium from leaking between the contacting walls. In alternative embodiments, flexible tubing is placed between the recipients instead of seals.

In an embodiment of the bioreactor utilizing the recipient, at least one recipient is rotatably mounted in a vessel, which may have any suitable radial cross-section such as e.g. circular or polygonal such as rectangular (optionally square). The vessel is partially filled with cultivation medium, so the liquid level optionally does not raise higher than the axis of the bioreactor, but at least contacts the inner elongate wall. The recipient is rotated about this axis, which is identical to the longitudinal axis of the recipient. The longitudinal axis of the recipient is an axis parallel to the longitudinal direction of the outer tubular wall. Optionally the outer tubular wall is provided with apertures or is made from a porous, e.g. liquid and gas permeable material. When such fluid permeable outer tubular wall is rotated in a vessel partially filled with medium, so that only part of the outer tubular wall is submerged in the medium, the packing may be provided with medium flowing into the annular volume through the outer tubular wall. Part of the medium may be dragged along with the packing when the recipient is to rotate in the vessel.

In some embodiments, at least one of the recipients comprises a magnetic element. The bioreactor further comprises a magnetic element, which co-operates with the magnetic element of the recipient. Both magnetic elements are positioned such that the rotation of the magnetic element of the bioreactor around the axis of rotation will induce the rotation of the magnetic element of the recipient, hence will rotate the complete recipient. The adjacent recipients may be mounted on a common shaft, around which they rotate. The adjacent recipients may be coupled to each other in a fixed position, so the rotation of recipient induces the rotation of the recipient as well.

In some embodiments, the recipients have a liquid and gas impermeable outer tubular wall, which has connectors for connecting the recipient to a gas or medium storage by optionally flexible tubing. The recipients may be rotated by means of a driving system for providing a bioreactor. The recipient is mounted on a rotatable shaft, which is rotatable about an axis of rotation coinciding with the axis of the recipient. The shaft is profiled and fits in a unique rotational position within the inner void of the inner elongate element. As such, by controlling the rotational position of the shaft, the position of the recipient about the axis is unambiguously defined. The driving system may further comprise a motor, such as a linear motor, or any other suitable means to precisely control the rotation of the shaft. A clamp screw or any suitable means to prevent the recipient to displace in longitudinal direction over the shaft may fix the position of the recipient on the shaft in longitudinal direction. It is understood that optionally more than one recipient may be mounted on a common shaft. The shape of the inner void of the inner elongate element and the perimeter of the shaft are chosen such that the shaft and the recipient may be mounted in a limited or even in a unique way.

In one embodiment of the recipient, the inner elongate element has a longitudinal recess in the inner wall of the inner elongate element. A ridge on the shaft fits into this recess. The recipient fits in an unambiguous way on the shaft. The ridge may be slidingly moveable in the recess. In an alternate embodiment of the recipient, the inner elongate element has two longitudinal recesses in the inner wall of the inner elongate element. Two mutually perpendicular ridges on the shaft fit into these recesses. The recipient fits in two ways on the shaft, the first position being 180° rotated about the axis relative to the second position. The ridges may be slidingly moveable in the recesses. In a further embodiment of the recipient, the inner elongate element has four longitudinal recesses, one in each of the inner walls of the inner elongate element provided by a compartment. The shaft has a substantially cross-like cross-section, comprising four mutually perpendicular ridges on the shaft. Each of the ridges fits into a recess of one of the compartments. The recipient fits in four ways on the shaft. The ridges may be slidingly moveable in the recesses.

In a further embodiment of the bioreactor recipient, the volume of the inner space is provided by a plurality of segments, in this particular case four quarters. Each of the sections provides one part of the outer tubular wall by means of an outer tubular wall part. Each of the segments provides one part of the inner elongate wall by means of an inner elongate wall part. Each of the segments has two radially extending segment walls. As an example segment has two radially extending segment walls. The cultivation medium further may fill the inner void of the inner elongate wall. Through the apertures, the cultivation medium may flow in or out of the sections, and optionally to the adjacent section. The recipient includes a body forming a longitudinal wall and ends in the form of caps. The caps may be removable, and in the connected position form a fluid- tight seal for containing any fluid within the body. Each cap may include an opening forming an inlet or outlet for receiving the culture medium, but the inlet and outlet could each be provided in the same cap as well, or in the longitudinal wall of the body. At least one or a pair of fluid-permeable structures, such as perforated partitions, are provided forming a compartment for containing any packing. The perforations in each partition may be provided in a shape and size to control the flow and residence time of the fluid in the compartment, and may be the same or different among the partitions. The packing may be provided in a manner such that it completely occupies the compartment of the recipient, and thus circumferentially contacts the inner surfaces of the wall of the body, as well as the fluid-permeable structures.

In some embodiments, the recipient may be associated with a rotary device. The device may include a pair of rollers for receiving, supporting, and rotating the recipient about the longitudinal axis of the body. The recipient may be provided with a generally cylindrical body adapted for engaging and being rotated by the rollers to help distribute any fluid (e.g., the culture medium, or any rinsing or recovery agent) through any packing present in the compartment. Tubular connectors may also be provided in association with the inlet and outlet for delivering fluid to the compartment. This may be done while the recipient is stationary or while it is rotating. In the latter case, the connectors may be connected in a manner that permits relative rotation, such as by using a rotary joint created by way of a snap-fit engagement or using a bearing. Seals, such as O-rings, may be used to help prevent any leakage and help maintain the sterile conditions desirable for cell culturing. One advantage of the recipient is the simplicity of the arrangement. For example, the recipient includes no sensors, probes, mixers, or the like, in the event it is desirable to include such structures, this is possible, and may be accomplished by connecting the recipient in a closed loop with a reservoir. The reservoir may include any number of sensors or the like for measuring one or more characteristics of the circulated fluid, and may include a single use vessel (such as a flexible bag) to avoid the need for cleaning and sterilization. A pump, such as a peristaltic pump, may also be provided for circulating the fluid through the loop.

In this embodiment, the recipient may be constructed according to any of the above details (thus forming a roller bottle), and includes an inlet and an outlet. Each of the inlet and outlet may be connected to a conduit that permits rotation of the recipient without unduly biding or twisting the conduit in a manner that does not interfere with the fluid transmission, and thus may allow for the continuous flow of fluid to and from the recipient while it is rotated. Specifically, the conduit may comprise a coiled tube having an open end for connecting to the inlet or outlet, respectively, and may at the opposite ends associate with any fluid reservoir. A suitable pumping arrangement may also be provided for moving fluid through the conduit and the recipient.

In one embodiment, the recipient may be rotated in a first direction, such as clockwise, for one or more complete rotations, using a suitable rotator (such as rollers). The number of rotations possible without binding of the conduit may vary, but it is envisioned that 2-3 rotations should be possible at a minimum. The recipient may then be rotated in a second, opposite direction for one or more complete rotations. More specifically, the rotation may be for the number of rotations in the first direction to return the coiled tube to its home position, plus a corresponding number of rotations in the second direction for so long as the coiled tube does not bind or otherwise interfere with the fluid transmission. The rotation and counter-rotation may occur continuously or intermittently, and the same is true for the delivery and recovery of fluid via the conduit. It can also be understood that, in the event the recipient includes a sensor, it may also be connected to any source of energy used for sensing. In such case, any transmission line, such as a cable, may also be coiled or spiral to accommodate the relative rotation in the manner contemplated above without twisting or binding. Again, it is desirable to place any sensors or the like in any recirculation loop, though, since this drives down the cost of the recipient.

In a further embodiment of a recipient in the form of a roller bottle, the inlet and outlet may be provided in a common wall. The inlet may also be associated with a tube positioned within a fluid permeable internal cylinder within the fixed bed, which helps to ensure the fluid introduced (gas, liquid, or both) does not simply immediately exit through the inlet. A gas may be introduced into a liquid by placing an injector, such as a rotameter, in the recirculation loop. The placement may be immediately upstream of the inlet. This manner of gas introduction in connection with liquid flow advantageously helps to reduce the incidence of foaming and improve the mass transfer rate, since the gas remains in pockets separated by the liquid. The transmission line associated with the outlet returns to the reservoir, which may be vented through a filter as shown, to avoid a vent for direct connection with the recipient.

In some embodiments, other elements may be added to the recipients and the bioreactors. As an example the inner elongate wall may be an elongate tubular, optionally cylindrical wall. The inner void of the inner tubular wall may be used to accommodate one or more sensors, such as temperature sensors, position sensors (e.g. for defining the orientation of the recipient relative to the axis of rotation), optical sensors (e.g. for generating data on the color of the cultivation medium, such as cell cultivation medium), pH-sensors, oxygen sensors (such as Dissolved Oxygen (DO)-sensors), $CO_2$~sensors, ammonia sensors or cell biomass sensors (e.g. turbidity densitometers). Such sensors may additionally or optionally be located in or on the closures. The recipient may also accommodate perfusion, continuous addition of fresh nutrient medium and the withdrawal of an equal volume of used medium, allowing the realization of cell cultivation conditions that are approximated as closely as possible to the in vivo situation. The combination of a perfusion ceil culture with e.g. an enzyme glucose biosensor allows the glucose consumption of the cell culture to be monitored continuously. It is also understood that heating elements, such as heating blankets, may be provided to the outer and optionally the inner wall for heating or maintaining the temperature of the medium and the packing in the recipient.

In an alternate embodiment, the bioreactor comprises a cell culture vessel comprising a substantially vertical and cylindrical culture vessel, although other forms can also be envisaged, for example any prismatic shape, preferably regular. The culture vessel comprises at least four zones in communication with one another. From the center of the vessel towards the outside, the vessel comprises a first zone, a third zone, second zone and a fourth zone.

In some embodiments, the culture vessel comprises medium circulation means in its bottom part. The medium circulation means are, in this preferential embodiment, composed of a magnetic device, for example a magnetic bar in rotation about a central rotation axis, real or virtual, a first end of which is housed in a top engagement means and a second end of which is housed in a bottom engagement means. The magnetic bar is driven by a rotary magnetic drive motor external to the culture vessel and which is not shown here. The circulation means comprise at least one medium inlet. The medium inlet comprises at least one first end which ends in a diversion baffle for the flow of medium. The magnetic bar functions as a centrifugal pump, that is to say the medium is sucked into a relatively central zone by the movement of the medium created by the bar and the medium is propelled outwards with respect to the central point. The medium diversion baffle guides the medium in the relatively central zone of the bar so that the medium is sucked therein and is then propelled outwards. Advantageously, the inlets are in the same plane (star configuration) and the number of inlets will be a number such that their positions will exhibit symmetry. More particularly, if three inlets are considered, it is advantageous for them each to be separated from one another by an angle of approximately 120°, if the number of inlets equal 4, the inlets will be separated from one another by an angle substantially equivalent to 90°, if the number of inlets is equal to 10, the inlets will be disposed with a separation angle approximately equal to 36°. The medium circulation means also comprises at least one medium outlet. The medium outlet is advantageously situated at the point where the medium is propelled by the centrifuge effect of the magnetic bar. Advantageously, the number of outlets will be a number such that their positions will exhibit symmetry. More particularly, if three outlets are considered it is advantageous for them each to be separated from the other by an angle of approximately 120°, if the number of outlets is equal to four, the outlets will be separated from one another by an angle substantially equivalent to 90°, if the number of outlets is equal to 10, the outlets will be disposed with a separation angle of approximately 36°. Preferably, the outlets are not situated in the same horizontal plane as the inlets. The bottom part of the culture vessel comprises at least one medium guiding means, adjacent to said at least one outlet, which guides the culture medium propelled towards to the top of the culture vessel.

In some embodiments, the first zone of the culture vessel is a substantially central zone and is a medium transfer zone. The first zone comprises a basal part and in particular embodiments optionally also a cylindrical part. The diameter of the basal part is less than the diameter of the culture vessel. The basal part is in medium communication with said at least one medium outlet of the medium circulation means. The basal part is reduced in the top part of the first zone to a cylinder with a smaller diameter than the basal part. The top cylindrical part comprises an external wall and is in direct medium communication with the basal part of said first medium transfer zone. The third zone is a medium transfer zone, external to the first medium transfer zone. The third zone also comprises a substantially basal part (in the form of a sleeve) and in particular embodiments optionally also a substantially cylindrical top part.

In some embodiments, the substantially cylindrical part of the third medium transfer zone is essentially concentric with the substantially cylindrical part of the first medium transfer zone and these two parts are in medium communication. The medium communication is achieved by means of an orifice or a tube, by overflowing (as shown in the figure) via overflow or any other possible means for achieving this communication. The second zone is a cell culture zone, with or without carriers or microcarriers. The second zone is also in the form of a sleeve, at the center of which are the first and third medium transfer zones. The second zone comprises a bottom wall and a top wall, each wall and being provided with orifices allowing a transfer of cultured medium essentially free from cells. The second culture zone is in medium communication with the relatively basal part of the third medium transfer zone by means of orifices in the bottom wall allowing the medium to pass. The fourth zone is a medium transfer zone, external to the second culture zone but internal to the culture vessel. The fourth zone is in medium communication with the second culture zone. It is also in medium communication with the medium circulation means, via said at least one inlet. The medium communication is achieved by means of an orifice or a tube, by overflowing or by any other possible means for achieving this communication.

In some embodiments, the culture device comprises a substantially cylindrical culture vessel, but other embodiments can also be envisaged, as mentioned previously, for example a substantially prismatic vessel, preferably regular. Obviously, this is also the case with the various medium and culture transfer zones. They can also be prismatic, preferably regular, any combination of shapes being possible. In this case, the term sleeve must be envisaged as an envelope with a cross-section similar to the cross-section of the prism envisaged. When the medium circulation means are in operation, the medium leaves them through said at least one outlet, when there are several of them, through the various outlets, and is diverted by the guiding means, it ends up in the substantially basal part of the first medium transfer zone. The structure of the first medium transfer zone and the output of the pump require the medium to be directed towards the substantially cylindrical part of the first medium transfer zone. When it reaches the top of the wall of the substantially cylindrical part, it overflows via overflow into the third medium transfer zone.

It is clear to the skilled in the art that, in this particular embodiment, the wall of the substantially cylindrical part of the first medium transfer zone is less high than the wall of the third medium transfer zone for reasons of efficiency and flow rate, but he will easily understand that the wall of the substantially cylindrical part of the first medium transfer zone can also be higher than the wall of the substantially cylindrical part of the third medium transfer zone. The medium is therefore subjected to the flow rate imposed by the pump and to gravity, it is directed downwards from the third medium transfer zone running down the substantially cylindrical part and reaches the substantially basal part of the third medium transfer zone. Next the flow of medium has a rising direction through a communicating vessels effect by the imposed flow rate of the pump and reaches the top of the second culture zone. The medium reaches the second culture zone from the third medium transfer zone via the orifices for the passage of medium substantially free from cells in the bottom wall of the second culture zone. As already mentioned, the medium passage orifices are sized according to the type of culture. If the culture is a culture without carrier, the wall comprising orifices will be a porous membrane where the pore size is less than the diameter of the cells. If the culture is on microcarriers or on carriers, the size of the orifices will be less than the size of the microcarriers or carriers. When the medium flow edge reaches the top of the wall of the second culture zone, it overflows into the fourth medium transfer zone. Naturally, if orifices are present or a tube, it must be understood that, when the medium flow edge reaches the orifice or tube, it flows into the fourth zone.

In one embodiment, the fourth medium transfer zone comprises an inclined wall on which the medium flows when it passes from the second zone to the fourth zone. The inclined wall preferably comprises a hydrophilic membrane in order to improve the formation of the film on said inclined wall. The film must preferably be laminar in order to prevent as far as possible the formation of foam. In order to stabilize the film, it is also possible to add additives to the culture medium in order to modify the rheological properties of the water, in particular of the culture medium, such as the additives included in the group consisting of surfactants, Pluronic F68, glycerine, quaternary ammoniums and any other additive for modifying the rheological properties of the culture medium. The hydrophilic membrane will for example be a membrane consisting of polyoxyethylene. The formation of the film on the inclined wall is an important step since it allows oxygenation on "thin film". Indeed the gaseous volume with respect to the quantity of medium in this fourth medium transfer zone is large and improves exchanges. In addition, the formation of the film on an inclined wall increases the gas-liquid contact surface area.

In some embodiments, the culture vessel preferably comprises a cover through which at least one gas inlet orifice and at least gas outlet orifice pass. The gas inlet orifice is preferably situated so as to communicate directly with the fourth medium transfer zone. In some variants, it may be preferable for the gas inlet orifice to be present on the vertical wall of the culture vessel or at the bottom of the culture vessel, that is to say the gas passes by means of an orifice through the wall of the culture vessel opposite to the cover, and for this orifice to be provided with a tube in order to end above the liquid level.

In this embodiment, the cover is fixed by fixing means to the top wall of the second culture zone. In variants, the cover can be made an integral part of the top wall of the second culture zone, this part opening when the cover of the culture vessel is raised. In this way, it is easy to take off a cell sample with or without carriers in order for example to evaluate the cell density, the structure of the cells and other physical characteristics of the cell which reflect the health of the culture. Indeed, connecting the two together makes it possible to open the culture compartment simply by raising the cover of the culture vessel. In the case of culture in suspension, it could be advantageous to connect a porous membrane to the top wall provided with orifices of the second culture zone, this assembly can improve the rigidity of the cover/membrane assembly for taking samples.

In some embodiments, the magnetic bar has the shape of a helix. The design of the magnetic device with a substantially central rotation axis will depend essentially on the volume of the culture. Indeed, for small cultures, the device sets out to be able to us a simple bar such as a magnetic chip for circulating the medium. For large volumes, the device envisages a magnetic rotor, also driven by an external motor, for example rotors like the ones used in aquariums which allow high medium circulation rates.

Some embodiments of the cell culture device can be envisaged using bubble production devices, more commonly referred to as "spargers" or "microspargers" according to the size of bubble produced. Advantageously, when bubbles will be used, the pierced end of the bubble production device, for example of the tube, will be immersed in the medium at the bottom of the fourth medium transfer zone or in the first medium transfer zone. When this type of oxygenation is chosen, it is always also possible to continue the oxygenation on thin film, which makes it possible to reduce the flow of gas and to form fewer bubbles and therefore to reduce the formation of foam. In this case, provision is also made for having two gas inlets in the cover of the culture vessel or on the vertical wall of the latter. In addition, it is also possible to envisage that the bubble production device be present solely as an SOS procedure, and used solely when necessary.

In some embodiments, the culture device also comprises a series of culture parameter sensors, for example for the dissolved oxygen partial pressure pO2, acidity pH, temperature, cloudiness, optical density, glucose, CO2, lactate, ammonium and any other parameter normally used for monitoring cell cultures. These sensors are preferably optical sensors which do not require connections between the inside of the culture vessel and the outside thereof. The preferential position of these sensors is a critical position in that it is advantageous for these to be situated close to the wall of the culture vessel, for them to be in contact with the medium and preferably in strategic positions, as in the zone through which the medium passes before it passes through the cells or just after.

In some embodiments, the cell culture device comprises a disposable bioreactor for all the reasons of simplicity and economy mentioned previously. Consequently, this is why the connections between the inside and the outside of the culture vessel have been reduced. In addition, the bioreactor comprises a particularly reliable bioreactor in which the risks of contamination are particularly low by being disposable.

An embodiment of a device also envisages a modular design which comprises a series of modules for cultures on a larger volume. For example, with this type of modular design, culture volumes of around 500 ml to 100 liters are for example envisaged, through the use of a very limited number of standard modules. In some embodiments the device provides a series of modules that can be "slipped" around the first medium transfer zone to be placed in a standard culture vessel comprising medium circulation means and a cover. In a particular variant, the device comprises a mounting system which comprises various standard modules. These standard modules are for example a circulation means module to be placed at the bottom of the assembly, one or more culture modules and a cover module. Although other means of fixing these modules can be envisaged, the modules will be clamped on one another, for example by means of rapid connectors perfectly impermeable from the liquid and gaseous point of view.

Consequently, according to the type of culture and the required volume, the user will be able to take from the stock a base module comprising the medium circulation means, he will also have to take therefrom the number of culture modules that he requires according to the required culture volume and then take a head module corresponding to the cover. Next, all these modules being packaged in sterile fashion, he will merely need to unpack them and "clip" them one above the other. The stacking can form the "disposable bioreactor" or can be placed in an appropriate vessel.

In some embodiments, the base module comprising the circulation means can be fixed to the bottom of the culture vessel can also be slid into the culture vessel in order to be able to dispose it and to use another one for another culture and thus prevent cross contaminations. These circulation means comprise a magnetic device, rotating about a central rotation axis, a first end of which is housed in a top engagement means and a second end of which is housed in a bottom engagement means. The circulation means comprise at least one medium inlet. The medium circulation means also comprise at least one medium outlet. The base module of the culture vessel comprises at least one medium guiding means adjacent to said at least one outlet, which guides the culture medium propelled towards the top of the culture vessel.

In some embodiments, the culture vessel comprises a series of culture modules which are stacked one above the other. It could also be envisaged that they be simply adjacent to one another, that is to say placed side by side. In some embodiments, the modules are clamped to one another by means of rapid connectors or clips. In addition, it may be advantageous for each module to comprise a gas or gas mixture inlet in communication with the fourth zone of each culture module. The vessel may also comprise for its part an outlet for the excess gas or gas mixture. For example, the gas inlet orifice may be present at the bottom of the culture vessel, that is to say the gas passes by means of an orifice through the wall of the culture vessel opposite to the cover and this orifice is provided with a tube in order to end above liquid level of the module. Consequently, the gaseous mixture reaches the fourth medium transfer zone of this module. The module placed above the module can also comprise a tube which enables the gaseous mixture present in the fourth zone of the culture module to communicate with the fourth zone of the module. This tube therefore advantageously passes through the bottom wall of the module.

In certain embodiments, for long duration culture, it may be advantageous to replace part of the culture medium with fresh medium or to carry out an addition of nutriment. Consequently, the base module can then comprise a nutriment inlet. Also advantageously, the culture vessel can comprise, at the medium circulation means, a medium outlet in order to prevent overflowing. In a similar manner, the culture vessel comprises a head module comprising a cover, advantageously connected to a top wall provided with medium passage orifices by fixing means in order to simplify taking samples in the module situated above. In addition, advantageously culture parameter sensors can also be provided in each culture module. It is also possible to provide sensors in only one or several culture modules at all zones or in the base module.

In some embodiments or the base module, the medium is propelled from the medium circulation means via said at least one outlet, when there are several of them, through the various outlets and is diverted by the guiding means. It ends up in the substantially basal part of the first medium transfer zone. The substantially basal part of this embodiment is a zone common to all the culture modules and, in the embodiment illustrated, is situated in the base module. This is valid whether the culture modules are stacked or juxtaposed. The structure of the first medium transfer zone and the output of the pump require the medium to be directed towards the substantially cylindrical part of the first medium transfer zone of the first module towards the substantially cylindrical part of the first medium transfer zone of the second module. In this embodiment, it is the assembling of the modules which creates a large first medium transfer zone comprising a substantially cylindrical part. When the medium reaches the top of the wall of the substantially cylindrical part of the second culture module, it overflows into the third medium transfer zone of the second culture module.

In some embodiments, the medium is therefore subjected to the flow rate imposed by the pump and to gravity, it is directed towards the bottom of the third medium transfer zone of the second culture module, flowing down the substantially cylindrical part of the second culture module, and reaches the substantially basal part of the third medium transfer zone of the second culture module. Next, the flow of medium has a rising direction through a communicating vessels effect and through the imposed flow rate of the pump and reaches the top of the second culture zone of the second culture module. The medium reaches the second zone of the second culture module from the third medium transfer zone of the second culture module via the orifices for the passages of medium substantially free from cells of the bottom wall of the second culture module. When the medium flow edge reaches the top of the external wall of the second culture zone of the second culture module, it overflows into the fourth medium transfer zone of the second culture module. Naturally, if orifices or a tube are present in this external wall of the culture zone, it is necessary to understand that, when the medium flow edge reaches the orifice or tube, it flows into the fourth zone of the second culture module. In the particularly preferential embodiment of the device, the fourth medium transfer zone of the second culture module comprises an inclined wall on which the medium flows when it passes from the second zone of the second culture module to the fourth zone of the second culture module. The inclined wall preferably comprises a hydrophilic membrane in order to improve the formation of the film on said inclined wall. The film must preferably be laminar in order to prevent as far as possible the formation of foam. In order to stabilize the film, it is also possible to add additives to the culture medium in order to modify the rheological properties of the water, as mentioned before. Next, the culture medium present in the fourth medium transfer zone of the second culture module overflows either through a tube or over the top of the wall of the fourth medium transfer zone of the second culture module into the third medium transfer zone of the first culture module.

In some embodiments, the medium is subjected to the flow rate imposed by the pump and to gravity, it is directed downwards from the third medium transfer zone of the first culture module, flowing down the substantially cylindrical part of the first culture module, and reaches the substantially basal part of the third medium transfer zone of the first culture module. Next, the flow of medium has an upward direction through a communicating vessels effect and through the flow rate imposed by the pump and reaches the top of the second culture zone of the first culture module. The medium reaches the second zone of the first culture module from the third medium transfer zone of the first culture module via the orifices for passage of medium substantially free from cells in the bottom wall of the first culture module. When the medium flow edge reaches the summit of the wall of the second culture zone of the first culture module, it overflows into the fourth medium transfer zone of the first culture module. Obviously, if orifices or a tube are present in this wall, it must be understood that, when the medium flow edge reaches the orifice or the tube, it flows into the fourth medium transfer zone of the first culture module. The fourth medium transfer zone of the first culture module can also comprise an inclined wall on which the medium flows when it passes from the second culture zone of the first culture module to the fourth medium transfer zone of the first culture module. The inclined wall is possibly provided with a hydrophilic membrane as above. Next, the medium returns to the base module and to the medium circulation means through the inlet (pipe), that is to say the culture medium present in the fourth medium transfer zone of the first culture module overflows either via a tube or over the top of the wall of the fourth medium transfer zone of the first culture module in a pipe which ends in a substantially central zone of a siphon created by said centrifugal pump which constitutes the medium circulation means.

In a variant of this embodiment, the stacked modules constitute the culture vessel. In this variant, there can exist for example three kinds of module, base modules, modules comprising the four zones, and a head module mx. The base module or basal module comprises medium circulation means and assembly means; it is designed to engage the first assembly means of a four zones module as explained above, and to constitute the bottom of the vessel. The head module is designed to engage the second assembly means of a four zones module. The four zones module engaged by the base module can be the same as that engaged by the head module or the four zones module engaged by the base module can be the first in a series of four zones modules and the one engaged by the head module is consequently the second four zones module in said series of four zones modules.

In some embodiments, all the modules comprise fixing means which makes it possible to obtain a single culture module which can be assembled both with another culture module and with the base module or the head module. These fixing means are for example two concentric circles provided with a circular seal, rapid connectors well known in the art of cell culture, a screw pitch and a serration or any other device for assembling these modules.

In this embodiment, the basal part of the base module is bond with orifices substantially tubular in shape which are orifices allowing in this case an introduction of gas or gas mixture. The gas inlet orifice is connected to a tube which ends above the level of the culture medium, enabling the gas or gas mixture to reach at least a fourth medium transfer zone of the culture device. All the ambient atmospheres of the fourth medium transfer zone are connected by similar tubes so that the gas mixture can reach the top. It is particularly advantageous in a device with modules stackable for height which can rise very high to provide a gaseous supply through the bottom of the reactor. In a variant, the basal part comprises a gas or gas mixture feed tube for bringing the gaseous substance into the zone in which the magnetic device is situated. In this way, the incoming gas is stirred by the rotation of the magnetic device and the dissolution of the oxygen is improved by the movement of the medium. The excess gas is also stirred and moves upwards again in the form of small bubbles. In addition, a recess is provided for accessing these orifices from the outside, which makes it possible to connect these orifices to a supply of gas, gas mixture, fresh medium, etc. The top part mob of the module is an element designed to be gripped by virtue of the fixing means and sealingly by virtue of the circular seal on the bottom part of the base module.

In some embodiments, the device also comprises a nutriment feed, either in a tube through the cover, or a tube through one of the walls of the device. Likewise, heating means can also be present in the first or fourth zone of the device or of a module or each four zones module. Possibly, the device can also comprise several medium circulation means, for example several centrifugal pumps. The devices enable a homogenous flow of culture medium upon entry trough the orifices in the bottom part of the second zone and consequently also during the further passage through this second zone. This in contrast to devices wherein the first zone is in direct contact with the second zone which results in a non-homogenous flow throughout the second zone. Such non-homogeneous flow results in the present of undersupplied or dead zones within the cell culture zone which are insufficiently supplied with oxygen and nutrients, and wherein cell growth and/or metabolism is far from optimal.

A particular embodiment of the devices and methods relates to devices and their use, wherein the third zone is a zone internal to said second zone and external to said first zone. The flow of the medium from the basal part of the first zone upwards via the top cylindrical part of the first zone, further downwards via the cylindrical top part of the third transfer zone to the basal part of the third zone generates the desired homogeneous flow upon entry in the bottom part of the second zone. The presence of the cylindrical parts and further allows an easy access to the medium for assaying its properties prior to entry in the second zone. The presence of a cylindrical element also prevents that a high pressure is built up in the device. The presence of cylindrical parts allows in addition the manufacture of a device comprising different modules.

Other embodiments relate to devices which are modified such in that the liquid flow through the cylindrical parts is bypassed. In these alternative embodiments of devices the third zone is a located entirely below the second zone and entirely above the first zone. In one embodiment of the device the part of the device corresponding to the cylindrical parts of the first zone and the second zone is replaced by a solid element in e.g., a plastic, glass or metal.

In another embodiment, the first zone and the second zone consist of a flattened shaped volume corresponding respectively to the basal parts and lack the cylindrical parts. With this adaptation the culture medium can equally overflow from the first zone to the third zone via the overflow. The flow of the medium created by the stirring device is rendered homogeneous by the separating wall between first zone and third zone and results in a homogeneous flow upon entry of the second zone.

In particular embodiments, the separating wall between the first zone and the third zone consist of a horizontal part as well as a of a vertical part, wherein this vertical part with the overflow has a height of about up to 5%, up to 10%, up to 20% or even up to 50% of the height of the third zone. In other particular embodiments, the vertical part of the separating wall between the first zone and the third zone is absent.

In particular embodiments the solid element is provided with channels adapted to incorporate for example a probe to measure a condition of the medium in the third zone (pH, oxygen, temperature). In other particular embodiments, solid element is provided with a channel comprising a safety pressure valve which can open when an excessive pressure is built up in the first zone and third zone. In an alternative embodiment of the device the cylindrical parts of the respectively the first zone and the third zone second zone are absent. The volume previously occupied by elements now becomes parts of the second zone resulting in more efficient use of the device resulting from the enlarged volume which is suitable for cell growth.

In order to prevent the direct inflow of medium from the first zone into the second zone without a homogenous flow distribution into the third zone, the orifices in the bottom wall of the second zone are closed at those regions which are located above an opening in the wall between the first and the third zone. The adaptation of the device by providing a closed region above the opening results in the overflow of the culture medium from the first culture medium transfer zone into the third culture medium transfer zone before it enters as a homogenous flow into the second zone.

In particular embodiments, a plurality of openings and corresponding closed regions is provided into respectively the separating wall between first zone and third zone, and into the wall between third zone and second zone. Typically such plurality of openings and corresponding closed regions are distributed symmetrically.

In an alternative embodiment of the device, the homogenous flow of the medium is achieved by providing a flow redistributing element within the third zone. Such element can have any shape suitable for an appropriate redistribution of the medium coming from the first zone to obtain a homogenous liquid flow in third zone prior to entry in the second zone. In a particular embodiment the element has the form of a set of radially extending rods with a circular, diamond or oval cross section, positioned above corresponding radially applied openings.

### *Viruses*

Certain aspects of the present disclosure relate to producing an Enterovirus A. Hand, foot, and mouth disease in humans (HFMD) is caused by several members of the human enterovirus A (HEV-A) group. Human enterovirus A belongs to the Picornaviridae family of non-enveloped, positive-sense RNA viruses, which also includes polioviruses and rhinoviruses. Members of the HEV-A group that can cause HFMD include Enterovirus 71 (EV71) and Coxsackieviruses. Accordingly, examples of suitable Enteroviruses A include, without limitation, Enterovirus 71 (EV71), Coxsackievirus A strains, including serotypes A1, A2, A4, A5, A6, A8, A9, A10, or A16, or Coxsackievirus B strains, including serotype B3 or B5 or any combination thereof. As used herein, the term "CA6" is used interchangeably with "CVA6" and "Coxsackievirus A6". As used herein, the term "CA16" is used interchangeably with "CVA16" and "Coxsackievirus A16". In some embodiments, the at least one virus may be one or more, two or more, or three viruses selected from EV71, CA6, and CA16. In some embodiments, the at least one virus may be EV71 and CA6. In some embodiments, the at least one virus may be EV71 and CA16. In some embodiments, the at least one virus may be CA6 and CA16. In some embodiments, the at least one virus may be EV71.

Accordingly, in some embodiments, an Enterovirus A of the present disclosure may be used in any of the vaccines and/or immunogenic compositions disclosed herein. For example, an Enterovirus A of the present disclosure may be used to provide one or more antigens useful for treating or preventing hand, foot, and mouth disease in a subject in need thereof and/or inducing an immune response, such as a protective immune response, against hand, foot, and mouth disease in a subject in need thereof.

An antigen of the present disclosure may be derived from an Enterovirus A of the present disclosure (e.g., an Enterovirus A produced and/or purified by the methods described herein). An antigen of the present disclosure may be any substance capable of eliciting an immune response. Examples of suitable antigens include, but are not limited to, whole virus, attenuated virus, inactivated virus, proteins, polypeptides (including active proteins and individual polypeptide epitopes within proteins), glycopolypeptides, lipopolypeptides, peptides, polysaccharides, polysaccharide conjugates, peptide and non-peptide mimics of polysaccharides and other molecules, small molecules, lipids, glycolipids, and carbohydrates.

An Enterovirus A of the present disclosure may include at least one non-human cell adaptation mutation. Adaptation mutations may be generated by adapting a virus to growth in a particular cell line. For example, a cell may be transfected with a virus and passaged such that the virus replicates and its nucleic acid mutates. Nucleic acid mutations may be point mutations, insertion mutations, or deletion mutations. Nucleic acid mutations may lead to amino acid changes within viral proteins that facilitate growth of the virus in a non-human cell. Adaptation mutations may facilitate phenotypic changes in the virus, including altered plaque size, growth kinetics, temperature sensitivity, drug resistance, virulence, and virus yield in cell culture. These adaptive mutations may be useful in vaccine manufacture by increasing the speed and yield of virus cultured in a cell line. In addition, adaptive mutations may enhance immunogenicity of viral antigens by altering the structure of immunogenic epitopes.

Accordingly, in certain embodiments, an Enterovirus A of the present disclosure may include at least one non-human cell adaptation mutation. In certain embodiments, the adaptation mutations are mutations of a viral antigen to a non-human cell. In some embodiments, the non-human cell may be a mammalian cell. Examples of non-human mammalian cells include, without limitation, those described above, such as, Vero cells (from monkey kidneys), MDBK cells, MDCK cells, ATCC CCL34 MDCK (NBL2) cells, MDCK 33016 (deposit number DSM ACC 2219 as described in WO97/37001) cells, BHK21-F cells, HKCC cells, or Chinese hamster ovary cells (CHO cells). In some embodiments, the non-human cell may be a monkey cell. In some embodiments, the monkey cell is from a Vero cell line. Examples of suitable Vero cell lines include, without limitation, WHO Vero 10-87, ATCC CCL-81, Vero 76 (ATCC Accession No. CRL-1587), or Vero C1008 (ATCC Accession No. CRL-1586).

Enteroviruses A such as EV71, CA6, and CA16 possess linear, positive sense, single-stranded RNA genomes. Each of these viral genomes encodes both structural and nonstructural polypeptides. Structural polypeptides encoded by each of these viruses include, without limitation, VP1, VP2, VP3, and VP4, which together may compose the viral capsid. Non-structural polypeptides encoded by each of these viruses include, without limitation, 2A, 2B, 2C, 3A, 3B, 3C, and 3D, which are involved in, for example, virus replication and virulence.

Accordingly, in certain embodiments, an Enterovirus A of the present disclosure may contain at least one, at least two, at least three, at least four, at least five, at least six, at least seven, at least eight, at least nine, at least 10, at least 11, at least 12, at least 13, at least 14, at least 15, at least 16, at least 17, at least 18, at least 19, at least 20, or more non-human cell adaptation mutations within one or more, two or more, three or more, four or more, five or more, six or more, seven or more, eight or more, nine or more, or ten or more viral antigens, including, without limitation, VP1, VP2, VP3, 2A, 2B, 2C, 3A, 3B, 3C, and 3D. In some embodiments, an Enterovirus A of the present disclosure includes whole, inactivated virus that may contain at least one, at least two, at least three, at least four, at least five, at least six, at least seven, at least eight, at least nine, at least 10, or more non-human cell adaptation mutations within the 5' or 3' untranslated region (UTR) of the virus.

In some embodiments, the at least one non-human cell adaptation mutation is within the VP1 polypeptide of EV71. In other embodiments, the at least one non-human cell adaptation mutation is within the VP1 polypeptide of CA6. In other embodiments, the at least one non-human cell adaptation mutation is within the VP2 polypeptide of CA6. In other embodiments, the at least one non-human cell adaptation mutation is within the VP3 polypeptide of CA6. In other embodiments, the at least one non-human cell adaptation mutation is within the 2A polypeptide of CA16. In other embodiments, the at least one non-human cell adaptation mutation is within the VP2 polypeptide of CA16. In other embodiments, the at least one non-human cell adaptation mutation is within the VP1 polypeptide of CA16. In some embodiments, antigens of the present disclosure may contain at least one non-human cell adaptation mutation within the 5' untranslated region (UTR) of an Enterovirus A of the present disclosure, including without limitation, EV71, CA6, and CA16.

In some embodiments, the at least one non-human cell adaptation mutation is within the VP1 polypeptide of EV71. The amino acid sequence of the VP1 polypeptide from an exemplary EV71 strain is set forth as:

GDRVADVIESSIGDSVSRALTQALPAPTGQNTQVSSHRLDTGEVPALQAAEIGASSNT

SDESMIETRCVLNSHSTAETTLDSFFSRAGLVGEIDLPLEGTTNPNGYANWDIDITGY

AQMRRKVELFTYMRFDAEFTFVACTPTGEVVPQLLQYMFVPPGAPKPESRESLAWQ

TATNPSVFVKLTDPPAQVSVPFMSPASAYQWFYDGYPTFGEHKQEKDLEYGACPNN

MMGTFSVRTVGSSKSKYPLVVRIYMRMKHVRAWIPRPMRNQNYLFKANPNYAGNS

IKPTGTSRNAITTL (SEQ ID NO: 1).

In some embodiments, the at least one non-human cell adaptation mutation occurs at one or more amino acid positions within the VP1 polypeptide of EV71. In some embodiments, the mutation may occur at one or more, two or more, three or more, or four amino acid positions selected from 7, 14, 145, and 282 of SEQ ID NO: 1, or at positions corresponding to positions 7, 14, 145, or 282 of SEQ ID NO: 1 when the VP1 polypeptide of EV71 is aligned to SEQ ID NO: 1 using a pairwise alignment algorithm. In some embodiments, the mutation occurs at position 7 of SEQ ID NO: 1 or at a position corresponding to position 7 of SEQ ID NO: 1 when aligned to SEQ ID NO: 1 using a pairwise alignment algorithm. In some embodiments, the mutation occurs at position 7 of SEQ ID NO: 1, or at two or more, three or more, or all four of positions 7, 14, 145, or 282 of SEQ ID NO: 1, or at position corresponding to position 7, 14, 145, or 282 of SEQ ID NO: 1 when the VP1 polypeptide of EV71 is aligned to SEQ ID NO: 1 using a pairwise alignment algorithm. In some embodiments, the mutation at position 7 is a valine to methionine substitution. In some embodiments, the mutation at position 14 is an aspartic acid to asparagine substitution. In some embodiments, the mutation at position 145 is a glutamic acid to glutamine substitution. In some embodiments, the mutation at position 282 is an asparagine to aspartic acid substitution.

In some embodiments, the at least one non-human cell adaptation mutation is within the VP1 polypeptide of CA6. The amino acid sequence of the VP1 polypeptide from an exemplary CA6 strain is set forth as:

NDPISNAIENAVSTLADTTISRVTAANTAASSHSLGTGRVPALQAAETGASSNASDEN LIETRCVMNRNGVNEASVEHFYSRAGLVGVVEVKDSGTSQDGYTVWPIDVMGFVQ QRRKLELSTYMRFDAEFTFVSNLNDSTTPGMLLQYMYVPPGAPKPDGRKSYQWQT ATNPSIFAKLSDPPPQVSVPFMSPASAYQWFYDGYPTFGEHKQATNLQYGQCPNNM MGHFAIRTVSESTTGKNVHVRVYMRIKHVRAWVPRPFRSQAYMVKNYPTYSQTISN TAADRASITTTDYEGGVPANPQRTF (SEQ ID NO: 2).

In some embodiments, the at least one non-human cell adaptation mutation occurs at one or more amino acid positions within the VP1 polypeptide of CA6. In some embodiments, the mutation may occur at one or more, two or more, three or more, or four amino acid positions selected from 46, 90, 96, and 268 of SEQ ID NO: 2, or at positions corresponding to positions 46, 90, 96, and 268 of SEQ ID NO: 2 when the VP1 polypeptide of CA6 is aligned to SEQ ID NO: 2 using a pairwise alignment algorithm. In some embodiments, the mutation occurs at one or more, two or more, three or more, or all four of positions 46, 90, 96, and 268 of SEQ ID NO: 2, or at position corresponding to position 46, 90, 96, and 268 of SEQ ID NO: 2 when the VP1 polypeptide of CA6 is aligned to SEQ ID NO: 2 using a pairwise alignment algorithm. In some embodiments, the mutation at position 46 is an alanine to valine substitution. In some embodiments, the mutation at position 90 is a glutamic acid to lysine substitution. In some embodiments, the mutation at position 96 is a threonine to alanine substitution. In some embodiments, the mutation at position 268 is a valine to isoleucine substitution.

In some embodiments, the at least one non-human cell adaptation mutation is within the VP2 polypeptide of CA6. The amino acid sequence of the VP2 polypeptide from an exemplary CA6 strain is set forth as:

SPSVEACGYSDRVAQLTVGNSTITTQEAANIVLSYGEWPGYCPSTDATAVDKPTRPD VSVNRFYTLSTKSWKTESTGWYWKFPDVLNDTGVFGQNAQFHYLYRSGFCMHVQC NASKFHQGALLVVVIPEFVVAASSPAMKPNGQGLYPDFAHTNPGKEGQVFRDPYVL DAGIPLSQALVFPHQWINLRTNNCATIIMPYVNALPFDSALNHSNFGLAVIPISPLKYC NGATTEVPITLTIAPLNSEFSGLRQAIKQ (SEQ ID NO: 3).

In some embodiments, the mutation may occur at amino acid position 144 of SEQ ID NO: 3, or at a position corresponding to position 144 of SEQ ID NO: 3 when the VP2 polypeptide of CA6 is aligned to SEQ ID NO: 3 using a pairwise alignment algorithm. In some embodiments, the mutation at position 144 is a glutamine to lysine substitution.

In some embodiments, the at least one non-human cell adaptation mutation is within the VP3 polypeptide of CA6. The amino acid sequence of the VP3 polypeptide from an exemplary CA6 strain is set forth as:

GLPTELKPGTNQFLTTDDGTSPPILPGFEPTPLIHIPGEFTSLLDLCRIETILEVNNTTGT TGVNRLLIPVRAQNNVDQLCASFQVDPGRNGPWQSTMVGQICRYYTQWSGSLKVTF MFTGSFMATGKMLIAYTPPGSAQPTTREAAMLGTHIVWDFGLQSSVTLVIPWISNTH FRAVKTGGVYDYYATGIVTIWYQTNFVVPPDTPSEANIIALGAAQENFTLKLCKDTD EIRQTAEYQ (SEQ ID NO: 4).

In some embodiments, the at least one non-human cell adaptation mutation occurs at one or more amino acid positions within the VP3 polypeptide of CA6. In some embodiments, the mutation may occur at amino acid position 102 of SEQ ID NO: 4, or at a position corresponding to position 102 of SEQ ID NO: 4 when the VP3 polypeptide of CA6 is aligned to SEQ ID NO: 4 using a pairwise alignment algorithm. In some embodiments, the mutation at position 102 is an isoleucine to valine substitution.

In some embodiments, the at least one non-human cell adaptation mutation is within the 5' UTR of CA6. The nucleic acid sequence of the 5' UTR from an exemplary CA6 strain is set forth as:

TTAAAACAGCTAGTGGGTTGCACCCACTCACAGGGCCCACTGGGCGCTAGCACA

CTGATTTCCCGGAATCCTTGTGCGCCTGTTTTATATCCCCTCCCCCATGCGCAACT

TAGAAGCAATCTACACCTTCGATCAATAGCAGGCGTGGCGCGCCAGCCATGTCTA

GATCAAGCACTTCTGTTTCCCCGGACTGAGTATCAATAAACTGCTCACGCGGTTG

AAGGAGAAAATGTTCGTTACCCGGCTAACTACTTCGAGAAACCTAGTAGCACCA

TGAAAATTGCAGAGCGTTtCGcTCAGCGcTtCCCCcGCGtAGATCAGGCTGATGAGT

CACTGCATTCCTCACGGGCGACCGTGGCAGTGGCTGCGTTGGCGGCCTGCCCATG

GGGTAACCCATGGGACGCTCTAATATGGACATGGTGTGAAGAGTCTATTGAGCT

AGTTAGTAGTCCTCCGGCCCCTGAATGCGGCTAATCCTAACTGCGGAGCACATAC

CCCCAAACCAGGGGGCGGTGTGTCGTAACGGGCAACTCTGCAGCGGAACCGACT

ACTTTGGGTGTCCGTGTTTCCTTTTATTCTTATATTGGCTGCTTATGGTGACAATT

GAAAGATTGTTACCATATAGCTATTGGATTGGCCATCCGGTGAATAACAGAGCCT

TGATATACCTTTTTGTAGGGTTTATACCACTTACTCTTCGCGTTGTTGAGACTCTA

AAGTACATTCTAATCTTGAACACTAGAAA (SEQ ID NO: 8).

In some embodiments, the at least one non-human cell adaptation mutation occurs at one or more nucleic acid positions within the 5' UTR of CA6. In some embodiments, the mutation may occur at one or more, two or more, three or more, four or more, five or more, six or more, seven or more, eight or more, or nine nucleic acid positions selected from 1, 13, 14, 15, 16, 21, 23, 34, and 40 of SEQ ID NO: 8, or at positions corresponding to positions 1, 13, 14, 15, 16, 21, 23, 34, and 40 of SEQ ID NO: 8 when the 5' UTR of CA6 is aligned to SEQ ID NO: 8 using a pairwise alignment algorithm. In some embodiments, the mutation occurs at one or more, two or more, three or more, four or more, five or more, six or more, seven or more, eight or more, or all nine of positions 1, 13, 14, 15, 16, 21, 23, 34, and 40 of SEQ ID NO: 8, or at position corresponding to position 1, 13, 14, 15, 16, 21, 23, 34, and 40 of SEQ ID NO: 8 when the 5' UTR of CA6 is aligned to SEQ ID NO: 8 using a pairwise alignment algorithm. In some embodiments, the mutation at position 1 is a thymine to guanine substitution. In some embodiments, the mutation at position 13 is a guanine to adenine substitution. In some embodiments, the mutation at position 14 is a thymine to guanine substitution. In some embodiments, the mutation at position 15 is a guanine to cytosine substitution. In some embodiments, the mutation at position 16 is a guanine to adenine substitution. In some embodiments, the mutation at position 21 is a cytosine to thymine substitution. In some embodiments, the mutation at position 23 is a cytosine to thymine substitution. In some embodiments, the mutation at position 34 is a guanine to thymine substitution. In some embodiments, the mutation at position 40 is a cytosine to guanine substitution.

In some embodiments, the at least one non-human cell adaptation mutation is within the 2A polypeptide of CA16. The amino acid sequence of the 2A polypeptide from an exemplary CA16 strain is set forth as:

GKFGQQSGAIYVGNYRVVNRHLATHNDWANLVWEDSSRDLLVSSTTAQGCDTIAR

CDCQTGIYYCSSKRKHYPVSFTKPSLIFVEASEYYPARYQSHLMLAVGHSEPGDCGGI

LRCQHGVVGIVSTGGNGLVGFADVRDLLWLDEEAMEQ (SEQ ID NO: 5).

In some embodiments, the at least one non-human cell adaptation mutation occurs at one or more amino acid positions within the 2A polypeptide of CA16. In some embodiments, the mutation may occur at amino acid position 2 of SEQ ID NO: 5, or at a position corresponding to position 2 of SEQ ID NO: 5 when the 2A polypeptide of CA16 is aligned to SEQ ID NO: 5 using a pairwise alignment algorithm. In some embodiments, the mutation at position 2 is a lysine to glutamic acid substitution.

In some embodiments, the at least one non-human cell adaptation mutation is within the VP2 polypeptide of CA16. The amino acid sequence of the VP2 polypeptide from an exemplary CA16 strain is set forth as:

SPSAEACGYSDRVAQLTIGNSTITTQEAANIVIAYGEWPEYCPDTDATAVDKPTRPDV

SVNRFFTLDTKSWAKDSKGWYWKFPDVLTEVGVFGQNAQFHYLYRSGFCVHVQCN

ASKFHQGALLVAVLPEYVLGTIAGGTGNENSHPPYATTQPGQVGAVLMHPYVLDAG

IPLSQLTVCPHQWINLRTNNCATIIVPYMNTVPFDSALNHCNFGLLVIPVVPLDFNAG

ATSEIPITVTIAPMCAEFAGLRQAVKQ (SEQ ID NO: 6).

In some embodiments, the at least one non-human cell adaptation mutation occurs at one or more amino acid positions within the VP2 polypeptide of CA16. In some embodiments, the mutation may occur at amino acid position 161 of SEQ ID NO: 6, or at a position corresponding to position 161 of SEQ ID NO: 6 when the VP2 polypeptide of CA16 is aligned to SEQ ID NO: 6 using a pairwise alignment algorithm. In some embodiments, the mutation at position 161 is a methionine to threonine substitution.

In some embodiments, the at least one non-human cell adaptation mutation is within the VP1 polypeptide of CA16. The amino acid sequence of the VP1 polypeptide from an exemplary CA16 strain is set forth as:

GDPIADMIDQTVNNQVNRSLTALQVLPTAANTEASSHRLGTGVVPALQAAETGASS

NASDKNLIETRCVLNHHSTQETAIGNFFSRAGLVSIITMPTTDTQNTDGYVNWDIDLM

GYAQLRRKCELFTYMRFDAEFTFVVAKPNGVLVPQLLQYMYVPPGAPKPTSRDSFA

WQTATNPSVFVKMTDPPAQVSVPFMSPASAYQWFYDGYPTFGEHLQANDLDYGQC

PNNMMGTFSIRTVGTEKSPHSITLRVYMRIKHVRAWIPRPLRNQPYLFKTNPNYKGN

DIKCTSTSRDKITTL (SEQ ID NO: 7).

In some embodiments, the at least one non-human cell adaptation mutation occurs at one or more amino acid positions within the VP1 polypeptide of CA16. In some embodiments, the mutation may occur at one or more, or two amino acid positions selected from 99 and 145 of SEQ ID NO: 7, or at a position corresponding to position 99 or 145 of SEQ ID NO: 7 when the VP1 polypeptide of CA16 is aligned to SEQ ID NO: 7 using a pairwise alignment algorithm. In some embodiments, the mutation at position 99 is an aspartic acid to glycine substitution. In some embodiments, the mutation at position 145 is a valine to glutamic acid substitution.

In some embodiments, the at least one non-human cell adaptation mutation is within the 5' UTR of CA16. The nucleic acid sequence of the 5' UTR from an exemplary CA16 strain is set forth as:

AGCCTGTGGGTTGTTCCCACCCACAGGGCCCAGTGGGCGCTAGCACACTGATTCT
GCGGGATCTTTGTGCGCCTGTTTTATAACCCCTTCCCTAAGCAGCAACTTAGAAG
TTTCACACAATCACGACCAGTAGTGGGCGTGGCGCGCCAGTCACGTCTTGGTCAA
GCACTTCTGTTCCCCCGGACTGAGTATCAATAGACTGCTCACGCGGTTGAAGGAG
AAAACGTTCGTTATCCGGCTAACTACTTCGAGAAACCTAGtAGCACCGTGAAAGT
TGCGGAGtGTttCGCTCAGCACTTCCCCCGTGTAGATCAGGTCGATGAGTCACTGTA
AACCCCACGGGCGACCGTGACAGTGGCTGCGTTGGCGGCCTGCCCATGGGGTAA
CCCATGGGACGCTCTAATACAGACATGGTGTGAAGAGTCTATTGAGCTAGTTAGT
AGTCCTCCGGCCCCTGAATGCGGCTAATCCTAACTGCGGAGCACGCACCCTCAAC
CCAGGGGGCGGCGTGTCGTAATGGGTAACTCTGCAGCGGAACCGACTACTTTGG
GTGTCCGTGTTTCCTTTTATTCCTTATTGGCTGCTTATGGTGACAATTGAAAAGTT
GTTACCATATAGCTATTGGATTGGCCATCCGGTGTCTAACAGAGCTATTGTTTAC
CTATTTATTGGATACGTCCCTCTTAATCTCAAGGCCATTCAAACTCTTGATTATAT
ATTGCTCCTTAACTGTAAGAAA (SEQ ID NO: 9).

In some embodiments, the at least one non-human cell adaptation mutation occurs at one or more nucleic acid positions within the 5' UTR of CA16. In some embodiments, the mutation may occur at one or more or two nucleic acid positions selected from 6 and 33 of SEQ ID NO: 9, or at positions corresponding to positions 6 or 33 of SEQ ID NO: 9 when the 5' UTR of CA16 is aligned to SEQ ID NO: 9 using a pairwise alignment algorithm. In some embodiments, the mutation occurs at both of positions 6 and 33 of SEQ ID NO: 9, or at positions corresponding to positions 6 and 33 of SEQ ID NO: 9 when the 5' UTR of CA16 is aligned to SEQ ID NO: 9 using a pairwise alignment algorithm. In some embodiments, the mutation at position 6 is a guanine to adenine substitution. In some embodiments, the mutation at position 33 is a guanine to cytosine substitution.

In the above embodiments of the present disclosure, an exemplary pairwise alignment algorithm is the Needleman-Wunsch global alignment algorithm, using default parameters (e.g. with Gap opening penalty=10.0, and with Gap extension penalty=0.5, using the EBLOSUM62 scoring matrix). This algorithm is conveniently implemented in the needle tool in the EMBOSS package.

In some embodiments, an Enterovirus A may be used in any of the vaccines and immunogenic compositions of the present disclosure. For example, the Enterovirus A of the present disclosure may be useful for treating or preventing hand, foot, and mouth disease in a subject in need thereof and/or inducing an immune response, such as a protective immune response, against hand, foot, and mouth disease in a subject in need thereof.

*Production of Antigens*

Antigens of the present disclosure for use in vaccines and/or immunogenic compositions including, without limitation, purified viruses, inactivated viruses, attenuated viruses, recombinant viruses, or purified and/or recombinant viral proteins for subunit vaccines to treat or prevent hand, foot, and mouth disease and/or induce an immune response, such as a protective immune response, against hand, foot, and mouth disease, may be produced and/or purified or otherwise isolated by any suitable method known in the art. Antigens of the present disclosure may include, without limitation, whole virus, attenuated virus, inactivated virus, proteins, polypeptides (including active proteins and individual polypeptide epitopes within proteins), glycopolypeptides, lipopolypeptides, peptides, polysaccharides, polysaccharide conjugates, peptide and non-peptide mimics of polysaccharides and other molecules, small molecules, lipids, glycolipids, and carbohydrates produced, derived, purified, and/or otherwise isolated from at least one virus that causes hand, foot, and mouth disease. For example, suitable antigens may include, without limitation, structural polypeptides such as VP1, VP2, VP3, and VP4, and non-structural polypeptides, such as 2A, 2B, 2C, 3A, 3B, 3C, and 3D from viruses such as EV71, CA6, and CA16.

Antigen of the present disclosure may be synthesized chemically or enzymatically, produced recombinantly, isolated from a natural source, or a combination of the foregoing. In certain embodiments, antigens of the present disclosure are produced, purified, isolated, and/or derived from at least one virus of the present disclosure that causes hand, foot, and mouth disease, such as EV71, CA6, and CA16. Antigens of the present disclosure may be purified, partially purified, or a crude extract. In some embodiments, antigens of the present disclosure are viruses, such as inactivated viruses, produced as described in the above section entitled "Production of Vaccines and Immunogenic Compositions."

In certain embodiments, one or more antigens of the present disclosure may be produced by culturing a non-human cell. Cell lines suitable for production of the one or more antigens of the present disclosure are preferably of mammalian origin, and include but are not limited to: VERO cells (from monkey kidneys), horse, cow (e.g. MDBK cells), sheep, dog (e.g. MDCK cells from dog kidneys, ATCC CCL34 MDCK (NBL2) or MDCK 33016, deposit number DSM ACC 2219 as described in WO97/37001), cat, and rodent (e.g. hamster cells such as BHK21-F, HKCC cells, or Chinese hamster ovary cells (CHO cells)), and may be obtained from a wide variety of developmental stages, including for example, adult, neonatal, fetal, and embryo. In certain embodiments, the cells are immortalized (e.g. PERC.6 cells, as described in WO01/38362 and WO02/40665, and as deposited under ECACC deposit number 96022940). In preferred embodiments, mammalian cells are utilized, and may be selected from and/or derived from one or more of the following non-limiting cell types: fibroblast cells (e.g. dermal, lung), endothelial cells (e.g. aortic, coronary, pulmonary, vascular, dermal microvascular, umbilical), hepatocytes, keratinocytes, immune cells (e.g. T cell, B cell, macrophage, NK, dendritic), mammary cells (e.g. epithelial), smooth muscle cells (e.g. vascular, aortic, coronary, arterial, uterine, bronchial, cervical, retinal pericytes), melanocytes, neural cells (e.g. astrocytes), prostate cells (e.g. epithelial, smooth muscle), renal cells (e.g. epithelial, mesangial, proximal tubule), skeletal cells (e.g. chondrocyte, osteoclast, osteoblast), muscle cells (e.g. myoblast, skeletal, smooth, bronchial), liver cells, retinoblasts, and stromal cells. WO97/37000 and WO97/37001 describe production of animal cells and cell lines that capable of growth in suspension and in serum free media and are useful in the production of viral antigens. In certain embodiments, the non-human cell is cultured in serum-free media.

Polypeptide antigens may be isolated from natural sources using standard methods of protein purification known in the art, including, but not limited to, liquid chromatography (e.g., high performance liquid chromatography, fast protein liquid chromatography, etc.), size exclusion chromatography, gel electrophoresis (including one-dimensional gel electrophoresis, two-dimensional gel electrophoresis), affinity chromatography, or other purification technique. In many embodiments, the antigen is a purified antigen, e.g., from about 50% to about 75% pure, from about 75% to about 85% pure, from about 85% to about 90% pure, from about 90% to about 95% pure, from about 95% to about 98% pure, from about 98% to about 99% pure, or greater than 99% pure.

One may employ solid phase peptide synthesis techniques, where such techniques are known to those of skill in the art. See Jones, The Chemical Synthesis of Peptides (Clarendon Press, Oxford) (1994). Generally, in such methods a peptide is produced through the sequential additional of activated monomeric units to a solid phase bound growing peptide chain.

Well-established recombinant DNA techniques can be employed for production of polypeptides, where, e.g., an expression construct comprising a nucleotide sequence encoding a polypeptide is introduced into an appropriate host cell (e.g., a eukaryotic host cell grown as a unicellular entity in *in vitro* cell culture, e.g., a yeast cell, an insect cell, a mammalian cell, etc.) or a prokaryotic cell (e.g., grown in *in vitro* cell culture), generating a genetically modified host cell; under appropriate culture conditions, the protein is produced by the genetically modified host cell.

Besides killed and attenuated virus immunogenic compositions, one can use a subunit immunogenic composition or other type of immunogenic composition which presents to the animal the antigenic components of hand, foot, and mouth disease virus. The antigenic component may be a protein, glycoprotein, lipid-conjugated protein or glycoprotein, a modified lipid moiety, or other viral component which, when injected into a human, stimulates an immune response in the human such that the human develops protective immunity against hand, foot, and mouth disease. For a subunit immunogenic composition, the virus can be cultured on mammalian cells, as described above. The cell culture can be homogenized and an immunogenic composition can be isolated by passage of the cell culture homogenate over the appropriate column or through the appropriate pore size filter or via centrifugation of the cell culture homogenate.

If the antigenic component is a protein, then one can isolate the nucleic acid which encodes that protein and generate an immunogenic composition that contains that isolated nucleic acid. The nucleic acid encoding the antigenic component can be placed on a plasmid downstream of a signal sequence of a eukaryotic promoter. That plasmid can contain one or more selectable markers and be transfected into an attenuated prokaryotic organism, such as Salmonella spp., Shigella spp., or other suitable bacteria. The bacteria can then be administered to the human so that the human can generate a protective immune response to the antigenic component. Alternatively, the nucleic acid encoding the antigenic component can be placed downstream of a prokaryotic promoter, have one or more selectable markers, and be transfected into an attenuated prokaryotic organism such as Salmonella spp., Shigella spp., or other suitable bacteria. The bacteria can then be administered to the eukaryotic subject for which immune response to the antigen of interest is desired. See, for example, U.S. Pat. No. 6,500,419 to Hone, et al.

For a subunit immunogenic composition, the nucleic acid encoding a proteinaceous antigenic component of a hand, foot, and mouth disease virus can be cloned into a plasmid such as those described in International Patent Application Publication Number WO 00/32047 (Galen) and International Patent Application Publication Number WO 02/083890 (Galen). Then the plasmid can be transfected into bacteria and the bacteria can produce the desired antigenic protein. One can isolate and purify the desired antigenic protein by a variety of methods described in both patent applications.

### *Virus Inactivation*

In some embodiments, an Enterovirus A virus produced and/or purified by the methods of the present disclosure is inactivated. Methods of inactivating or killing viruses to destroy their ability to infect mammalian cells are known in the art. Such methods include both chemical and physical means. Suitable means for inactivating a virus include, without limitation, treatment with an effective amount of one or more agents selected from detergents, formalin (also referred to herein as "formaldehyde"), beta-propiolactone (BPL), binary ethylamine (BEI), acetyl ethyleneimine, heat, electromagnetic radiation, x-ray radiation, gamma radiation, ultraviolet radiation (UV radiation),UV-A radiation, UV-B radiation, UV-C radiation, methylene blue, psoralen, carboxyfullerene (C60) and any combination of any thereof.

Agents for chemical inactivation and methods of chemical inactivation are well-known in the art and described herein. In some embodiments, the Enterovirus A is chemically inactivated with one or more of BPL, formalin, or BEI. In certain embodiments where the Enterovirus A is chemically inactivated with BPL, the virus may contain one or more modifications. In some embodiments, the one or more modifications may include a modified nucleic acid. In some embodiments, the modified nucleic acid is an alkylated nucleic acid. In other embodiments, the one or more modifications may include a modified polypeptide. In some embodiments, the modified polypeptide contains a modified amino acid residue including one or more of a modified cysteine, methionine, histidine, aspartic acid, glutamic acid, tyrosine, lysine, serine, and threonine. In certain embodiments where the Enterovirus A is chemically inactivated with formalin, the virus may contain one or more modifications. In some embodiments, the one or more modifications may include a modified polypeptide. In some embodiments, the one or more modifications may include a crosslinked polypeptide. In some embodiments where the Enterovirus A is chemically inactivated with formalin, the vaccine or immunogenic composition further includes formalin. In certain embodiments of the present disclosure, the Enterovirus A was inactivated with BEI. In certain embodiments where the Enterovirus A was inactivated with BEI, the virus contains one or more modifications. In some embodiments, the one or more modifications includes a modified nucleic acid. In some embodiments, the modified nucleic acid is an alkylated nucleic acid.

In some embodiments where the Enterovirus A is chemically inactivated with BEI or BPL, any residual unreacted BEI or BPL may be neutralized (i.e., hydrolyzed) with sodium thiosulfate. Generally, sodium thiosulfate is added in excess. In some embodiments, sodium thiosulfate may be added at a concentration that ranges from about 25 mM to about 100 mM, from, about 25 mM to about 75 mM, or from about 25 mM to about 50 mM. In certain embodiments, sodium thiosulfate may be added at a concentration of about 25 mM, about 26 mM, about 27 mM, about 28 mM, about 29 mM, about 30 mM, about 31 mM, about 32 mM, about 33 mM, about 34 mM, about 35 mM, about 36 mM, about 37 mM, about 38 mM, about 39 mM, or about 40 mM at a ratio of 1 part concentrated sodium thiosulfate to 20 parts of BEI. In some embodiments, the solutions may be mixed using a mixer, such as an in-line static mixer, and subsequently filtered (e.g., clarified). Generally, the pumping of the two solutions through the mixer results in complete mixing and neutralization of BEI by the sodium thiosulfate.

Certain embodiments of the present disclosure relate to a method for inactivating an Enterovirus A. In some embodiments, the method involves treating the virus preparation with an effective amount of BEI. In certain embodiments, treating with an effective amount of BEI includes, without limitation, treating with BEI in an amount that ranges from about 0.25% v/v to about 3.0% v/v. In certain embodiments, the isolated and treated virus is selected from one or more of EV71, CA6, and CA16. In certain embodiments of the method, the virus preparation is treated with BEI at a temperature that ranges from about 25°C to about 42°C. In certain embodiments of the method, the virus preparation is treated with BEI for a period of time that ranges from about 1 hour to about 10 hours. In certain embodiments, the method further involves inactivating (i.e., hydrolyzing) unreacted BEI with an effective amount of sodium thiosulfate. In some embodiments, the effective amount of sodium thiosulfate ranges from about 25 mM to about 100 mM, from, about 25 mM to about 75 mM, or from about 25 mM to about 50 mM.

In some embodiments, the method involves treating the virus preparation with an effective amount of beta-propiolactone (BPL); and, optionally, treating the virus preparation with an effective amount of formalin concurrently with or after treating the virus preparation with an effective amount of beta-propiolactone (BPL). Alternatively, in some embodiments, the method involves treating the virus preparation with an effective amount of beta-propiolactone (BPL) for a first period of time; and treating the virus preparation with an effective amount of BPL for a second period of time to completely inactivate the virus preparation. In some embodiments the first and/or second period of time ranges from about 12 hours to about 36 hours. In certain embodiments the first and/or second period of time is about 24 hours. In certain embodiments, treating with an effective amount of BPL includes, without limitation, treating with BPL in an amount that ranges from about 0.05% v/v to about 3.0% v/v, from 0.1% v/v to about 2% v/v, or about 0.1% v/v to about 1% v/v. In certain embodiments, treating with an effective amount of BPL includes, without limitation, treating with 0.05% v/v, 0.06% v/v, 0.07% v/v, 0.08% v/v, 0.09% v/v, 0.1% v/v, 0.2% v/v, 0.3% v/v, 0.4% v/v, 0.5% v/v, 0.6% v/v, 0.7% v/v, 0.8% v/v, 0.9% v/v, or 1% v/v BPL. In certain embodiments, the isolated and treated virus is selected from one or more of EV71, CA6, and CA16. In certain embodiments of the method, the virus preparation is treated with BEI at a temperature that ranges from about 2°C to about 8°C. In certain embodiments, the method involves heating the virus preparation at a temperature of 37°C for a period of time sufficient to hydrolyze the BPL. In certain embodiments, the period of time ranges from about 1 hour to about 6 hours. Alternatively, in some embodiments, the method further involves inactivating (i.e., hydrolyzing) unreacted BPL with an effective amount of sodium thiosulfate. In some embodiments, the effective amount of sodium thiosulfate ranges from about 25 mM to about 100 mM, from, about 25 mM to about 75 mM, or from about 25 mM to about 50 mM.

In some embodiments, the method involves treating the virus preparation with an effective amount of formalin; and purifying the virus preparation from the formalin. In certain embodiments, treating with an effective amount of formalin includes, without limitation, treating with formalin in an amount that ranges from about 0.05% v/v to about 3.0% v/v, from 0.1% v/v to about 2% v/v, or about 0.1% v/v to about 1% v/v. In certain embodiments, the isolated and formalin treated virus is selected from one or more of EV71, CA6, and CA16. In certain embodiments, the virus preparation is purified to a high degree from the formalin in an amount that is about 70%, about 75%, about 80%, about 85%, about 90%, about 95%, about 96%, about 97%, about 98%, about 99%, or more.

### Formulations of Vaccines and/or immunogenic compositions

Further aspects of the present disclosure relate to compositions, immunogenic compositions, and/or vaccines containing a virus (e.g., an Enterovirus A of the present disclosure) produced by the methods of the present disclosure. Such compositions, vaccines, and/or immunogenic compositions may be useful for treating or preventing hand, foot, and mouth disease in a subject in need thereof and/or inducing an immune response, such as a protective immune response, against hand, foot, and mouth disease in a subject in need thereof.

Typically, vaccines and/or immunogenic compositions of the present disclosure are prepared as injectables either as liquid solutions or suspensions; solid forms suitable for solution in, or suspension in, liquid prior to injection may also be prepared. Such preparations may also be emulsified or produced as a dry powder. The active immunogenic ingredient is often mixed with excipients which are pharmaceutically acceptable and compatible with the active ingredient. Suitable excipients are, for example, water, saline, dextrose, sucrose, glycerol, ethanol, or the like, and combinations thereof. In addition, if desired, the vaccine or immunogenic composition may contain auxiliary substances such as wetting or emulsifying agents, pH buffering agents, or adjuvants which enhance the effectiveness of the vaccine or immunogenic composition.

Vaccines or immunogenic compositions may be conventionally administered parenterally, by injection, for example, either subcutaneously, transcutaneously, intradermally, subdermally or intramuscularly. Additional formulations which are suitable for other modes of administration include suppositories and, in some cases, oral, peroral, intranasal, buccal, sublingual, intraperitoneal, intravaginal, anal and intracranial formulations. For suppositories, traditional binders and carriers may include, for example, polyalkalene glycols or triglycerides; such suppositories may be formed from mixtures containing the active ingredient in the range of 0.5% to 10%, or even 1-2%. In certain embodiments, a low melting wax, such as a mixture of fatty acid glycerides or cocoa butter is first melted and the hand, foot, and mouth disease vaccine or immunogenic composition antigens described herein are dispersed homogeneously, for example, by stirring. The molten homogeneous mixture is then poured into conveniently sized molds, allowed to cool, and to solidify.

Formulations suitable for intranasal delivery include liquids (e.g., aqueous solution for administration as an aerosol or nasal drops) and dry powders (e.g. for rapid deposition within the nasal passage). Formulations include such normally employed excipients as, for example, pharmaceutical grades of mannitol, lactose, sucrose, trehalose, xylitol, and chitosan. Mucosadhesive agents such as chitosan can be used in either liquid or powder formulations to delay mucociliary clearance of intranasally-administered formulations. Sugars such as mannitol and sucrose can be used as stability agents in liquid formulations and as stability, bulking, or powder flow and size agents in dry powder formulations. In addition, adjuvants such as monophosphoryl lipid A (MLA), or derivatives thereof, or CpG oligonucleotides can be used in both liquid and dry powder formulations as an immunostimulatory adjuvant.

Formulations suitable for oral delivery include liquids, solids, semi-solids, gels, tablets, capsules, lozenges, and the like. Formulations suitable for oral delivery include tablets, lozenges, capsules, gels, liquids, food products, beverages, nutraceuticals, and the like. Formulations include such normally employed excipients as, for example, pharmaceutical grades of mannitol, lactose, starch, magnesium stearate, sodium saccharine, cellulose, magnesium carbonate, and the like. Other hand, foot, and mouth disease vaccine and immunogenic compositions may take the form of solutions, suspensions, pills, sustained release formulations or powders and contain 10-95% of active ingredient, or 25-70%. For oral formulations, cholera toxin is an interesting formulation partner (and also a possible conjugation partner).

The hand, foot, and mouth disease vaccines and/or immunogenic compositions when formulated for vaginal administration may be in the form of pessaries, tampons, creams, gels, pastes, foams or sprays. Any of the foregoing formulations may contain agents in addition to hand, foot, and mouth disease vaccine and immunogenic composition antigens, such as carriers, known in the art to be appropriate.

In some embodiments, the hand, foot, and mouth disease vaccines and/or immunogenic compositions of the present disclosure may be formulated for systemic or localized delivery. Such formulations are well known in the art. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like. Systemic and localized routes of administration include, e.g., intradermal, topical application, intravenous, intramuscular, etc.

The vaccines and/or immunogenic compositions of the present disclosure may be administered in a manner compatible with the dosage formulation, and in such amount as will be therapeutically effective and immunogenic. The quantity to be administered depends on the subject to be treated, including, e.g., the capacity of the individual's immune system to mount an immune response, and the degree of protection desired. Suitable dosage ranges are of the order of several hundred micrograms active ingredient per vaccination with an exemplary range from about 0.1 μg to 10 μg (even though higher amounts in the 1-10 mg range are contemplated), such as in the range from about 0.1 μg to 5 μg, or even in the range from 0.6 μg to 3 μg or in the range from about 1 μg to 3 μg, or even in the range of 0.1 μg to 1 μg. In certain embodiments, the dosage can be about 0.1 μg, about 0.2 μg, about 0.3 μg, about 0.4 μg, about 0.5 μg, about 0.6 μg, about 0.7 μg, about 0.8 μg, about 0.9 μg, about 1 μg, about 1.1 μg, about 1.2 μg, about 1.3 μg, about 1.4 μg, about 1.5 μg, about 1.6 μg, about 1.7 μg, about 1.8 μg, about 1.9 μg, about 2 μg, about 2.1 μg, about 2.2 μg, about 2.3 μg, about 2.4 μg, about 2.5 μg, about 2.6 μg, about 2.7 μg, about 2.8 μg, about 2.9 μg, or about 3 μg per dose. In certain embodiments, vaccines and/or immunogenic compositions of the present disclosure may be administered in an amount of 1 μg per dose. In vaccines and/or immunogenic compositions of the present disclosure that are multivalent, for example divalent or trivalent vaccines and/or immunogenic compositions comprising antigens from two or more of EV71, CA6, and CA16, the dosage of each component is administered at an equivalent dosage ratio (i.e., 1:1 for divalent and 1:1:1 for trivalent vaccines and/or immunogenic compositions).

Suitable regimens for initial administration and booster shots are also variable but are typified by an initial administration followed by subsequent inoculations or other administrations.

The manner of application may be varied widely. Any of the conventional methods for administration of a vaccine or immunogenic composition are applicable. These include oral application on a solid physiologically acceptable base or in a physiologically acceptable dispersion, parenterally, by injection or the like. The dosage of the vaccine or immunogenic composition will depend on the route of administration and will vary according to the age of the person to be vaccinated and the formulation of the antigen. The vaccine or immunogenic composition can have a unit dosage volume of more than 0.5mL, of 0.5mL or of less than 0.5mL, as described herein. For instance, it can be administered at a volume of 0.25mL.

Delivery agents that improve mucoadhesion can also be used to improve delivery and immunogenicity especially for intranasal, oral or lung based delivery formulations. One such compound, chitosan, the N-deacetylated form of chitin, is used in many pharmaceutical formulations. It is an attractive mucoadhesive agent for intranasal vaccine delivery due to its ability to delay mucociliary clearance and allow more time for mucosal antigen uptake and processing. In addition, it can transiently open tight junctions which may enhance transepithelial transport of antigen to the NALT. In a recent human trial, a trivalent inactivated influenza vaccine administered intranasally with chitosan but without any additional adjuvant yielded seroconversion and HI titers that were only marginally lower than those obtained following intramuscular inoculation.

Chitosan can also be formulated with adjuvants that function well intranasally such as the genetically detoxified E. coli heat-labile enterotoxin mutant LTK63. This adds an immunostimulatory effect on top of the delivery and adhesion benefits imparted by chitosan resulting in enhanced mucosal and systemic responses.

Finally, it should be noted that chitosan formulations can also be prepared in a dry powder format that has been shown to improve vaccine stability and result in a further delay in mucociliary clearance over liquid formulations. This was seen in a recent human clinical trial involving an intranasal dry powder diphtheria toxoid vaccine formulated with chitosan in which the intranasal route was as effective as the traditional intramuscular route with the added benefit of secretory IgA responses. The vaccine was also very well tolerated. Intranasal dry powdered vaccines for anthrax containing chitosan and MLA, or derivatives thereof, induce stronger responses in rabbits than intramuscular inoculation and are also protective against aerosol spore challenge.

Intranasal vaccines represent an exemplary formulation as they can affect the upper and lower respiratory tracts in contrast to parenterally administered vaccines which are better at affecting the lower respiratory tract. This can be beneficial for inducing tolerance to allergen-based vaccines and inducing immunity for pathogen-based vaccines.

In addition to providing protection in both the upper and lower respiratory tracts, intranasal vaccines avoid the complications of needle inoculations and provide a means of inducing both mucosal and systemic humoral and cellular responses via interaction of particulate and/or soluble antigens with nasopharyngeal-associated lymphoid tissues (NALT).

Vaccines and/or immunogenic compositions of the present disclosure are pharmaceutically acceptable. They may include components in addition to the antigen and adjuvant e.g. they will typically include one or more pharmaceutical carrier(s) and/or excipient(s). A thorough discussion of such components is available in Gennaro (2000) Remington: The Science and Practice of Pharmacy. 20th edition, ISBN: 0683306472.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, etc.

Vaccines and/or immunogenic compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The pH of a vaccine or immunogenic composition will generally be between 5.0 and 8.1, and more typically between 6.0 and 8.0 e.g. 6.5 and 7.5, or between 7.0 and 7.8. A manufacturing process of the present disclosure may therefore include a step of adjusting the pH of the bulk vaccine prior to packaging.

The vaccine or immunogenic composition is preferably sterile. It is preferably non pyrogenic e.g. containing <1 EU (endotoxin unit, a standard measure) per dose, and preferably <0.1 EU per dose. It is preferably gluten free.

In certain embodiments, the vaccines and/or immunogenic compositions of the present disclosure may include a detergent in an effective concentration. In some embodiments, an effective amount of detergent may include without limitation, about 0.00005% v/v to about 5% v/v or about 0.0001% v/v to about 1% v/v. In certain embodiments, an effective amount of detergent is about 0.001% v/v, about 0.002% v/v, about 0.003% v/v, about 0.004% v/v, about 0.005% v/v, about 0.006% v/v, about 0.007% v/v, about 0.008% v/v, about 0.009% v/v, or about 0.01% v/v. Without wishing to be bound by theory, detergents help maintain the vaccines and/or immunogenic compositions of the present disclosure in solution and helps to prevent the vaccines and/or immunogenic compositions from aggregating.

Suitable detergents include, for example, polyoxyethylene sorbitan ester surfactant (known as 'Tweens'), octoxynol (such as octoxynol-9 (Triton X 100) or t octylphenoxypolyethoxyethanol), cetyl trimethyl ammonium bromide ('CTAB'), and sodium deoxycholate, particularly for a split or surface antigen vaccine. The detergent may be present only at trace amounts. Other residual components in trace amounts could be antibiotics (e.g. neomycin, kanamycin, polymyxin B). In some embodiments, the detergent contains polysorbate. In some embodiments, the effective concentration of detergent includes ranges from about 0.00005% v/v to about 5% v/v.

The vaccines and/or immunogenic compositions are preferably stored at between 2°C and 8°C. They should not be frozen. They should ideally be kept out of direct light. The antigen and emulsion will typically be in admixture, although they may initially be presented in the form of a kit of separate components for extemporaneous admixing. Vaccines and/or immunogenic compositions will generally be in aqueous form when administered to a subject.

*Adjuvants*

Compositions, immunogenic compositions, and/or vaccines of the present disclosure may be used in combination with one or more adjuvants. Such adjuvanted vaccines and/or immunogenic compositions of the present disclosure may be useful for treating or preventing hand, foot, and mouth disease in a subject in need thereof and/or inducing an immune response, such as a protective immune response, against hand, foot, and mouth disease in a subject in need thereof.

Various methods of achieving an adjuvant effect for vaccines are known and may be used in conjunction with the hand, foot, and mouth vaccines and/or immunogenic compositions disclosed herein. General principles and methods are detailed in "The Theory and Practical Application of Adjuvants", 1995, Duncan E. S. Stewart-Tull (ed.), John Wiley & Sons Ltd, ISBN 0-471-95170-6, and also in "Vaccines: New Generation Immunological Adjuvants", 1995, Gregoriadis G et al. (eds.), Plenum Press, New York, ISBN 0-306-45283-9.

In some embodiments, a hand, foot, and mouth vaccine or immunogenic composition includes the antigens and an adjuvant. Antigens may be in a mixture with at least one adjuvant, at a weight-based ratio of from about 10:1 to about $10^{10}$:1 antigen:adjuvant, e.g., from about 10:1 to about 100:1, from about 100:1 to about $10^3$:1, from about $10^3$:1 to about $10^4$:1, from about $10^4$:1 to about $10^5$:1, from about $10^5$:1 to about $10^6$:1, from about $10^6$:1 to about $10^7$:1, from about $10^7$:1 to about $10^8$:1, from about $10^8$:1 to about $10^9$:1, or from about $10^9$:1 to about $10^{10}$:1 antigen:adjuvant. One of skill in the art can readily determine the appropriate ratio through information regarding the adjuvant and routine experimentation to determine optimal ratios.

Exemplary adjuvants may include, but are not limited to, aluminum salts, toll-like receptor (TLR) agonists, monophosphoryl lipid A (MLA), MLA derivatives, synthetic lipid A, lipid A mimetics or analogs, cytokines, saponins, muramyl dipeptide (MDP) derivatives, CpG oligos, lipopolysaccharide (LPS) of gram-negative bacteria, polyphosphazenes, emulsions, virosomes, cochleates, poly(lactide-co-glycolides) (PLG) microparticles, poloxamer particles, microparticles, liposomes, Complete Freund's Adjuvant (CFA), and Incomplete Freund's Adjuvant (IFA). In some embodiments, the adjuvant is MLA or derivatives thereof.

In some embodiments, the adjuvant is an aluminum salt. In some embodiments, the adjuvant includes at least one of alum, aluminum phosphate, aluminum hydroxide, potassium aluminum sulfate, and Alhydrogel 85. In some embodiments, aluminum salt adjuvants of the present disclosure have been found to increase adsorption of the antigens of the HFMD vaccines and/or immunogenic compositions of the present disclosure. Accordingly, in some embodiments, at least 75%, at least 80%, at least 85%, at least 90%, at least 91%, at least 92%, at least 93%, at least 94%, at least 95%, at least 96%, at least 97%, at least 98%, at least 99%, or 100% of the antigen is adsorbed to the aluminum salt adjuvant.

Certain embodiments of the present disclosure include a method for preparing an adjuvanted hand, foot, and mouth vaccine or immunogenic composition, which involves (a) mixing the vaccine or immunogenic composition with an aluminum salt adjuvant, with the vaccine or immunogenic composition including one or more antigens from at least one virus that causes hand, foot, and mouth disease and (b) incubating the mixture under suitable conditions for a period of time that ranges from about 16 hours to about 24 hours, with at least 75%, at least 80%, at least 85%, at least 90%, at least 91%, at least 92%, at least 93%, at least 94%, at least 95%, at least 96%, at least 97%, at least 98%, at least 99%, or 100% of the antigen adsorbed to the aluminum salt adjuvant. In certain embodiments of the method, the at least one virus that causes hand, foot, and mouth disease is selected from one or more of EV71, CA6, and CA16. In some embodiments of the method, the mixture is incubated at a temperature that ranges from about 2°C to about 8°C. In some embodiments of the method, the mixture is incubated under constant mixing using any suitable mixer known in the art. In some embodiments of the method, the mixture is incubated at pH that ranges in value from about 6.5 to about 8, from about 6.8 to about 7 8, from about 6.9 to about 7.6, or from about 7 to about 7.5. In certain preferred embodiments, the mixture is incubated at a neutral pH. In some embodiments of the method, the aluminum salt adjuvant is selected from alum, aluminum phosphate, aluminum hydroxide, potassium aluminum sulfate, and Alhydrogel 85.

Monophosphoryl Lipid A (MLA), a non-toxic derivative of lipid A from Salmonella, is a potent TLR-4 agonist that has been developed as a vaccine adjuvant (Evans *et al.* 2003). In pre-clinical murine studies intranasal MLA has been shown to enhance secretory, as well as systemic, humoral responses (Baldridge *et al.* 2000; Yang *et al.* 2002). It has also been proven to be safe and effective as a vaccine adjuvant in clinical studies of greater than 120,000 patients (Baldrick *et al.*, 2002; 2004). MLA stimulates the induction of innate immunity through the TLR-4 receptor and is thus capable of eliciting nonspecific immune responses against a wide range of infectious pathogens, including both gram negative and gram positive bacteria, viruses, and parasites (Baldrick *et al.* 2004; Persing *et al.* 2002). Inclusion of MLA in intranasal formulations should provide rapid induction of innate responses, eliciting nonspecific immune responses from viral challenge while enhancing the specific responses generated by the antigenic components of the vaccine.

Accordingly, in one embodiment, the present disclosure provides a composition comprising monophosphoryl lipid A (MLA), 3 De-O-acylated monophosphoryl lipid A (3D-MLA), or a derivative thereof as an enhancer of adaptive and innate immunity. Chemically 3D-MLA is a mixture of 3 De-O-acylated monophosphoryl lipid A with 4, 5 or 6 acylated chains. A preferred form of 3 De-O-acylated monophosphoryl lipid A is disclosed in European Patent 0 689 454 B1 (SmithKline Beecham Biologicals SA). In another embodiment, the present disclosure provides a composition comprising synthetic lipid A, lipid A mimetics or analogs, such as BioMira's PET Lipid A, or synthetic derivatives designed to function like TLR-4 agonists.

Additional exemplary adjuvants include, without limitation, polypeptide adjuvants that may be readily added to the antigens described herein by co-expression with the polypeptide components or fusion with the polypeptide components to produce chimeric polypeptides. Bacterial flagellin, the major protein constituent of flagella, is an adjuvant which has received increasing attention as an adjuvant protein because of its recognition by the innate immune system by the toll-like receptor TLR5 (65). Flagellin signaling through TLR5 has effects on both innate and adaptive immune functions by inducing DC maturation and migration as well as activation of macrophages, neutrophils, and intestinal epithelial cells resulting in production of proinflammatory mediators (66-72).

TLR5 recognizes a conserved structure within flagellin monomers that is unique to this protein and is required for flagellar function, precluding its mutation in response to immunological pressure (73). The receptor is sensitive to a 100 fM concentration but does not recognize intact filaments. Flagellar disassembly into monomers is required for binding and stimulation.

As an adjuvant, flagellin has potent activity for induction of protective responses for heterologous antigens administered either parenterally or intranasally and adjuvant effects for DNA vaccines have also been reported. A Th2 bias is observed when flagellin is employed which would be appropriate for a respiratory virus such as influenza but no evidence for IgE induction in mice or monkeys has been observed. In addition, no local or systemic inflammatory responses have been reported following intranasal or systemic administration in monkeys. The Th2 character of responses elicited following use of flagellin is somewhat surprising since flagellin signals through TLR5 in a MyD88-dependent manner and all other MyD88-dependent signals through TLRs have been shown to result in a Th1 bias. Importantly, pre-existing antibodies to flagellin have no appreciable effect on adjuvant efficacy making it attractive as a multi-use adjuvant.

A common theme in many recent intranasal vaccine trials is the use of adjuvants and/or delivery systems to improve vaccine efficacy. In one such study an influenza H3 vaccine containing a genetically detoxified E. coli heat-labile enterotoxin adjuvant (LT R192G) resulted in heterosubtypic protection against H5 challenge but only following intranasal delivery. Protection was based on the induction of cross neutralizing antibodies and demonstrated important implications for the intranasal route in development of new vaccines.

Cytokines, colony-stimulating factors (e.g., GM-CSF, CSF, and the like); tumor necrosis factor; interleukin-2, -7, -12, interferons and other like growth factors, may also be used as adjuvants as they may be readily included in the hand, foot, and mouth vaccines or immunogenic compositions by admixing or fusion with the polypeptide component.

In some embodiments, the hand, foot, and mouth vaccine and immunogenic compositions disclosed herein may include other adjuvants that act through a Toll-like receptor such as a nucleic acid TLR9 ligand comprising a 5'-TCG-3' sequence; an imidazoquinoline TLR7 ligand; a substituted guanine TLR7/8 ligand; other TLR7 ligands such as Loxoribine, 7-deazadeoxyguanosine, 7-thia-8-oxodeoxyguanosine, Imiquimod (R-837), and Resiquimod (R-848).

Certain adjuvants facilitate uptake of the vaccine molecules by APCs, such as dendritic cells, and activate these. Non-limiting examples are selected from the group consisting of an immune targeting adjuvant; an immune modulating adjuvant such as a toxin, a cytokine, and a mycobacterial derivative; an oil formulation; a polymer; a micelle forming adjuvant; a saponin; an immunostimulating complex matrix (ISCOM matrix); a particle; DDA; aluminum adjuvants; DNA adjuvants; MLA; and an encapsulating adjuvant.

Additional examples of adjuvants include agents such as aluminum salts such as hydroxide or phosphate (alum), commonly used as 0.05 to 0.1 percent solution in buffered saline (see, e.g., Nicklas (1992) Res. Immunol. 143:489-493), admixture with synthetic polymers of sugars (e.g. Carbopol®) used as 0.25 percent solution, aggregation of the protein in the vaccine by heat treatment with temperatures ranging between 70° to 101°C for 30 second to 2 minute periods respectively and also aggregation by means of cross-linking agents are possible. Aggregation by reactivation with pepsin treated antibodies (Fab fragments) to albumin, mixture with bacterial cells such as C. parvum or endotoxins or lipopolysaccharide components of gram-negative bacteria, emulsion in physiologically acceptable oil vehicles such as mannide mono-oleate (Aracel A) or emulsion with 20 percent solution of a perfluorocarbon (Fluosol-DA) used as a block substitute may also be employed. Admixture with oils such as squalene and IFA may also be used.

DDA (dimethyldioctadecylammonium bromide) is an interesting candidate for an adjuvant, but also Freund's complete and incomplete adjuvants as well as quillaja saponins such as QuilA and QS21 are interesting. Further possibilities include poly[di(earboxylatophenoxy)phosphazene (PCPP) derivatives of lipopolysaccharides such as monophosphoryl lipid A (MLA), muramyl dipeptide (MDP) and threonyl muramyl dipeptide (tMDP). The lipopolysaccharide based adjuvants may also be used for producing a predominantly Th1-type response including, for example, a combination of monophosphoryl lipid A, such as 3-de-O-acylated monophosphoryl lipid A, together with an aluminum salt.

Liposome formulations are also known to confer adjuvant effects, and therefore liposome adjuvants may be used in conjunction with the hand, foot, and mouth disease vaccines and/or immunogenic compositions.

Immunostimulating complex matrix type (ISCOM® matrix) adjuvants may also be used with the hand, foot, and mouth disease vaccine antigens and immunogenic compositions, especially since it has been shown that this type of adjuvants are capable of up-regulating MHC Class II expression by APCs. An ISCOM matrix consists of (optionally fractionated) saponins (triterpenoids) from Quillaja saponaria, cholesterol, and phospholipid. When admixed with the immunogenic protein such as the hand, foot, and mouth disease vaccine or immunogenic composition antigens, the resulting particulate formulation is what is known as an ISCOM particle where the saponin may constitute 60-70% w/w, the cholesterol and phospholipid 10-15% w/w, and the protein 10-15% w/w. Details relating to composition and use of immunostimulating complexes can for example be found in the above-mentioned text-books dealing with adjuvants, but also Morein B et al., 1995, Clin. Immunother. 3: 461-475 as well as Barr I G and Mitchell G F, 1996, Immunol. and Cell Biol. 74: 8-25 provide useful instructions for the preparation of complete immunostimulating complexes.

The saponins, whether or not in the form of iscoms, that may be used in the adjuvant combinations with the hand, foot, and mouth disease vaccine antigens and immunogenic compositions disclosed herein include those derived from the bark of Quillaja Saponaria Molina, termed Quil A, and fractions thereof, described in U.S. Pat. No. 5,057,540 and "Saponins as vaccine adjuvants", Kensil, C. R., Crit Rev Ther Drug Carrier Syst, 1996, 12 (1-2):1-55; and EP 0 362 279 B1. Exemplary fractions of Quil A are QS21, QS7, and QS17.

β-Escin is another hemolytic saponins for use in the adjuvant compositions of the hand, foot, and mouth disease vaccines and/or immunogenic compositions. Escin is described in the Merck index (12th ed: entry 3737) as a mixture of saponins occurring in the seed of the horse chestnut tree, Lat: Aesculus hippocastanum. Its isolation is described by chromatography and purification (Fiedler, Arzneimittel-Forsch. 4, 213 (1953)), and by ionexchange resins (Erbring et al., U.S. Pat. No. 3,238,190). Fractions of escin have been purified and shown to be biologically active (Yoshikawa M, et al. (Chem Pharm Bull (Tokyo) 1996 August;44(8):1454-1464)). β-escin is also known as aescin.

Another hemolytic saponin for use in the hand, foot, and mouth disease vaccines and/or immunogenic compositions is Digitonin. Digitonin is described in the Merck index (12th Edition, entry 3204) as a saponin, being derived from the seeds of Digitalis purpurea and purified according to the procedure described Gisvold et al., J.Am.Pharm.Assoc., 1934, 23, 664; and Ruhenstroth-Bauer, Physiol.Chem., 1955, 301, 621. Its use is described as being a clinical reagent for cholesterol determination.

Another interesting possibility of achieving adjuvant effect is to employ the technique described in Gosselin et al., 1992. In brief, the presentation of a relevant antigen such as an antigen in a hand, foot, and mouth disease vaccines and/or immunogenic compositions of the present disclosure can be enhanced by conjugating the antigen to antibodies (or antigen binding antibody fragments) against the $F_C$ receptors on monocytes/macrophages. Especially conjugates between antigen and anti-$F_C$RI have been demonstrated to enhance immunogenicity for the purposes of vaccination. The antibody may be conjugated to the hand, foot, and mouth disease vaccine or immunogenic composition antigens after generation or as a part of the generation including by expressing as a fusion to any one of the polypeptide components of the hand, foot, and mouth disease vaccine and immunogenic composition antigens.

Other possibilities involve the use of the targeting and immune modulating substances (i.e. cytokines). In addition, synthetic inducers of cytokines such as poly I:C may also be used.

Suitable mycobacterial derivatives may be selected from the group consisting of muramyl dipeptide, complete Freund's adjuvant, RIBI, (Ribi ImmunoChem Research Inc., Hamilton, Mont.) and a diester of trehalose such as TDM and TDE.

Examples of suitable immune targeting adjuvants include CD40 ligand and CD40 antibodies or specifically binding fragments thereof (cf. the discussion above), mannose, a Fab fragment, and CTLA-4.

Examples of suitable polymer adjuvants include a carbohydrate such as dextran, PEG, starch, mannan, and mannose; a plastic polymer; and latex such as latex beads.

Yet another interesting way of modulating an immune response is to include the immunogen (optionally together with adjuvants and pharmaceutically acceptable carriers and vehicles) in a "virtual lymph node" (VLN) (a proprietary medical device developed by ImmunoTherapy, Inc., 360 Lexington Avenue, New York, N.Y. 10017-6501). The VLN (a thin tubular device) mimics the structure and function of a lymph node. Insertion of a VLN under the skin creates a site of sterile inflammation with an upsurge of cytokines and chemokines. T- and B-cells as well as APCs rapidly respond to the danger signals, home to the inflamed site and accumulate inside the porous matrix of the VLN. It has been shown that the necessary antigen dose required to mount an immune response to an antigen is reduced when using the VLN and that immune protection conferred by vaccination using a VLN surpassed conventional immunization using Ribi as an adjuvant. The technology is described briefly in Gelber C et al., 1998, "Elicitation of Robust Cellular and Humoral Immune Responses to Small Amounts of Immunogens Using a Novel Medical Device Designated the Virtual Lymph Node", in: "From the Laboratory to the Clinic, Book of Abstracts, Oct. 12-15, 1998, Seascape Resort, Aptos, Calif."

Oligonucleotides may be used as adjuvants in conjunction with the hand, foot, and mouth disease vaccine and immunogenic composition antigens and may contain two or more dinucleotide CpG motifs separated by at least three or more or even at least six or more nucleotides. CpG-containing oligonucleotides (in which the CpG dinucleotide is unmethylated) induce a predominantly Th1 response. Such oligonucleotides are well known and are described, for example, in WO 96/02555, WO 99/33488 and U.S. Pat. Nos. 6,008,200 and 5,856,462.

Such oligonucleotide adjuvants may be deoxynucleotides. In certain embodiments, the nucleotide backbone in the oligonucleotide is phosphorodithioate, or a phosphorothioate bond, although phosphodiester and other nucleotide backbones such as PNA including oligonucleotides with mixed backbone linkages may also be used. Methods for producing phosphorothioate oligonucleotides or phosphorodithioate are described in U.S. Pat. No. 5,666,153, U.S. Pat. No. 5,278,302 and WO95/26204.

Exemplary oligonucleotides have the following sequences. The sequences may contain phosphorothioate modified nucleotide backbones:

(SEQ ID NO:10) OLIGO 1: TCC ATG ACG TTC CTG ACG TT (CpG 1826);
(SEQ ID NO:11) OLIGO 2: TCT CCC AGC GTG CGC CAT (CpG 1758);
(SEQ ID NO:12) OLIGO 3: ACC GAT GAC GTC GCC GGT GAC GGC ACC ACG;
(SEQ ID NO:13) OLIGO 4: TCG TCG TTT TGT CGT TTT GTC GTT (CpG 2006); and
(SEQ ID NO:14) OLIGO 5: TCC ATG ACG TTC CTG ATG CT (CpG 1668)

Alternative CpG oligonucleotides include the above sequences with inconsequential deletions or additions thereto. The CpG oligonucleotides as adjuvants may be synthesized by any method known in the art (e.g., EP 468520). For example, such oligonucleotides may be synthesized utilizing an automated synthesizer. Such oligonucleotide adjuvants may be between 10-50 bases in length. Another adjuvant system involves the combination of a CpG-containing oligonucleotide and a saponin derivative particularly the combination of CpG and QS21 is disclosed in WO 00/09159.

Many single or multiphase emulsion systems have been described. One of skill in the art may readily adapt such emulsion systems for use with hand, foot, and mouth disease vaccine and immunogenic composition antigens so that the emulsion does not disrupt the antigen's structure. Oil in water emulsion adjuvants per se have been suggested to be useful as adjuvant compositions (EPO 399 843B), also combinations of oil in water emulsions and other active agents have been described as adjuvants for vaccines (WO 95/17210; WO 98/56414; WO 99/12565; WO 99/11241). Other oil emulsion adjuvants have been described, such as water in oil emulsions (U.S. Pat. No. 5,422,109; EP 0 480 982 B2) and water in oil in water emulsions (U.S. Pat. No. 5,424,067; EP 0 480 981 B).

The oil emulsion adjuvants for use with the hand, foot, and mouth disease vaccines and/or immunogenic compositions described herein may be natural or synthetic, and may be mineral or organic. Examples of mineral and organic oils will be readily apparent to one skilled in the art.

In order for any oil in water composition to be suitable for human administration, the oil phase of the emulsion system may include a metabolizable oil. The meaning of the term metabolizable oil is well known in the art. Metabolizable can be defined as "being capable of being transformed by metabolism" (Dorland's Illustrated Medical Dictionary, W.B. Sanders Company, 25th edition (1974)). The oil may be any vegetable oil, fish oil, animal oil or synthetic oil, which is not toxic to the recipient and is capable of being transformed by metabolism. Nuts (such as peanut oil), seeds, and grains are common sources of vegetable oils. Synthetic oils may also be used and can include commercially available oils such as NEOBEE® and others. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and may be used with the hand, foot, and mouth disease vaccine and immunogenic composition antigens. Squalene is a metabolizable oil virtue of the fact that it is an intermediate in the biosynthesis of cholesterol (Merck index, 10th Edition, entry no.8619).

Exemplary oil emulsions are oil in water emulsions, and in particular squalene in water emulsions.

In addition, the oil emulsion adjuvants for use with the hand, foot, and mouth disease vaccine and immunogenic composition antigens may include an antioxidant, such as the oil $\alpha$-tocopherol (vitamin E, EP 0 382 271 B1).

WO 95/17210 and WO 99/11241 disclose emulsion adjuvants based on squalene, $\alpha$-tocopherol, and TWEEN 80 (TM), optionally formulated with the immunostimulants QS21 and/or 3D-MLA. WO 99/12565 discloses an improvement to these squalene emulsions with the addition of a sterol into the oil phase. Additionally, a triglyceride, such as tricaprylin ($C_{27}H_{50}O_6$), may be added to the oil phase in order to stabilize the emulsion (WO 98/56414).

The size of the oil droplets found within the stable oil in water emulsion may be less than 1 micron, may be in the range of substantially 30-600 nm, substantially around 30-500 nm in diameter, or substantially 150-500 nm in diameter, and in particular about 150 nm in diameter as measured by photon correlation spectroscopy. In this regard, 80% of the oil droplets by number may be within these ranges, more than 90% or more than 95% of the oil droplets by number are within the defined size ranges. The amounts of the components present in oil emulsions are conventionally in the range of from 2 to 10% oil, such as squalene; and when present, from 2 to 10% alpha tocopherol; and from 0.3 to 3% surfactant, such as polyoxyethylene sorbitan monooleate. The ratio of oil: alpha tocopherol may be equal or less than 1 as this provides a more stable emulsion. SPAN 85 (TM) may also be present at a level of about 1%. In some cases it may be advantageous that the hand, foot, and mouth disease vaccines and/or immunogenic compositions disclosed herein will further contain a stabilizer.

The method of producing oil in water emulsions is well known to one skilled in the art. Commonly, the method includes the step of mixing the oil phase with a surfactant such as a PBS/TWEEN80® solution, followed by homogenization using a homogenizer, it would be clear to one skilled in the art that a method comprising passing the mixture twice through a syringe needle would be suitable for homogenizing small volumes of liquid. Equally, the emulsification process in microfluidizer (M110S microfluidics machine, maximum of 50 passes, for a period of 2 minutes at maximum pressure input of 6 bar (output pressure of about 850 bar)) could be adapted by one skilled in the art to produce smaller or larger volumes of emulsion. This adaptation could be achieved by routine experimentation comprising the measurement of the resultant emulsion until a preparation was achieved with oil droplets of the required diameter.

Alternatively the hand, foot, and mouth disease vaccines and/or immunogenic compositions may be combined with vaccine vehicles composed of chitosan (as described above) or other polycationic polymers, polylactide and polylactide-coglycolide particles, poly-N-acetyl glucosamine-based polymer matrix, particles composed of polysaccharides or chemically modified polysaccharides, liposomes and lipid-based particles, particles composed of glycerol monoesters, etc. The saponins may also be formulated in the presence of cholesterol to form particulate structures such as liposomes or ISCOMs. Furthermore, the saponins may be formulated together with a polyoxyethylene ether or ester, in either a non-particulate solution or suspension, or in a particulate structure such as a paucilamelar liposome or ISCOM.

Additional illustrative adjuvants for use in the hand, foot, and mouth disease vaccines and/or immunogenic compositions as described herein include SAF (Chiron, Calif., United States), MF-59 (Chiron, see, e.g., Granoff et al. (1997) Infect Immun. 65 (5):1710-1715), the SBAS series of adjuvants (e.g., SB-AS2 (an oil-in-water emulsion containing MLA and QS21); SBAS-4 (adjuvant system containing alum and MLA), available from SmithKline Beecham, Rixensart, Belgium), Detox (Enhanzyn®) (GlaxoSmithKline), RC-512, RC-522, RC-527, RC-529, RC-544, and RC-560 (Glaxo-SmithKline) and other aminoalkyl glucosaminide 4-phosphates (AGPs), such as those described in pending U.S. patent application Ser. Nos. 08/853,826 and 09/074,720.

Other examples of adjuvants include, but are not limited to, Hunter's TiterMax® adjuvants (CytRx Corp., Norcross, Ga.); Gerbu adjuvants (Gerbu Biotechnik GmbH, Gaiberg, Germany); nitrocellulose (Nilsson and Larsson (1992) Res. Immunol. 143:553-557); alum (e.g., aluminum hydroxide, aluminum phosphate) emulsion based formulations including mineral oil, non-mineral oil, water-in-oil or oil-in-water emulsions, such as the Seppic ISA series of Montamide adjuvants (e.g., ISA-51, ISA-57, ISA-720, ISA-151, etc.; Seppic, Paris, France); and PROVAX® (IDEC Pharmaceuticals); OM-174 (a glucosamine disaccharide related to lipid A); Leishmania elongation factor; non-ionic block copolymers that form micelles such as CRL 1005; and Syntex Adjuvant Formulation. See, e.g., O'Hagan et al. (2001) Biomol Eng. 18(3):69-85; and "Vaccine Adjuvants: Preparation Methods and Research Protocols" D. O'Hagan, ed. (2000) Humana Press.

Other exemplary adjuvants include adjuvant molecules of the general formula:

$$HO(CH_2CH_2O)_n\text{-}A\text{-}R, \qquad (I)$$

where, n is 1-50, A is a bond or --C(O)--, R is $C_{1-50}$ alkyl or Phenyl $C_{1-50}$ alkyl.

One embodiment consists of a vaccine formulation comprising a polyoxyethylene ether of general formula (I), where n is between 1 and 50, 4-24, or 9; the R component is $C_{1-50}$, $C_4$-$C_{20}$ alkyl, or $C_{12}$ alkyl, and A is a bond. The concentration of the polyoxyethylene ethers should be in the range 0.1-20%, from 0.1-10%, or in the range 0.1-1%. Exemplary polyoxyethylene ethers are selected from the following group: polyoxyethylene-9-lauryl ether, polyoxyethylene-9-steoryl ether, polyoxyethylene-8-steoryl ether, polyoxyethylene-4-lauryl ether, polyoxyethylene-35-lauryl ether, and polyoxyethylene-23-lauryl ether. Polyoxyethylene ethers such as polyoxyethylene lauryl ether are described in the Merck index (12th edition: entry 7717). These adjuvant molecules are described in WO 99/52549.

The polyoxyethylene ether according to the general formula (I) above may, if desired, be combined with another adjuvant. For example, an adjuvant combination may include the CpG as described above.

Further examples of suitable pharmaceutically acceptable excipients for use with the hand, foot, and mouth disease vaccines and/or immunogenic compositions disclosed herein include water, phosphate buffered saline, isotonic buffer solutions.

Further aspects of the present disclosure relate to methods for using vaccines and/or or immunogenic compositions of the present disclosure containing one or more antigens from at least one virus that causes hand, foot and mouth disease to treat or prevent hand, foot, and mouth disease in a subject in need thereof and/or to induce an immune response to hand, foot, and mouth disease in a subject in need thereof. In some embodiments, the present disclosure relates to methods for treating or preventing hand, foot, and mouth disease in a subject in need thereof by administering to the subject a therapeutically effective amount of a vaccine and/or or immunogenic composition of the present disclosure containing one or more antigens from at least one virus that causes hand, foot and mouth disease. In some embodiments, the present disclosure relates to methods for inducing an immune response to hand, foot, and mouth disease in a subject in need thereof by administering to the subject a therapeutically effective amount of a vaccine and/or or immunogenic composition of the present disclosure containing one or more antigens from at least one virus that causes hand, foot and mouth disease.

In some embodiments, the protective immune response includes an immune response against one or more of EV71, CA6, and CA16. In some embodiments, the protective immune response includes an immune response against one or more EV71 viral genotypes such as B4, C2, C4, and C5.

The hand, foot, and mouth disease vaccines and/or immunogenic compositions disclosed herein may be used to protect or treat a mammal or bird susceptible to, or suffering from a viral infection, by means of administering the vaccine by intranasal, peroral, oral, buccal, sublingual, intramuscular, intraperitoneal, intradermal, transdermal, subdermal, intravaginal, anal, intracranial, intravenous, transcutaneous, or subcutaneous administration. Methods of systemic administration of the vaccines and/or immunogenic compositions of the present disclosure may include conventional syringes and needles, or devices designed for ballistic delivery of solid vaccines (WO 99/27961), or needleless pressure liquid jet device (U.S. Pat. No. 4,596,556; U.S. Pat. No. 5,993,412), or transdermal patches (WO 97/48440; WO 98/28037). The hand, foot, and mouth disease vaccines and/or immunogenic compositions of the present disclosure may also be applied to the skin (transdermal or transcutaneous delivery WO 98/20734; WO 98/28037). The hand, foot, and mouth disease vaccines and/or immunogenic compositions of the present disclosure therefore may include a delivery device for systemic administration, pre-filled with the hand, foot, and mouth disease vaccine or immunogenic compositions. Accordingly there is provided methods for treating or preventing hand, foot, and mouth disease and for inducing an immune response in a subject such as a mammal or bird, including the step of administering a vaccine or immunogenic composition of the present disclosure and optionally including an adjuvant and/or a carrier, to the subject, where the vaccine or immunogenic composition is administered via the parenteral or systemic route.

The vaccines and/or immunogenic compositions of the present disclosure may be used to protect or treat a mammal or bird susceptible to, or suffering from a viral infection, by means of administering the vaccine or immunogenic composition via a mucosal route, such as the oral/alimentary or nasal route. Alternative mucosal routes are intravaginal and intra-rectal. The mucosal route of administration may be via the nasal route, termed intranasal vaccination. Methods of intranasal vaccination are well known in the art, including the administration of a droplet, spray, or dry powdered form of the vaccine into the nasopharynx of the individual to be immunized. Nebulized or aerosolized vaccine formulations are potential forms of the hand, foot, and mouth disease vaccines and/or immunogenic compositions disclosed herein. Enteric formulations such as gastro resistant capsules and granules for oral administration, suppositories for rectal or vaginal administration are also formulations of the vaccines and/or immunogenic compositions of the present disclosure.

The hand, foot, and mouth disease vaccines and/or immunogenic compositions of the present disclosure may also be administered via the oral route. In such cases the pharmaceutically acceptable excipient may also include alkaline buffers, or enteric capsules or micro granules. The hand, foot, and mouth disease vaccines and/or immunogenic compositions of the present disclosure may also be administered by the vaginal route. In such cases, the pharmaceutically acceptable excipients may also include emulsifiers, polymers such as CARBOPOL®, and other known stabilizers of vaginal creams and suppositories. The hand, foot, and mouth disease vaccines and/or immunogenic compositions may also be administered by the rectal route. In such cases the excipients may also include waxes and polymers known in the art for forming rectal suppositories.

In some embodiments, the administering step includes one or more administrations. Administration can be by a single dose schedule or a multiple dose (prime-boost) schedule. In a multiple dose schedule the various doses may be given by the same or different routes *e.g.* a parenteral prime and mucosal boost, a mucosal prime and parenteral boost, *etc.* Typically they will be given by the same route. Multiple doses will typically be administered at least 1 week apart *(e.g.* about 2 weeks, about 3 weeks, about 4 weeks, about 6 weeks, about 8 weeks, about 12 weeks, about 16 weeks, *etc.).* Giving two doses separated by from 25-30 days *(e.g.* 28 days) is particularly useful.

The methods of the present disclosure include administration of a therapeutically effective amount or an immunogenic amount of the vaccines and/or immunogenic compositions of the present disclosure. A therapeutically effective amount or an immunogenic amount may be an amount of the vaccines and/or immunogenic compositions of the present disclosure that will induce a protective immunological response in the uninfected, infected or unexposed subject to which it is administered. Such a response will generally result in the development in the subject of a secretory, cellular and/or antibody-mediated immune response to the vaccine. Usually, such a response includes but is not limited to one or more of the following effects; the production of antibodies from any of the immunological classes, such as immunoglobulins A, D, E, G or M; the proliferation of B and T lymphocytes; the provision of activation, growth and differentiation signals to immunological cells; expansion of helper T cell, suppressor T cell, and/or cytotoxic T cell.

Preferably, the therapeutically effective amount or immunogenic amount is sufficient to bring about treatment or prevention of disease symptoms. The exact amount necessary will vary depending on the subject being treated; the age and general condition of the subject to be treated; the capacity of the subject's immune system to synthesize antibodies; the degree of protection desired; the severity of the condition being treated; the particular hand, foot, and mouth disease antigen polypeptide selected and its mode of administration, among other factors. An appropriate therapeutically effective amount or immunogenic amount can be readily determined by one of skill in the art. A therapeutically effective amount or immunogenic amount will fall in a relatively broad range that can be determined through routine trials.

The present disclosure will be more fully understood by reference to the following Examples. They should not, however, be construed as limiting any aspect or scope of the present disclosure in any way.

EXAMPLES

**Example 1: Cell growth evaluation in iCELLis NANO**

Vero cell line growth in the iCELLis NANO bio-reactor was evaluated.

Methods

*Cell and nucleus counting*

For the iCELLis NANO, cells were counted using the nucleus count method, whereas for the Cell-Stacks, cells were counted using the Trypan blue method.

*Glucose/lactate measurement*

Lactate was measured using a ScoutTM lactometer. Glucose was measured using an ACCU- CHEK Aviva Plus system.

*Vero resuscitation*

Vero cell resuscitation was performed. A 1-ml vial of Vero cells was quickly thawed (less than 3 minutes), wiped with IsaSept, and mixed with 19 mL of pre-warmed DMEM D1145 supplemented with 10% FBS and 2 mM glutamine, in a T-75 flask. An aliquot (0.4 ml) was taken for cell count and viability and the T-75 flask was placed at 37°C in a $CO_2$ (5%) incubator. The next day, the spent medium was discarded and replaced with fresh medium (20 ml) and the culture was continued.

*Culture and passage in T-175 flasks*

Vero cell culture and passage were performed. T-175 were seeded with 2.6 E6 cells (at 0.015 E6 cells/$cm^2$) in a working volume of 55 mL (vol./surf. = 0.31mL/$cm^2$) and cultured at 37°C in a CO2 (5%) incubator until confluence was reached. For passage, the T-175 was rinsed twice with 25 ml of DPBS and cells were trypsinized with 4 ml TrypLE for 5 minutes at 37°C in a CO2 (5%) incubator. 21 mL of complete medium was added and an aliquot was taken for cell count.

*Culture and passage in Cell Stacks*

Vero cell culture and passage were performed. Cell-Stacks were seeded with 9.5 E6 cells/plate (at 0.015 E6 cells/$cm^2$) in a working volume of 200 mL/plateau (vol./surf. = 0.31mL/$cm^2$) and cultured at 37°C in a CO2 (5%) incubator until confluence was reached. For passage, the Cell-Stack was rinsed twice with 100 ml of DPBS/plateau and cells were trypsinized with 10 ml TrypLE/plateau for 5 minutes at 37°C in a CO2 (5%) incubator. 50 mL/plateau of complete medium was added, cells were collected and a aliquot was taken for cell count.

*Infection of control Cell Stack*

The culture medium was removed and the Cell Stack (CS) was rinsed twice with 100 ml DPBS/plateau. The infection medium (200 mL/plateau; containing the virus) was filled in the CS, and the infection was pursued under 5 % CO2 at 37°C.

*Culture in NANO*

The NANO was assembled and prefilled with 600 ml culture medium and the regulations were started. Once the set-point temperature (37°C) and pH (7.2-7.4) were reached the NANO was seeded. For seeding, the desired number of cells was re-suspended in 200 ml culture medium in a 1 L bottle and the seed was loaded into the bio-reactor using the sampling loop. The sampling loop was rinsed by circulating the medium back and forward in the bottle. The recirculation loop was activated 4 hours to 16 hours (overnight) after seeding to allow optimal attachment of cells to the carriers. Monitoring (glucose and lactate measurement, check of the culture parameters) of the NANO was performed on a daily basis.

Prior to infection, the bioreactor was rinsed twice with DPBS. The recirculation pump and all regulations were stopped and the culture medium (800 mL) was removed from the NANO. Then 800 ml of DPBS was filled in the bioreactor, heating and stirring were started and rinsing was allowed to proceed for 5 min. DPBS was discarded and the rinsing was repeated. For infection, the desired amount of virus particle was mixed with 800 mL of the infection medium. The infection medium (containing virus) was filled in the bioreactor and the regulations were started. A recirculation bottle containing the remaining infection medium (without virus) was connected to the circulation loop and started the next day. The controller set-points utilized during the NANO culture are summarized in Table 1 below.

**Table 1:** Controller set-points during iCELLis NANO culture

| | iCELLis NANO bioreactor |
|---|---|
| pH | |
| pH Setpoint | 7.40 |
| DO | |
| DO Setpoint | 50 % |
| Temperature | |
| T° Setpoint | 37°C |
| Stirring | |
| Stirring speed | 600 rpm / 40% |

*TCID50 Tests*

TCID50 tests were performed.

*Evaluating Vero cell growth in iCELLis NANO*

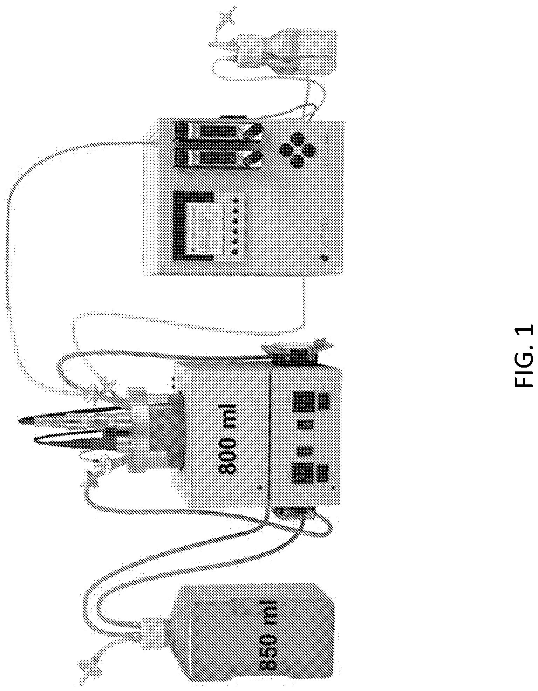

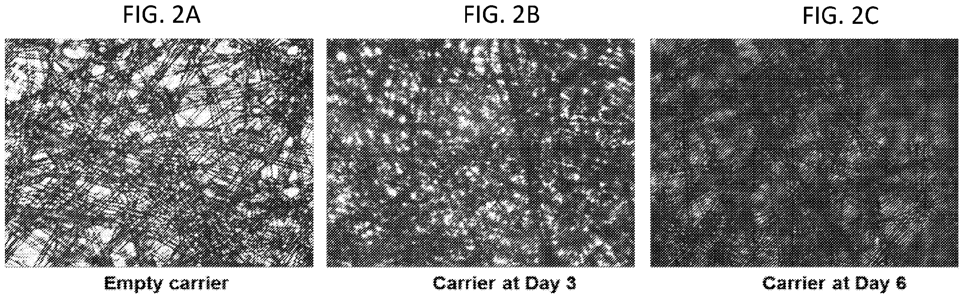

The culture in the bio-reactor was performed using a 40 mL fixed bed (2-cm bed height) Compaction 1X NANO. The bio-reactor was inoculated with a cell density of $0.015E^6$ cells/$cm^2$ in a running volume of 800 mL. A bottle containing the remaining 850 mL was connected through a recirculation loop to the bio-reactor to reach a total culture volume of 1,650 mL. The Advanced controller was programmed to maintain T° at 37°C, pH at 7.4 (by $CO_2$ or NaOH injection), DO above 50% (by air/$O_2$ injection) and agitation at 600 rpm (corresponding to a falling film speed of 2 cm/sec). A MasterFlex pump mounted with EasyLoad heads was used for medium recirculation between the NANO and the Recirculation bottle with a speed of 20 rpm (**FIG. 1**). Temperature, pH and DO were recorded using the Advanced controller. On Day 3 and 6, a full medium replacement was made and the culture was stopped at Day 8. Carriers were samples out at different time points and colored with Cyrstal Violet (**FIG. 2A to 2C**). As controls, a Cell-Stack 10 (CS-10) and a Cell-Stack 2 (CS-2) were cultured (using the same cell density at inoculation and medium volume to surface ratio) for 3 and 4 days, respectively, without medium replacement and cells were trypsinized and counted.

Results

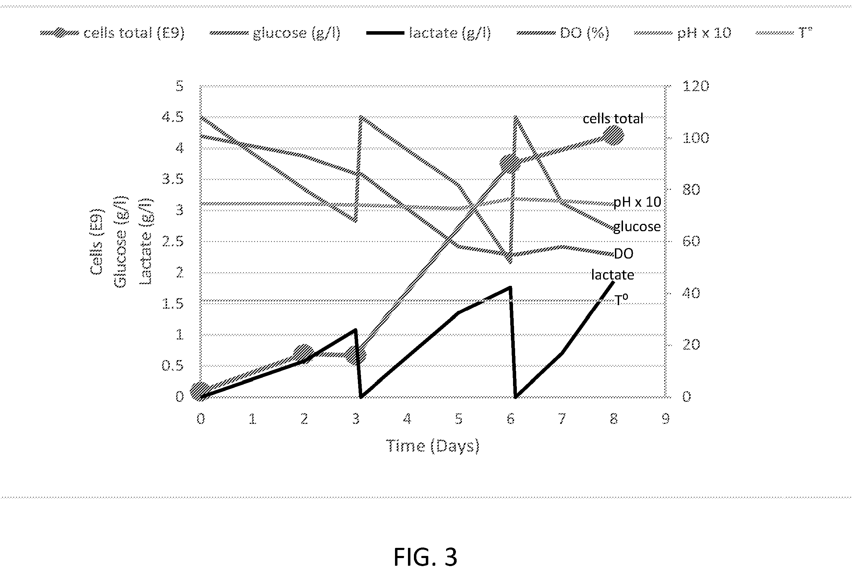

A continuous glucose consumption and lactate production was observed throughout the culture. Medium replacements were performed at Day 3 and 6 to avoid glucose shortage and lactate accumulation above the critical concentration of 20-25 mM. Oxygen consumption increased over time and dissolved oxygen (DO) was maintained above the set-point of 50% by automated injection of air/oxygen. Temperature and pH remained stable (**FIG. 3**).

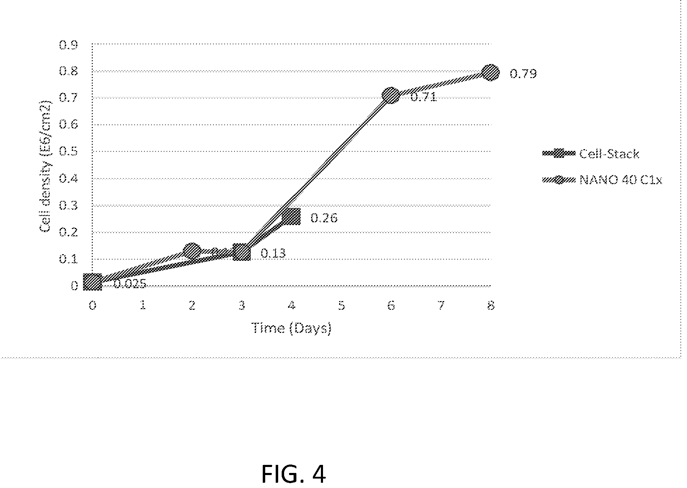

The maximal cell density reached on Day 8 was ~ 0.8E6 cells/$cm^2$. In control Cell-Stacks, cell densities of 0.13 and 0.26 $E6/cm^2$ were reached at Day 3 and 4, respectively (**FIG. 4**). These results indicated that in the iCELLis NANO, Vero cells achieved a more than 3-fold higher density compared to Cell Stack. One NANO 40 mL fixed bed compaction 1X produced as many cells as three CS-10s.

For culture medium consumption, in the iCELLis NANO, a total of approx. 5 liters were used to produce 4.2 E9 cells, indicating that 1 ml allowed the production of 0.85 E6 cells. By comparison, in Cell-Stack plates, 1 ml of culture medium produced from 0.42 to 0.82E6 cells. Therefore, the higher cell density observed in the NANO was obtained with similar medium volume consumption per cell.

**Example 2: EV71 infection evaluation in iCELLis NANO at a cellular density of 0.52 E6 cells/$cm^2$**

The kinetics and yields of EV71 virus production by Vero cells grown in the iCELLis NANO was evaluated. A 40 ml fixed-bed Compaction 1 X bio-reactor was used and EV71 infection was performed at a cellular density of 0.52 $E^6$ cells/$cm^2$.

Methods

The NANO was seeded as described in Example 1 and the total culture volume used was 1,650 mL. At Day 3 the culture medium was fully replaced. On Day 6, cell density reached 0.52 E6 cells/cm. A previously characterized Vero-adapted EV71 viral strain was used. Amino acid mutations in the passage 8 (P8) Vero-adapted EV71 strain are shown in Table 2.

**Table 2:** Amino acid mutations in Vero-adapted EV71.

|  | Amino Acid Sequence | |
|---|---|---|
|  | EV71 P0 | EV71 P8 -Adapted strain |
| VP1 | 7-V | 7-M |
|  | 14-D | 14-N |
|  | 145-E | 145-Q |
|  | 282-N | 282-D |

The bio- reactor was rinsed twice with DPBS and infection with EV71 was started as described in Example 1 using DMEM D1145 supplemented with 2 mM glutamine but devoid of FBS. Given the fact that the cell density in the bio-reactor was twice the cell density of the control Cell-Stack, twice the volume of infection medium was used (3,300 mL, i.e. Vol./surf. ratio of 0.62 mL/cm2).

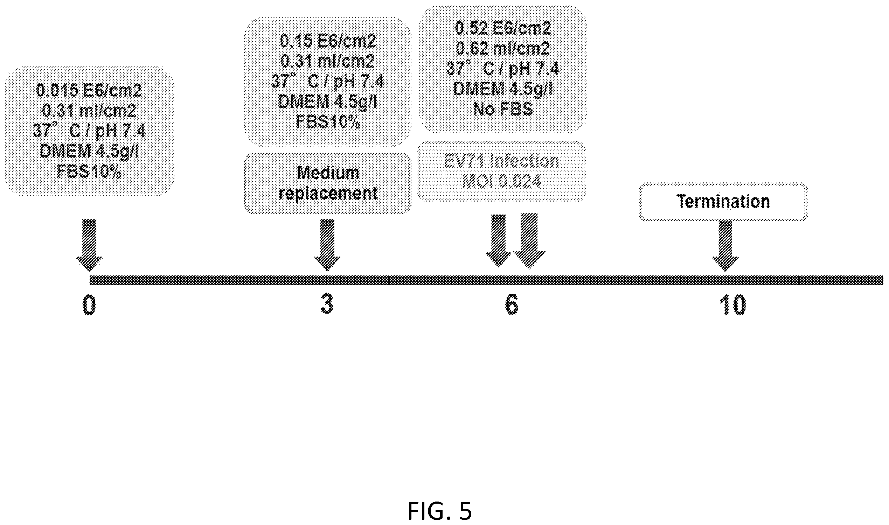

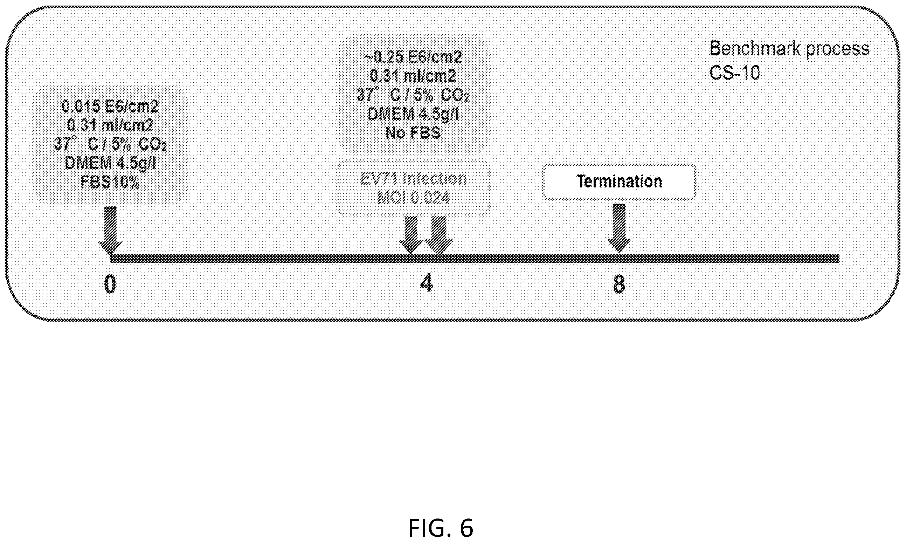

As a control, a Cell-Stack 10 (6360 cm$^2$) was seeded using the same inoculation density and cells were allowed to growth at 37°C in a $CO_2$(5%) incubator. After 4 days (i.e. on Day 3), culture medium was discarded and the Cell-Stack was rinsed twice with DPBS and EV71 infection was performed using a MOI of 0.024. The infection medium used was identical to the culture medium but devoid of FBS. Composition of the medium used for Vero cell growth and infection is summarized in Table 3. A constant Vol./Surf. ratio of 0.31 mL/cm$^2$ was used for the control experiment. Schematics of the EV71 iCELLis NANO and control Cell-Stack cultures are shown in **FIG. 5** and **FIG. 6**, respectively.

**Table 3:** Composition of media used during Vero cell growth and EV71 infection.

|  | Medium used for Vero cells **growth** | Medium used for Vero cells **infection** |
|---|---|---|
| EV71 | DMEM #1145 (Sigma) 4.5 g/L glucose 2 mM glutamine 10 % FBS #10099-141 (Gibco) | DMEM #1145 (Sigma) 4.5 g/L glucose 2 mM glutamine |

Results

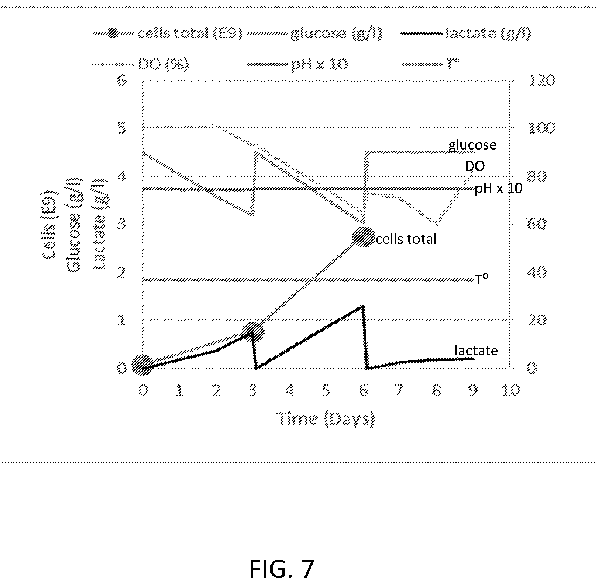

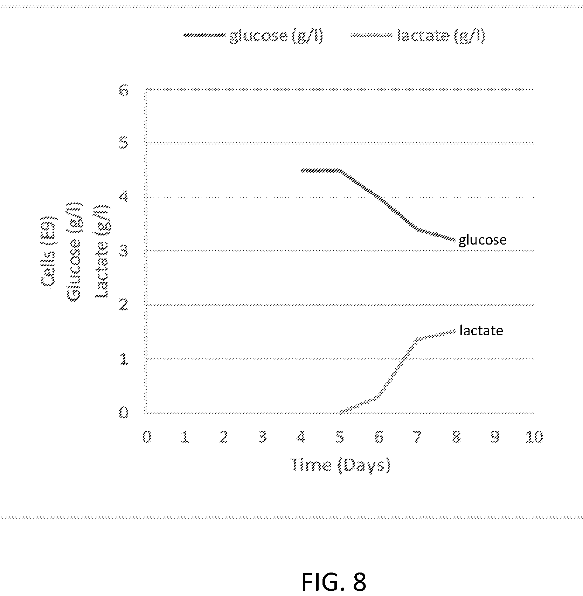

During the pre-culture phase (i.e., before virus infection), a continuous glucose consumption and lactate production was observed throughout the culture (**FIG. 7 and FIG. 8**). Medium replacement was performed on Day 3 to avoid glucose shortage and lactate accumulation above the critical concentration of 20-25 mM. Oxygen consumption increased over time and dissolved oxygen (DO) was maintained above the set-point of 50% by automated injection of air/oxygen. Temperature and pH remained stable.

The cell density reached 0.55 E6 cells/cm$^2$ on Day 6, the day of infection. After infection, glucose consumption and lactate production were extremely low (for glucose: not measurable, for lactate: less than 1g/L-1/day). Consistently, oxygen consumption decreased as indicated by a marked DO increase (**FIG. 7**). Floating cell debris were visible starting from 2 DPI and increased in number over time.

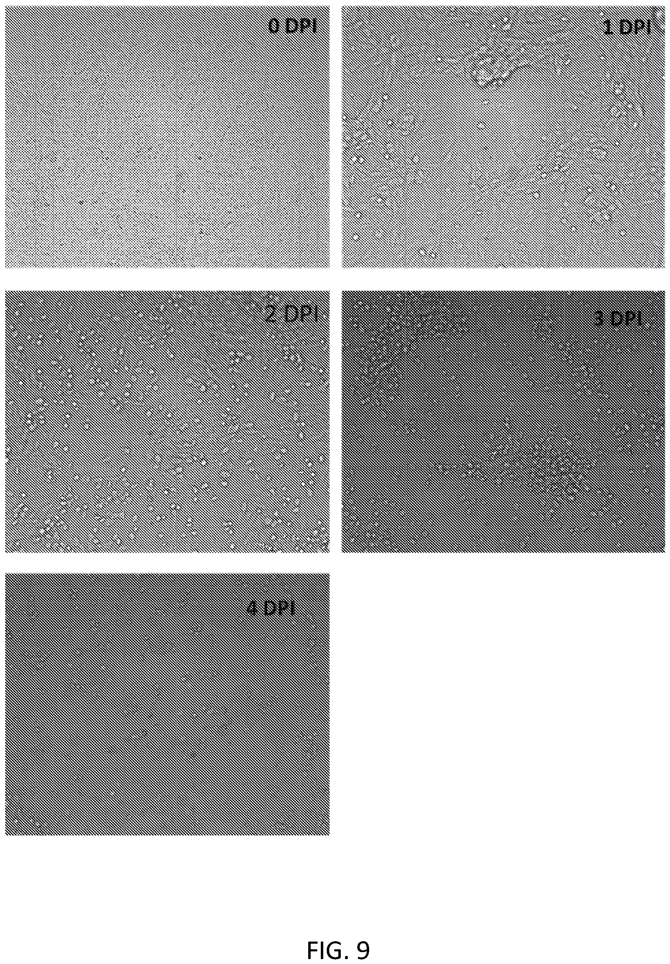

In the control Cell-Stack, a cytopathic effect (CPE) was visible from 2 DPI and increased over time (**FIG. 9**). At 4 DPI less than ~5 % of the Cell-Stack was still covered with adherent cells.

TCID50 tests results for samples at indicated days post infection (DPI) are shown in Tables 4 and 5. TCID50 results were similar between tests performed in Singapore and Brussels.

**Table 4.** TCID50 tests performed in Singapore.

| Sample | iCELLis NANO (TCID50/mL) |
|---|---|
| 1 DPI | 1.00 E7 |

(continued)

| Sample | iCELLis NANO (TCID50/mL) |
|---|---|
| 2 DPI | 1.00 E7 |
| 3 DPI (AM) | 1.00 E7 |
| 3 DPI (PM) | 1.00 E7 |
| 4 DPI (AM) | 1.78 E7 |
| 4 DPI (PM) | 3.16 E7 |
| Positive Control | 1.00 E7 |

**Table 5.** TCID50 tests performed in Brussels.

| Sample | iCELLis NANO (TCID50/mL) | Control Cell-Stack (TCID50/mL) |
|---|---|---|
| 1 DPI | 1.78 E6 | 3.16 E5 |
| 2 DPI | 1.00 E7 | 3.16 E6 |
| 3 DPI (PM) | 5.62 E6 | 3.16 E7 |
| 4 DPI (PM) | 1.78 E6 | 5.62 E6 |
| **Positive Control** | **1.78 E7** | |

**Example 3: EV71 infection evaluation in iCELLis NANO at a cell density of 0.25 E6 cells/cm$^2$**

The kinetics and yields of EV71 virus production by Vero cells grown in the iCELLis NANO was evaluated. A 40 ml fixed-bed Compaction 1.5 X bio-reactor was used and EV71 infection was performed at a cell density of 0.25 E$^6$ cells/cm$^2$.

Methods

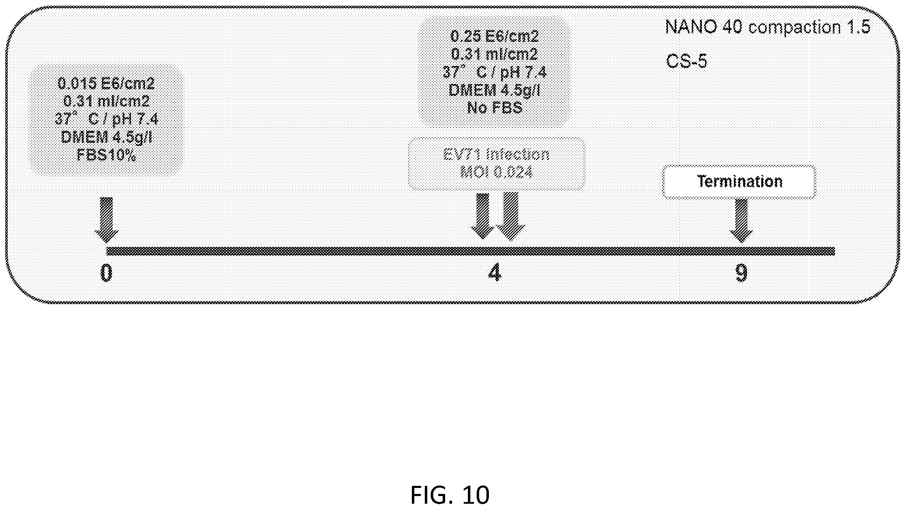

The NANO was seeded as described in Example 1 and the total culture volume used was 2,500 mL. No medium replacement was performed during the pre-culture phase. On Day 3 cell density reached 0.25 E$^6$ cells/cm$^2$. The bio-reactor was rinsed with DPBS and infection with EV71 was started using a MOI of 0.024. The infection medium used was identical to culture medium but devoid of FBS, as described in Example 2. The culture volume used during infection was 2,500 mL (i.e. vol./surf. ratio of 0.31 mL/cm$^2$). As a control, a Cell-Stack 5 (CS-5) was cultured and infected as described in Example 2. Composition of the medium used for Vero cell growth and infection is summarized in Table 3 of Example 2. A schematic of the EV71 iCELLis NANO culture is shown in **FIG. 10.**

Results

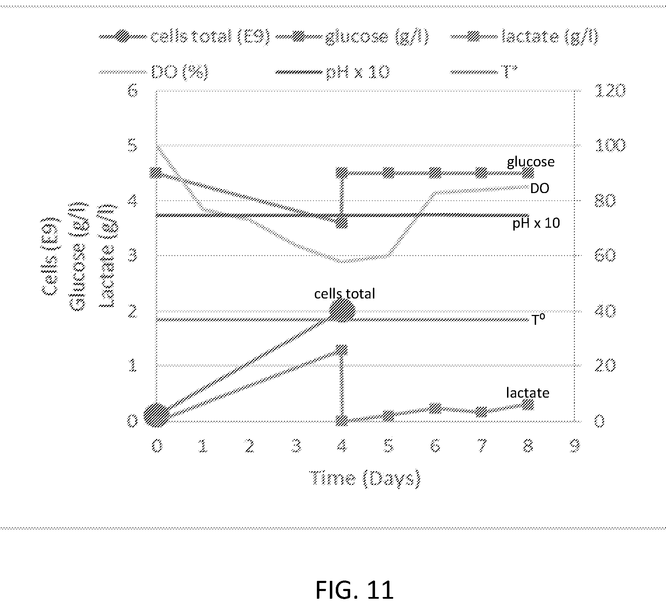

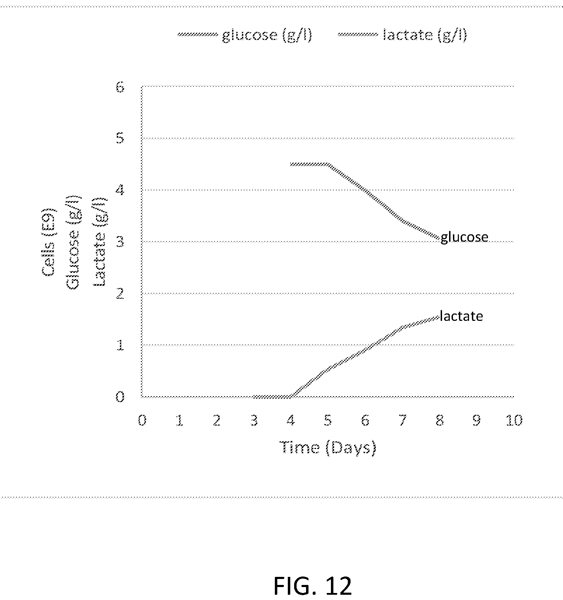

During the pre-culture phase (i.e., before virus infection), glucose consumption and lactate production was observed throughout the culture (**FIG. 11 and FIG. 12**). Oxygen consumption increased as indicated by a drop in DO, which was maintained above the set-point of 50% by automated injection of air/oxygen. Temperature and pH remained stable. The total amount of cells on Day 4 was 2.0 E$^9$, as compared to ~ 1.5 E$^9$ cells using iCELLis NANO 40 compaction 1X. This increase (~35 %) was consistent with the higher surface developed using compaction 1.5X.

On Day 4, the cell density reached 0.25 E6 cells/cm$^2$ and infection was initiated. After infection, glucose, oxygen consumption, and lactate production were extremely low. Floating cell debris were visible starting from 2 DPI and increased in number over time. The control Cell-Stack 5 behaved identically to the control of Experiment 2, with a CPE visible starting from 2 DPI.

TCID50 test results for samples at indicated days post infection (DPI) are shown in Tables 6 and 7. The highest

titer (3.16 E$^7$ TCID50/mL, Table 6) was obtained after 4 DPI in the NANO and was slightly higher than the highest titer obtained in the control Cell-Stack

(1.0 E$^7$ TCID50/mL, Table 6). A secondary TCID50 analysis performed on the same samples confirmed that the virus titer obtained in the NANO was slightly higher than in the Control Cell-Stack (Table 7).

**Table 6:** TCID50 performed in Brussels.

| Sample | iCELLis NANO (TCID50/mL) | Control Cell-Stack (TCID50/mL) |
|---|---|---|
| 1 DPI | 3.16 E5 | 1.75 E5 |
| 2 DPI | 3.16 E6 | 1.00 E6 |
| 3 DPI (AM) | 3.16 E6 | 1.00 E6 |
| 3 DPI (PM) | 1.00 E7 | 3.16 E6 |
| 4 DPI | 3.16 E7 | 1.00 E7 |
| **Positive Control** | **1.00 E7** | |

**Table 7:** Secondary TCID50 performed in Brussels.

| Sample | iCELLis NANO (TCID50/mL) | Control Cell-Stack (TCID50/mL) |
|---|---|---|
| 1 DPI | 3.16 E5 | 1.78 E6 |
| 2 DPI | 5.62 E6 | 3.16 E6 |
| 3 DPI (PM) | 3.16 E6 | 3.16 E6 |
| 4 DPI | 1.00 E7 | 1.78 E6 |
| **Positive Control** | **1.78 E6** | |

**Example 4: Polio S2 infection evaluation in iCELLis NANO at a cell density of 0.52 E6 cells/cm$^2$**

Polio S2 virus production by Vero cells grown in the iCELLis NANO bio-reactor was evaluated. A 40ml fixed-bed Compaction 1 X bio-reactor was used and Polio infection was performed at a cell density of 0.52 E$^6$ cells/cm$^2$.

Methods

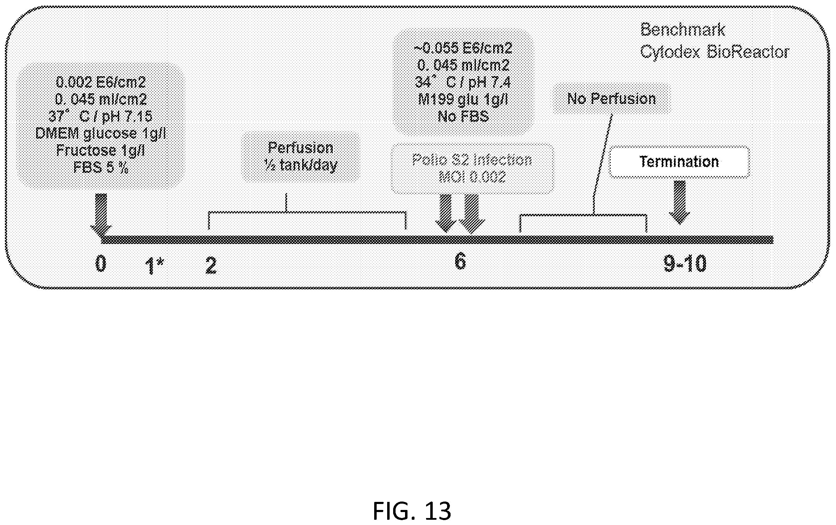

A Cytodex bio-reactor was used to culture Polio S2. The medium used for the growth phase was DME containing 1g/L glucose supplemented with fructose (1g/L) and 5% FBS. The pH set point was 7.15, the seeding density was 0.002-0.0045 E$^6$ cells/cm$^2$, the culture volume to surface ratio was 0.045 mL/cm$^2$, and the density at infection was 0.055 E$^6$ cells/cm$^2$. **FIG. 13** summarizes the Polio S2 Cytodex process.

The iCELLis NANO was seeded as described using a total culture volume of 1,650 mL and cells were allowed to grow. At Day 3 the culture medium was replaced. On Day 6 cell density reached 1.55 E6 cells/cm2. The bio-reactor was rinsed with DPBS and infection with Polio S2 was initiated with a MOI of 0.002 using M199 as infection medium. As the cell density in the bio-reactor was twice the cell density of the control Cell-Stack, twice the volume of infection medium was used (3,300 mL, i.e. vol./surf. ratio of 0.62 mL/cm$^2$). During infection, temperature was set to 34 °C.

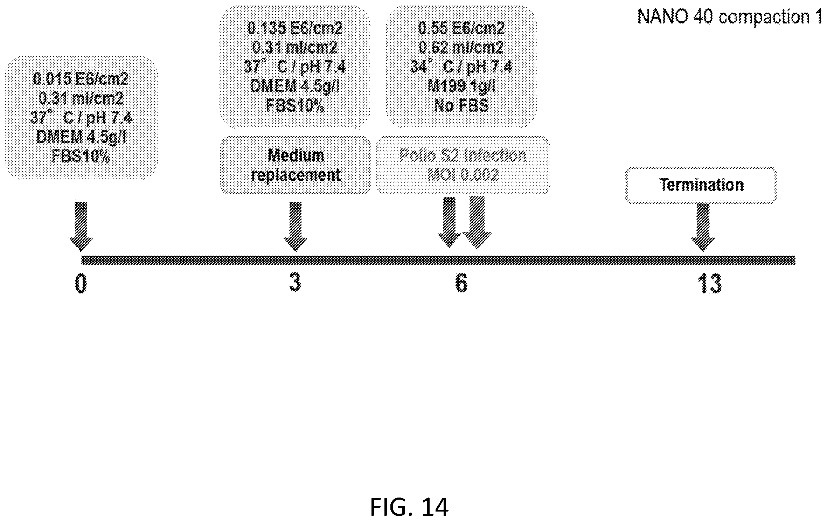

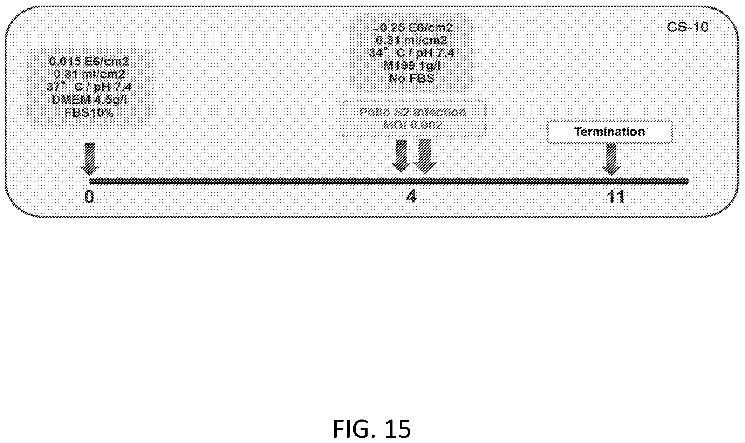

As a control, a Cell-Stack 10 (6300 cm$^2$) was seeded using the same inoculation density and cells were allowed to grow at 37°C in a CO$_2$ (5%) incubator. After 4 days, culture medium was discarded and the Cell-Stack was rinsed twice with DPBS. Polio S2 infection was performed using the same culture medium devoid of FBS. A constant vol./surf. ratio of 0.31 mL/cm$^2$ was used during the control experiment. Composition of the medium used for Vero cell growth and Polio infection is summarized in Table 8. Schematics of the Polio iCELLis NANO and control Cell-Stack cultures are shown in **FIG. 14** and **FIG. 15,** respectively. The controller set-points utilized during the NANO culture are summarized in Table 9.

**Table 8:** Composition of media used during Vero cell growth and Polio infection.

|  | Medium used for Vero cells growth | Medium used for Vero cells infection |
|---|---|---|
| Polio S2 | DMEM #1145 (Sigma) 4.5 g/L glucose 2 mM glutamine 10 % FBS #10099-141 (Gibco) | M199 #31150-30 (Gibco) |

**Table 9**: Controller set-points during Polio S2 iCELLis NANO culture

|  |  | iCELLis NANO bioreactor |
|---|---|---|
| pH |  |  |
|  | pH Setpoint | 7.40 |
| DO |  |  |
|  | DO Setpoint | 50 % |
| Temperature |  |  |
|  | T° Setpoint | 37°C(Vero growth) 34°C (Polio S2 infection) |
| Stirring |  |  |
|  | Stirring speed | 600 rpm / 40% |

Results

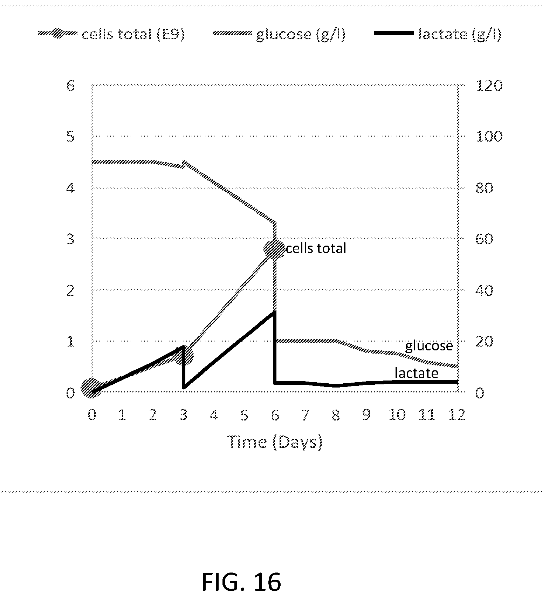

During the pre-culture, glucose consumption and lactate production was observed (**FIG. 16**). On Day 3, cell density reached 0.135 $E^6$ cells/cm$^2$ and a medium replacement was performed. On Day 6, the cell density reached 0.55 $E^6$ cells/cm$^2$, and infection was performed using M199 medium (glucose concentration: 1 g/L). After infection, glucose consumption and lactate production were extremely low (for glucose: below the detection limit, for lactate: less than 1g.L$^{-1}$/day). Floating cell debris were visible starting from 3 DPI and increased over time.

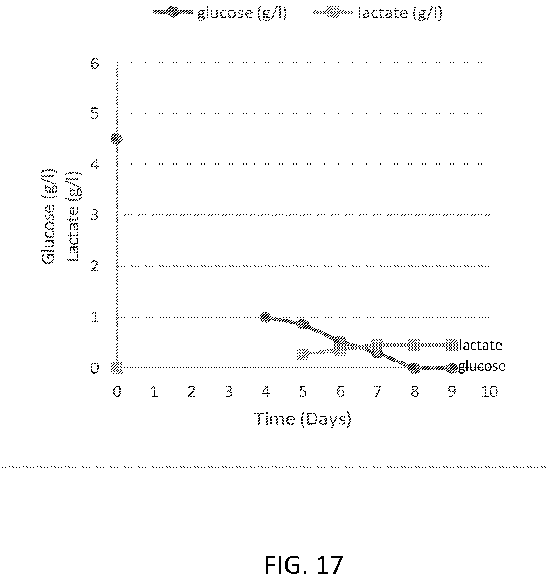

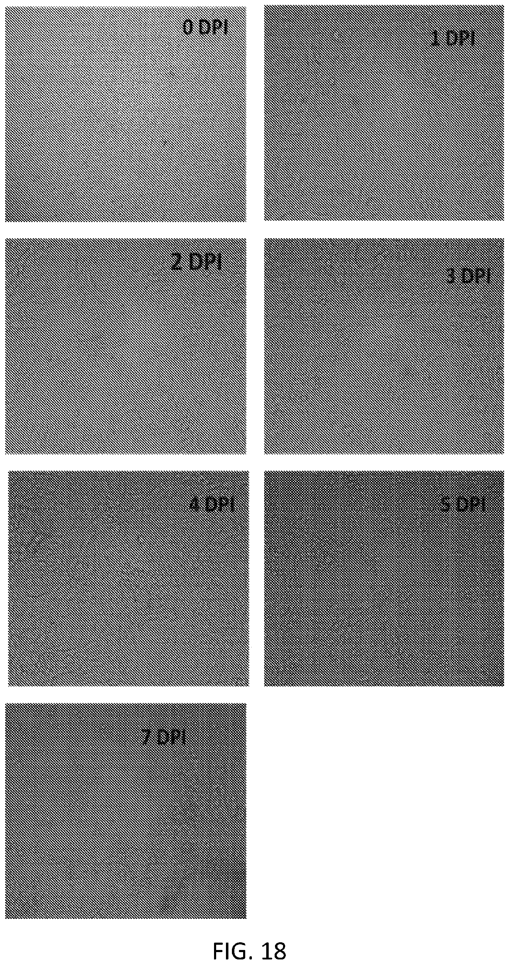

In the control Cell-Stack, a continuous glucose drop was observed and was down to detection limit on Day 8 (4 DPI) (**FIG. 17**). Turbidity in the supernatant was observed from 4 DPI, and increased over time, indicating cytopathic effect (CPE) (**FIG. 18**).

TCID50 test results for samples at indicated days post infection (DPI) are shown in Table 10. The highest titer obtained in the NANO ($10^{7.9}$) was similar to the highest titer obtained in the control Cell Stack ($10^{8.3}$ TCID50/mL). D-antigen content tests were performed by ELISA and the results were consistent with the TCID50 values.

**Table 10:** TCID50 test results.

| Sample | iCELLis NANO (TCID50/mL) | Control CS-10 (TCID50/mL) |
|---|---|---|
| 2 DPI | <10 E4.9 | - |
| 4 DPI | 10 E6.6 | - |
| 5 DPI | 10 E7.5 | 10 E7.7 |
| 6 DPI | 10 E7.9 | - |
| 7 DPI | 10 E7.9 | 10 E8.3 |

**Example 5: Polio S2 infection evaluation in iCELLis NANO at a cell density of 0.25 E6 cells/cm$^2$**

Polio S2 virus production by Vero cells grown in the iCELLis NANO bio-reactor was evaluated. A 40 ml fixed-bed Compaction 1.5 X bio-reactor was used, and infection was performed at a cell density of 0.25 $E^6$ cells/cm$^2$.

Methods

The NANO was seeded as described in Example 1 and the total culture volume used was 2,500 mL. No medium replacement was performed during the pre-culture phase. On Day 4, cell density reached 0.23 $E^6$ cells/cm$^2$. The bioreactor was rinsed with DPBS and infection with Polio S2 was started with a MOI of 0.02 using M199 as infection medium. The culture volume used during infection was 2,500 mL (*i.e.* vol./surf. ratio of 0.31 mL/cm$^2$).

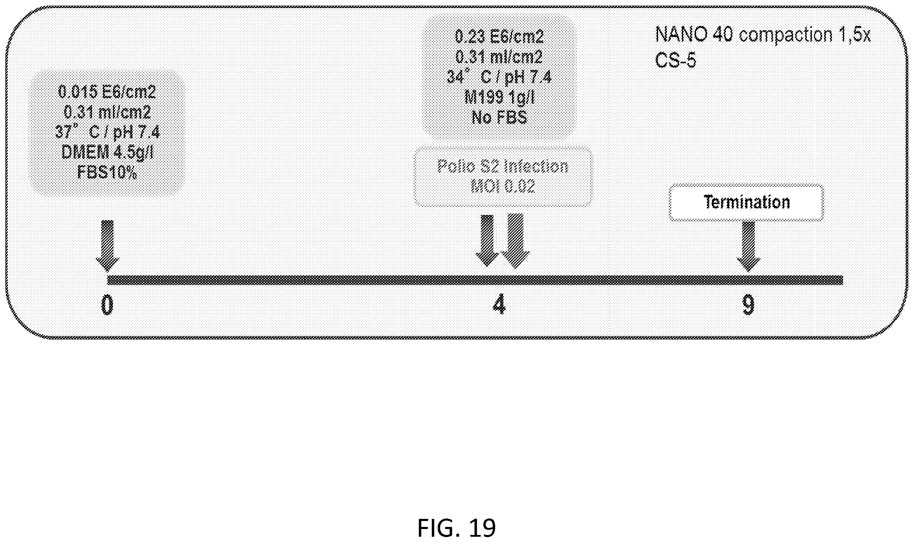

As a control, a Cell-Stack 5 was cultured and infected as described in Experiment 2. On Day 6 glucose concentration dropped down to 0.28 g/L. 0.72 g glucose was dissolved in 25 ml of DPBS, filtered on 0.22 micron, and added to reach a final concentration of 1 g/L. Composition of the medium used for Vero cell growth and Polio infection is summarized in Table 8 of Example 4. A schematic of the Polio iCELLis NANO culture is shown in **FIG. 19.** The controller set-points utilized during the NANO culture are summarized in Table 9 of Example 4.

Results

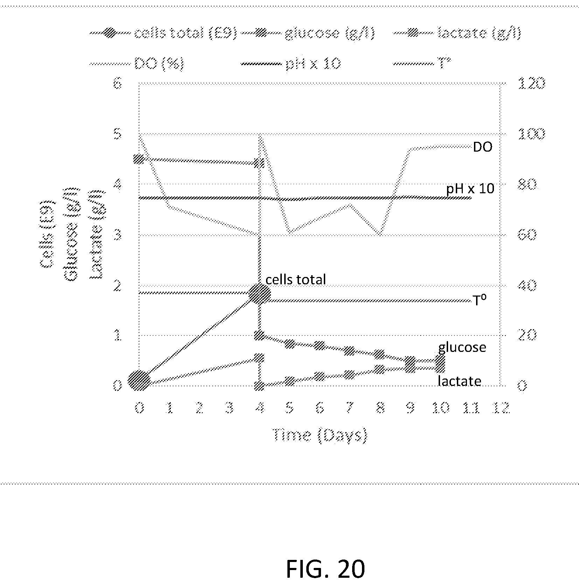

During the pre-culture phase, a lactate increase was observed. Oxygen consumption increased as indicated by a drop in dissolved oxygen (DO), which was maintained above the set-point of 50% by automated injection of air/oxygen. Temperature and pH remained stable (**FIG. 20**).

On Day 4, the cell density reached 0.23 $E^6$ cells/cm$^2$ and infection was initiated. After Polio S2 infection, glucose, oxygen consumption, and lactate production were extremely low (**FIG. 20**). Floating cell debris were visible starting from 2 DPI and increased in number over time.

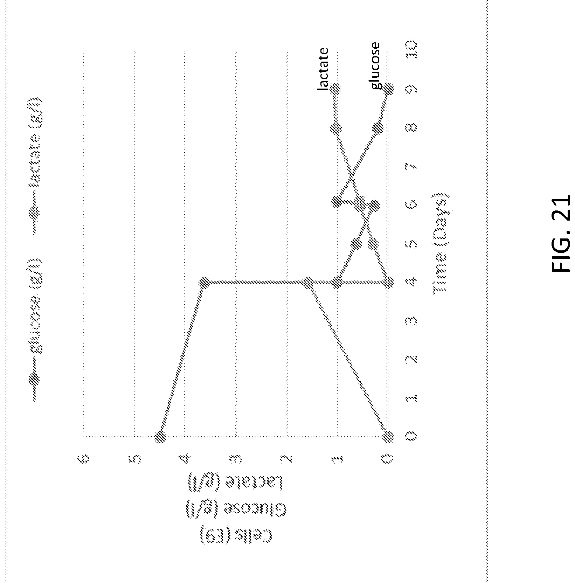

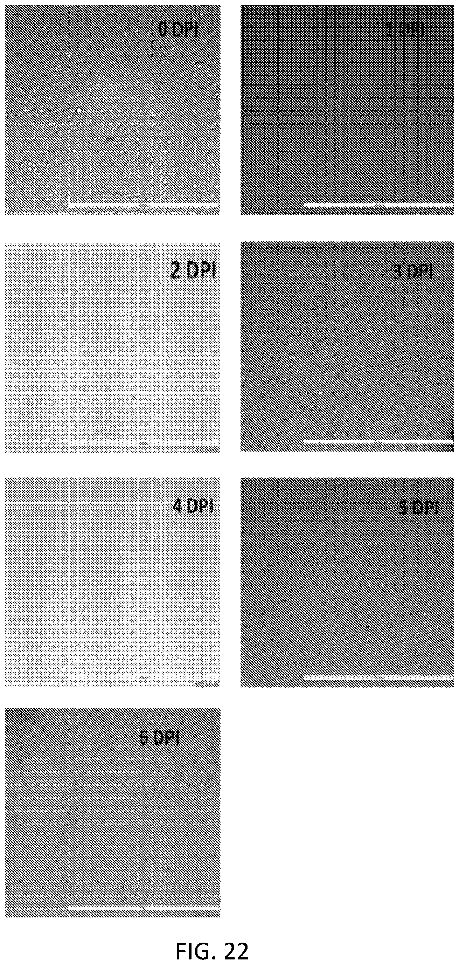

In the control Cell-Stack, a continuous glucose drop was observed down to 0.28 g/L on Day 6. 1.72 g of glucose were added and the culture was pursued (**FIG. 21**). Turbidity in the supernatant was observed from 4 DPI, and increased over time, indicating cytopathic effect (CPE) (**FIG. 22**).

TCID50 test results for samples at indicated days post infection (DPI) are shown in Table 11. The highest titer obtained in the NANO ($10^{7.8}$ TCID50/mL) was similar to the highest titer obtained in the control Cell Stack (108.3 TCID50/mL). D-antigen content tests were performed by ELISA and the results were consistent with the TCID50 values.

**Table 11:** TCID50 test results

| Sample | iCELLis NANO (TCID50/mL) | Control CS-5 (TCID50/mL) |
|---|---|---|
| 1 DPI | 10 E5.1 | 10 E4.6 |
| 2 DPI | 10 E5.9 | 10 E5.8 |
| 3 DPI | 10 E5.9 | 10 E5.9 |
| 4 DPI | 10 E7.1 | 10 E6.6 |
| 5 DPI | 10 E7.7 | 10 E7.1 |
| 6 DPI | 10 E7.6 | 10 E8.0 |
| 7 DPI | 10 E7.8 | 10 E8.3 |

**Example 6: Summary of experimental parameters**

A summary of key parameters from Experiments 1-5, performed in iCELLis NANO and Cell Stacks, are shown in Tables 12 and 13 below.

**Table 12:** Summary of key parameters of experiments performed in iCELLis NANO.

| Experiment | Cell density at inoculation (E6/cm2) | Cell density at infection (E6/cm2) | MOI | NANO compaction | NANO fixed bed volume (mL) | NANO surface (m2) | Total media vol. used for cell growth (mL) | Total media vol. used for infection (mL) |
|---|---|---|---|---|---|---|---|---|
| Exp. 1 | 0.015 | - | - | 1X | 40 | 0.53 | 4,950 | - |
| Exp. 2 | 0.015 | 0.52 | 0.024 | 1X | 40 | 0.53 | 3,300 | 3,300 |
| Exp. 3 | 0.015 | 0.25 | 0.024 | 1.5X | 40 | 0.8 | 2,480 | 2,480 |

(continued)

| Experiment | Cell density at inoculation (E6/cm2) | Cell density at infection (E6/cm2) | MOI | NANO compaction | NANO fixed bed volume (mL) | NANO surface (m2) | Total media vol. used for cell growth (mL) | Total media vol. used for infection (mL) |
|---|---|---|---|---|---|---|---|---|
| Exp. 4 | 0.015 | 0.55 | 0.002 | 1X | 40 | 0.53 | 3,300 | 3,300 |
| Exp. 5 | 0.015 | 0.22 | 0.020 | 1.5X | 40 | 0.8 | 2,480 | 2,480 |

**Table 13:** Summary of key parameters of control experiments performed in Cell Stack.

| Experiment | Cell density at inoculation (E6/cm2) | Estimate of Cell density at infection (E6/cm2) | MOI | Cell Stack (number of plateau) | Cell Stack surface (m2) | Total media vol. used for cell growth (mL) | Total media vol. used for infection (mL) |
|---|---|---|---|---|---|---|---|
| Exp. 1 | 0.015 | - | - | 10 | 0.63 | 2,000 | - |
| Exp. 2 | 0.015 | -0.25 | 0.024 | 10 | 0.63 | 2,000 | 2,000 |
| Exp. 3 | 0.015 | -0.25 | 0.024 | 5 | 0.32 | 1,000 | 1,000 |
| Exp. 4 | 0.015 | -0.25 | 0.002 | 10 | 0.63 | 2,000 | 2,000 |
| Exp. 5 | 0.015 | -0.25 | 0.020 | 5 | 0.32 | 1,000 | 1,000 |

**Example 7: Optimization study: impact of decreasing EV71 MOI in iCELLis NANO cultures by 20-fold**

The impact of reducing the EV71 MOI in iCELLis NANO cultures by 20-fold, from 0.02 down to 0.001, was evaluated. Reducing the MOI lowers the volume and costs of the corresponding virus seed bank.

Methods

To evaluate the impact of a 20-fold decrease of cell density at inoculation, two iCELLis NANO cultures were performed and infected at densities of either 0.25 or $0.5 \times 10E^6$ cells/cm$^2$ using an MOI of 0.001. These cultures were then compared to iCELLis NANO cultures performed under similar conditions but infected with a MOI of 0.02. The viral productivity was evaluated using the TCID50 method.

Results

TCID50 results are presented in Table 14. At the low cell density of $0.25 \times 10E^6$ cells/cm$^2$, a high MOI resulted in a 5-fold higher productivity. At the cell density of $0.5 \times 10E^6$ cells/cm$^2$, no significant difference between low and high MOI was observed. In addition, experiments performed in Cell-Stacks indicated that MOI (from 0.02 to 0.0001) did not significantly impact productivity. Based on these results, a low MOI (0.001) was chosen for subsequent studies.

**Table 14:** Impact of MOI on maximal viral productivity

| Cell density at infection (106 cells/cm2) | High MOI (0.02) (TCID50/mL) | Low MOI (0.001) (TCID50/mL) |
|---|---|---|
| 0.25 | 1.00E+08 | 1.78E+07 |
| 0.50 | 1.78E+07 | 1.00E+07 |

**Example 8: Optimization study: determination of optimal cell density at EV71 infection and productivity peak**

Cellular density at infection critically impacts viral productivity. The optimal cell density at EV71 infection and the kinetics of virus release were evaluated in order to determine the productivity peak.

Methods

A series of five iCELLis NANO cultures were infected with EV71 (MOI of 0.001) at different cell densities. The following culture conditions were used: cell density at inoculation: 15,000 cells/cm$^2$, growth media: Sigma D1145+10% FBS+ 4 mM glutamine, infection media : growth media without FBS, volume/surface ratio: 0.3 mL/cm$^2$. A full media replacement during the growth phase was made to reach the following densities: 0.55, 0.7 and 0.86×10E6 cells/cm$^2$. Cultures were fully monitored (glucose consumption, lactate production, cell density, DO, pH) and viral productivity was measured by the TCID$_{50}$ method at each day after infection.

Results

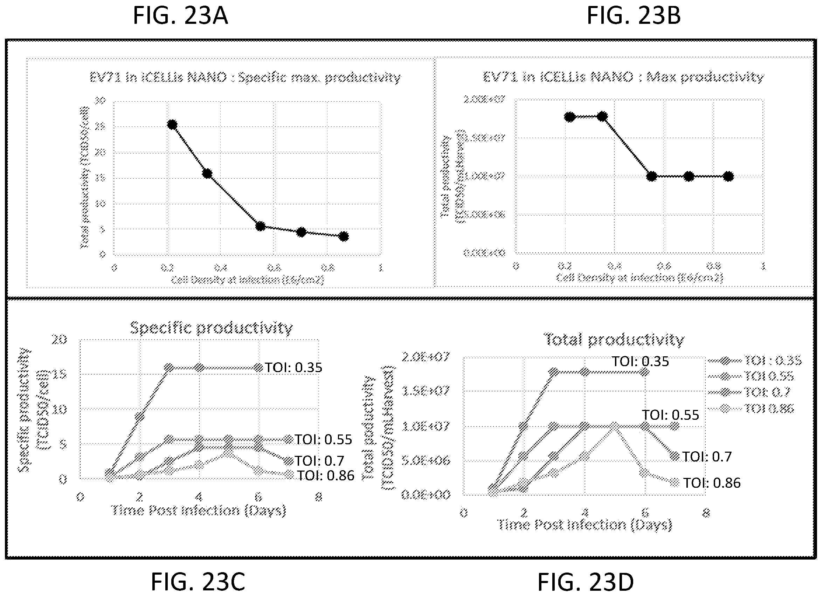

Specific (per cell) productivity decreased as the cell density increased (**FIG. 23A to 23D**). The highest volumetric productivities were observed with the lowest cell densities, indicating that the higher cell density did not compensate for the lower specific productivity.

Overall, a similar maximal productivity was obtained at both 0.2 and 0.35×10E6 cells/cm$^2$ densities, indicating that total productivity was independent of cell density within this range. The maximal productivity was achieved after 3 days post-infection (day of infection is Day 0) at lower cell densities and remained stable for at least 4 more days. At higher cell densities, a productivity peak was reached after 4-5 days post-infection and then productivity decreased.

In conclusion, the data indicated, without wishing to be bound by theory, that a low cell density (*e.g.,* 0.2-0.35×10$^5$ cells/cm$^2$) may be advantageous for reasons including without limitation: specific productivity (per cell), volumetric productivity (per mL of harvest), stability over time, less media consumption, and less HCP and DNA contaminants originating from the cells at low densities. Conversely, the data indicate, without wishing to be bound by theory, that high cell densities (*e.g.,* >0.5×10$^5$ cells/cm$^2$) may result in lower productivity, poorer stability over time, higher media consumption, and more HCP and DNA contaminants originating from higher cell densities.

**Example 9: Optimization study: impact of decreasing cell density at EV71 inoculation**

Potential benefits of reducing the volume of inoculum include: (i) less labor time and (ii) less risk of contamination. The impact of reducing the cell density at inoculation by 3-fold was evaluated.

Methods

An iCELLis NANO was inoculated with a low density of 5,000 cells/cm$^2$ and compared to an iCELLis NANO inoculated with a cell density of 15,000 cells/cm$^2$. Both iCELLis NANO were infected at a cellular density of 350,000 cells/cm$^2$ using an MOI of 0.001. Volume/surface ratios during growth and infection phases were 0.3 mL/cm$^2$.

Results

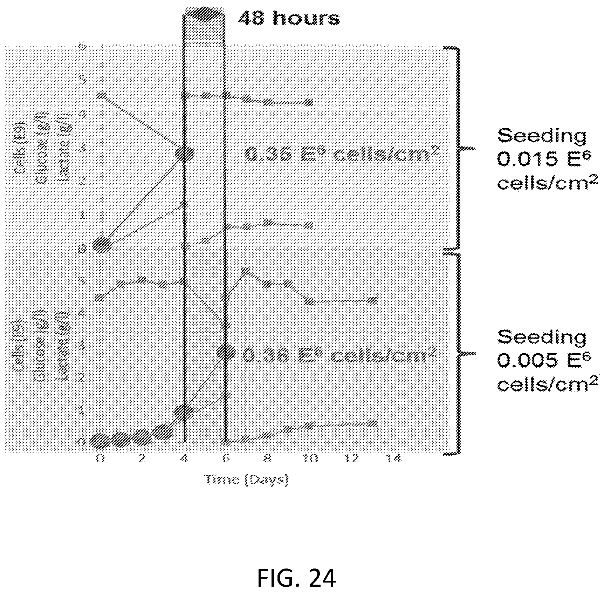

During the growth phase, glucose consumption and lactate production were similar between the two conditions (**FIG. 24**). A -48 hour delay to reach the target cell density at infection was observed when the low cell density at inoculation was used. No significant difference in viral productivity was observed between the two cultures (Table 15).

**Table 15:** Viral productivity

|  | 15,000 cells/cm2 seeding density | 5,000 cells/cm2 seeding density |
|---|---|---|
| Maximal viral productivity (TCID50/mL) | 1.78E+07 | 3.16E+07 |

The data indicated that reducing cell density at inoculation to 5,000 cells/cm$^2$ (i) had no impact on viral productivity, (ii) did not increase media/metabolite consumption, and (iii) prolonged the growth phase by up to 48 hours. In conclusion, the data indicated, without wishing to be bound by theory, that reducing cell density at inoculation may be advantageous for reasons including without limitation: inoculum size, labor time, foot-print in the pre-culture step, number of incubators, and risk of contamination.

**Example 10: Optimization study: impact of decreasing FBS concentration and volume/surface ratio during growth phase.**

The impact of reducing the culture volume used (reduction of the volume/surface ratio) or reducing the FBS concentration on cell growth or viral productivity was evaluated.

Methods

A series of experiments was performed in T-flasks and cell density was monitored, together with glucose consumption and lactate production. A culture in iCELLis NANO using 5% FBS at growth phase and a low inoculation density of 5,000 cells/cm2 was also performed. Cells were allowed to grow to a density of ~0.25×10E6 cells/cmand then EV71 infected (MOI= 0.001). Viral productivity was assayed.

Results

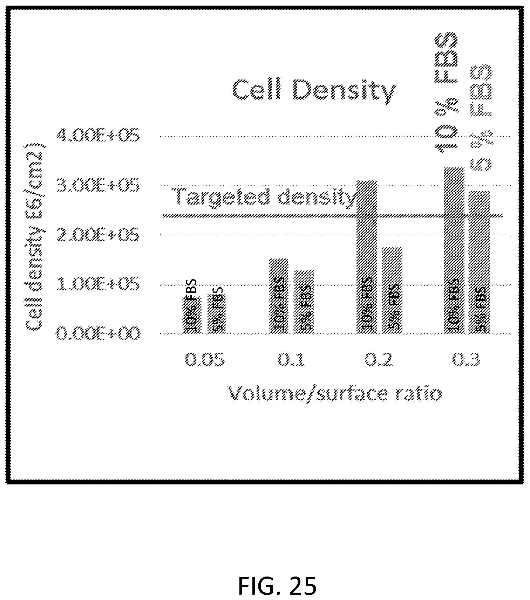

Two conditions in the T-flask cultures reached the target cell density of 200,000 cells/cm2: (1) 10% FBS with a volume/surface ratio of 0.2, and (2) 5% FBS with a volume/surface ratio of 0.3 (**FIG. 25**). In the iCELLis NANO cultures, a 2-fold reduction of FBS concentration had no significant impact on the growth phase or viral productivity (Table 16).

**Table 16:** Viral productivity

|  | Growth phase @ 10 % FBS | Growth phase @ 5 % FBS |
|---|---|---|
| Maximal viral productivity (TCID50/mL) | 3.16E+07 | 1.00E+08 |

The data indicated that reducing the FBS concentration from 10% to 5% during the growth phase had no significant impact on growth phase duration or viral productivity.

**Example 11: Summary of optimized EV71 iCELLis NANO culture conditions**

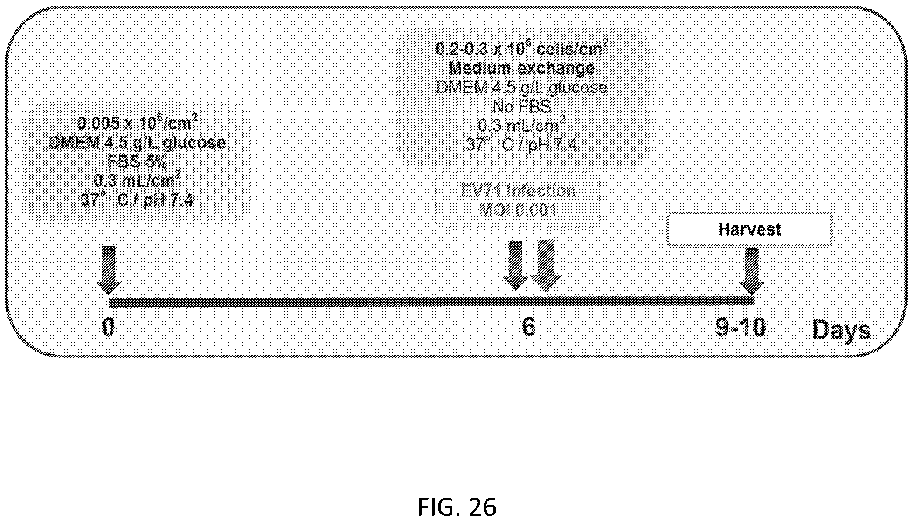

Culture parameters for EV71 in the iCELLis NANO both pre and post optimization experiments (Examples 7-10) are shown in Table 17. The resulting iCELLis NANO experimental set-up is also summarized in **FIG. 26**. In the optimized experimental set-up, the iCELLis NANO was inoculated with a cell density of 5,000 cells/cmand the cells were allowed to grow in DMEM medium (Sigma D1145, containing 4.5 g/L glucose) supplemented with 4 mM glutamine and 5 % FBS, at 37°C and pH 7.4 for 6 days (without media exchange) until they reached a density of $0.2 \times 10^6$ cells/cm2. Volume/surface ratio was 0.3 mL/cm2. For infection, two rinsing steps were performed to remove albumin and other FBS-contained proteins. Infection was performed in DMEM (Sigma D1145) medium supplemented with 4 mM glutamine without FBS, using the same volume/surface ratio of 0.3 mL/cm2 and a MOI of 0.001. Harvest was performed 3-4 days after infection.

**Table 17:** Key culture parameters for the iCELLis optimized process.

|  | Pre-optimization | Post-optimization |
|---|---|---|
| Inoculum | 15,000 cells/cm2 | 5,000 cells/cm2 |
| Growth medium | DMEM+10% FBS | DMEM+10% FBS |
| Vol/surf. (growth phase) | 0.3 | 0.3 |
| Infection medium | DMEM no FBS | DMEM no FBS |
| Vol/surf. (infection phase) | 0.3 | 0.3 |
| MOI | 0.02 | 0.001 |
| Viral productivity (log TCID50/mL) | 7-8 | 7-8 |

**Example 12: Optimization: EV71 process robustness**

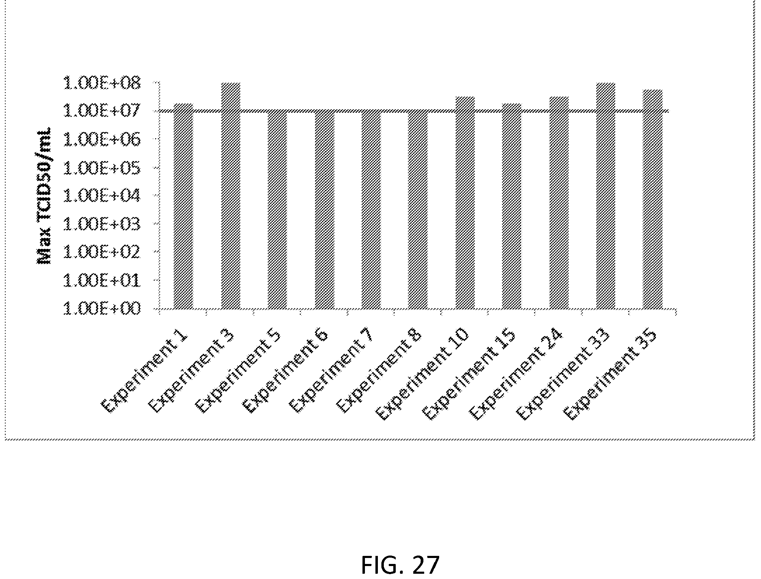

A compilation of the maximal EV71 productivities for iCELLis NANO experiments is presented in **FIG. 27**. Each of these experiments was performed using MOI ranging from 0.001 to 0.02, inoculum densities from 5,000 to 15,000 cells/cm2, densities at infection ranging from 0.2 to ~0.8×10^6 cells/cm2, and various FBS concentrations. The viral productivity varied by less than one log and ranged between 107-108 TCID50/mL of harvest.

**Example 13: EV71 process in iCELLis500/100**

The ability to scale-up EV71 production, from iCELLis NANO (2 cm bed height) to iCELLis500/100 (2 cm bed height) was evaluated. The post-optimization cell culture parameters presented in Example 11 were utilized, with the exception that a 10% FBS concentration was used during the growth phase.

Methods

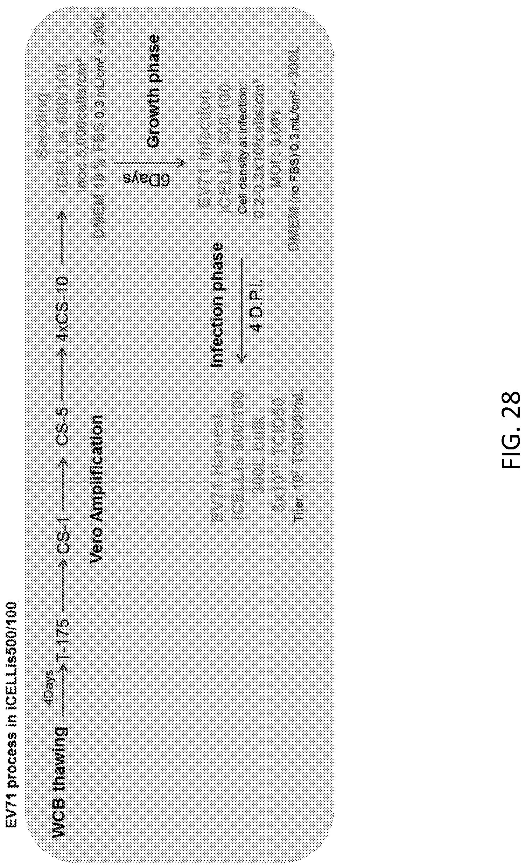

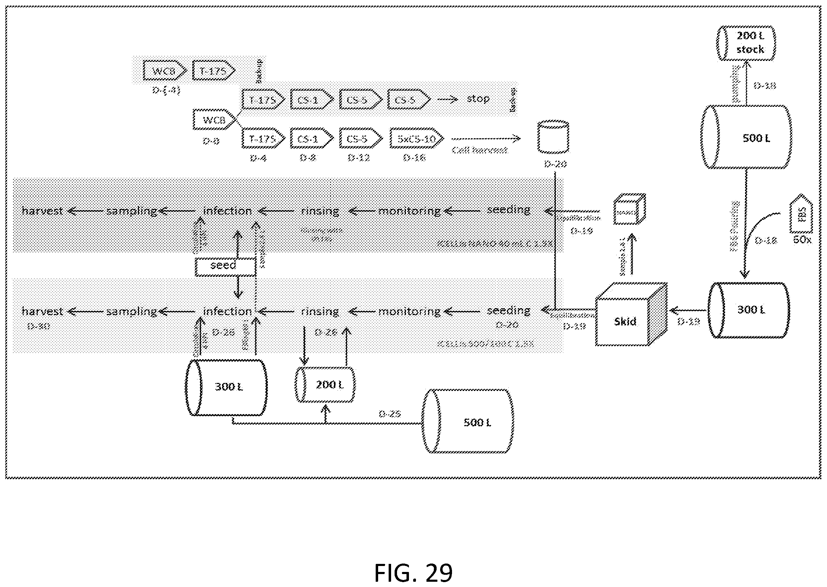

The run was performed using the iCELLis500/100 bio-reactor (2 cm bed height, 5L fixed-bed developing a surface of 100 m2), equipped with a single-use bio-mass probe and piloted by the iCELLis Skid. An overall view of the culture process is depicted in **FIG. 28,** and the experimental set-up of the iCELLis500/100 run is shown in **FIG. 29.** As controls, an iCELLis NANO and a Cell-Stack culture were performed using the same raw materials as those used for the iCELLis500/100 culture.

*Inoculum*

5 billion Vero cells ($5\times10^9$) were used to seed the iCELLis500/100 at a density of 5,000 cells/cm2. The inoculum was made through a series of amplification steps. 4 Cell-Stack 10 were used to produce the desired amount of inoculum for the iCELLis 100 m2.

*Growth phase*

Growth phase was performed using 0.3 mL/cm2 of DMEM media (4.5 g/L glucose) supplemented with 4 mM glutamine and 10 % FBS (no antibiotics were added) for 6 days, until cell density reached the optimal density of $0.2\times10^6$ cells/cm2. No media replacement was performed. Temperature was 37°C and pH was maintained at 7.4 by automated infection of CO2 and NaOH. Density of oxygen was maintained above 50% by automated injection of air and/or oxygen.

The culture was performed in a recirculated batch mode using a total of 300 L which was divided as follows: 65 L in the bio-reactor and the remaining 235 L in a 500 L bag in a palletank. Recirculation between the bio-reactor and the bag was performed using the Skid's peristaltic pump at a rate of 0.25 L/min, which allowed a complete recirculation of the culture media every 20 hours. Re-circulation was initiated 40 hours after seeding to (i) allow optimal fixation of the cells to the carriers and (ii) to reduce dilution of secreted growth factors.

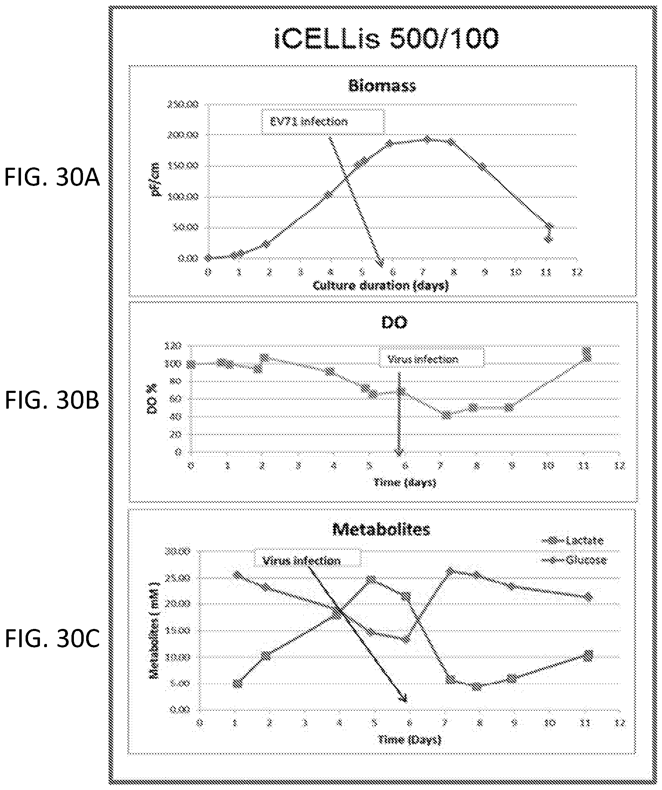

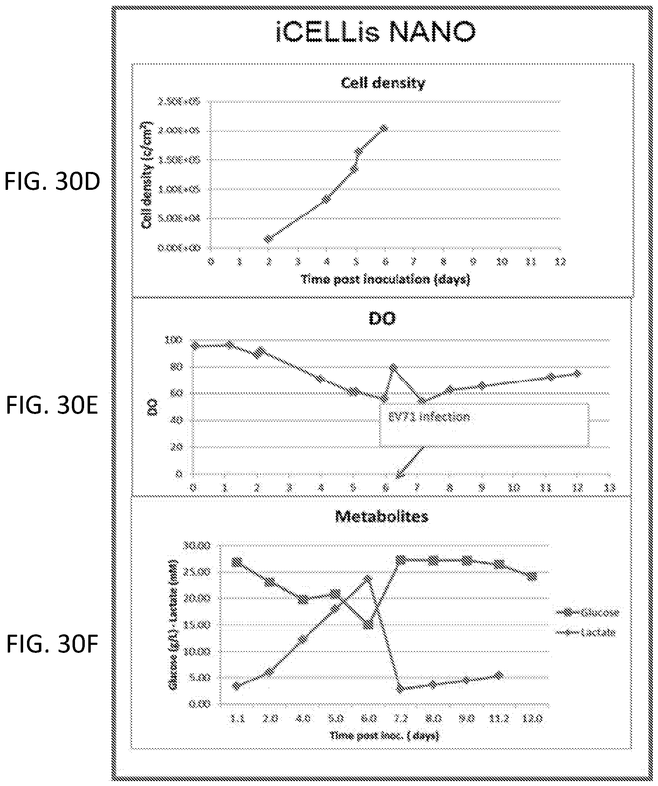

A continuous monitoring of the following parameters was automatically performed by the Skid controller: T°, pH, DO before and after fixed-bed, conductivity, and bio-mass (capacitance). The bio-mass probe was used to evaluate the cell density (and therefore time for infection) and to follow the cytopathic effect, which caused a progressive and massive drop of the bio-mass signal (**FIG. 30A to 30C**). The amount of air, oxygen, CO2, and NaOH consumed during the culture was also recorded. Daily samplings were performed to measure pH (off readings), glucose, and lactate concentrations.

*Infection phase*

Infection phase was performed in serum-free infection medium using 0.3 mL/cm2 of DMEM (4.5 g/L glucose) supplemented with 4 mM glutamine, under identical T°, pH, and DO conditions as the growth phase.

Prior to infection, the fixed-bed of the bio-reactor was rinsed twice with 65 L of infection medium to remove FBS and other serum proteins. Infection was performed by mixing the appropriate amount of virus seed in the bio-reactor to reach a MOI of 0.001. During this step, the re-circulation between the bio-reactor and the bag was stopped for 4 hours, to prevent virus losses in the bag, and re-started.

Cell culture monitoring was performed identically as during the growth phase. In addition, samples were taken for QC analysis (TCID50, HCP, DNA quantification) in the reactor and in the re-circulation bag.

*Harvest and clarification*

In order to perform a virus kinetic study over a prolonged period of time, infection was allowed to proceed until 6 days post-infection (6 DPI). An intermediate harvest (40 L) was taken at 3 DPI and the final harvest was made at 6 DPI. Both harvests were clarified on Bio-20 (PALL) deep filters for subsequent DSP studies.

*iCELLis500 handling*

The iCELLis500/100 run was performed under sterile conditions, without antibiotics. All connections were performed aseptically using a Bio-Welder or aseptic connectors. Culture samples were taken under a laminar flow, using appropriate manifolds.

Results

The iCELLis500/100 culture performed in a similar manner as the iCELLis NANO control in terms of cell growth, metabolite consumption (glucose, dissolved oxygen), and metabolite production (lactate) (**FIG. 30A to 30E**).

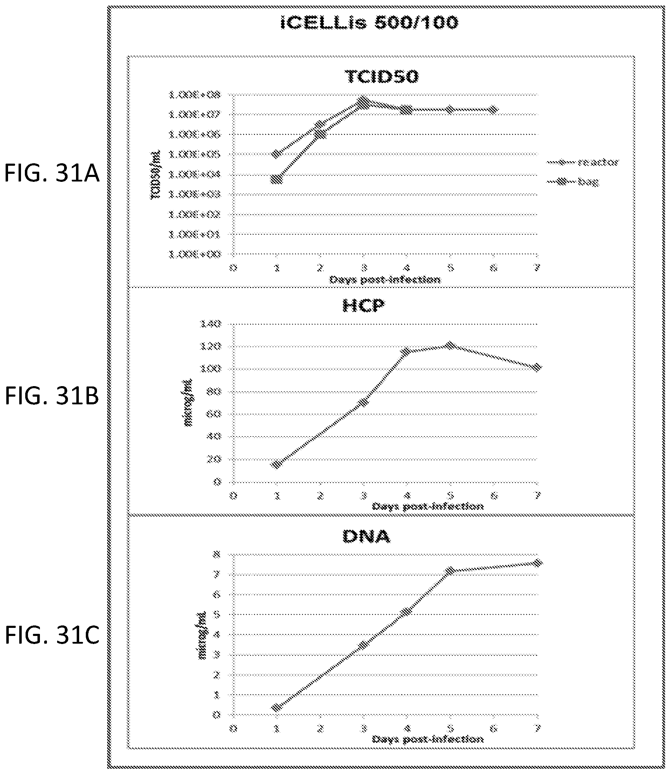

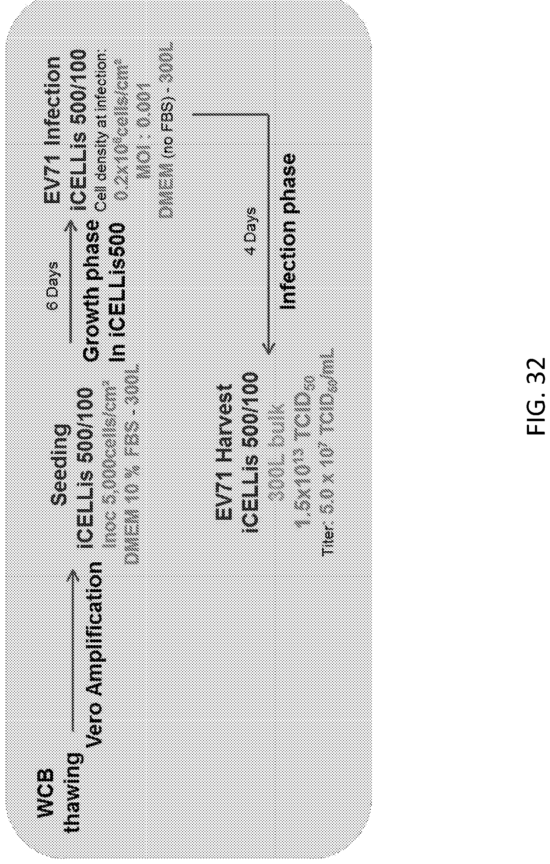

The viral productivity in iCELLis500/100 reached a maximal titer of $5.0 \times 10^7$ TCID50/mL of harvest at 3 DPI, yielding a total of $1.5 \times 10^{13}$ TCID50 (**FIG. 31A to 31C, and FIG. 32**). The productivity remained stable up to 6 DPI. HCP and DNA reach a maximum at 4 and 5 DPI, respectively.

These results demonstrated the successful scale-up of the EV71 culture process developed and optimized at small scale in iCELLis NANO. The virus titer and total yield obtained in iCELLis500/100 were similar to those obtained at small scale.

**Example 14: iCELLis500/100 with 5% FBS during growth phase**

EV71 is produced in iCELLis500/100 as described in Example 13, with the exception that a reduced 5% FBS concentration is used.

**Example 15: EV71 process in serum-free media (SFM)**

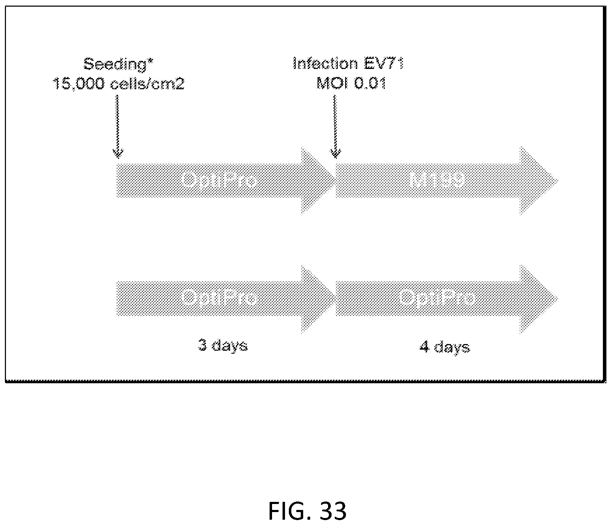

EV71 infection in T-flasks was performed using serum-free (SFM, OptiPro) or 10% FBS (M199) media (**FIG. 33**). Viral production in a progressively adapted Vero cell line and in a permanently serum-free media adapted cell line was evaluated. Cell growth and viral productivity (TCID50) were similar when serum-free or 10% FBS media was used during the viral infection phase (Table 18). EV71 production in serum-free conditions is subsequently evaluated in iCELLis NANO using a GMP Vero SFM-adapted cell line.

**Table 18:** Maximum viral productivity (TCID50/mL)

|  | Progressively adapted Vero cell line | Permanently serum-free media adapted Vero cell line |
|---|---|---|
| Growth OptiPro Infection M199 | 1.0 E+07 | 1.0 E+07 |
| Growth OptiPro Infection OptiPro | 5.0 E+07 | 5.0 E+07 |

**Example 16: Process development strategy**

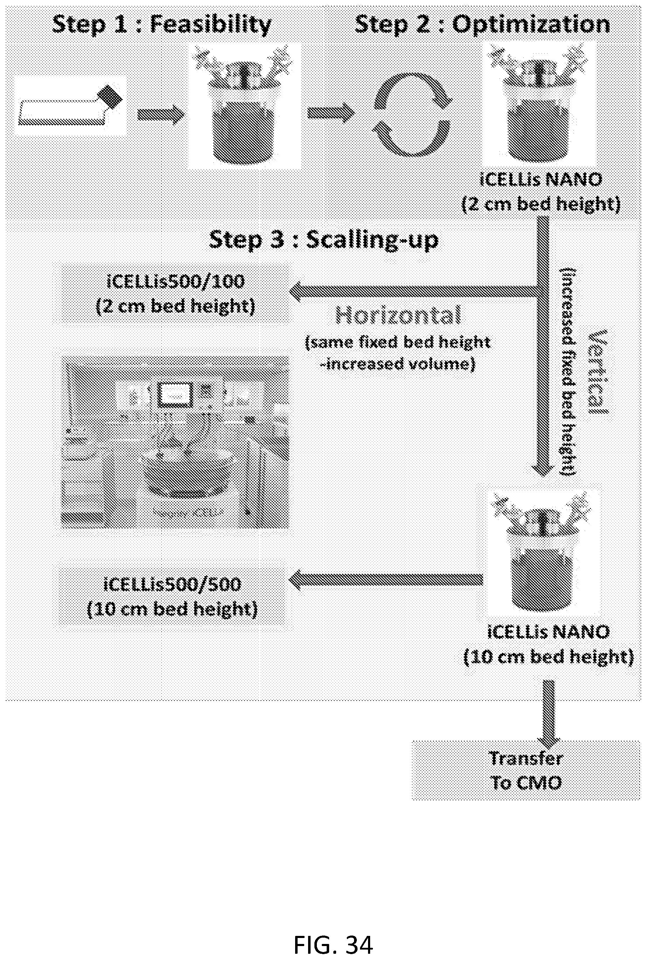

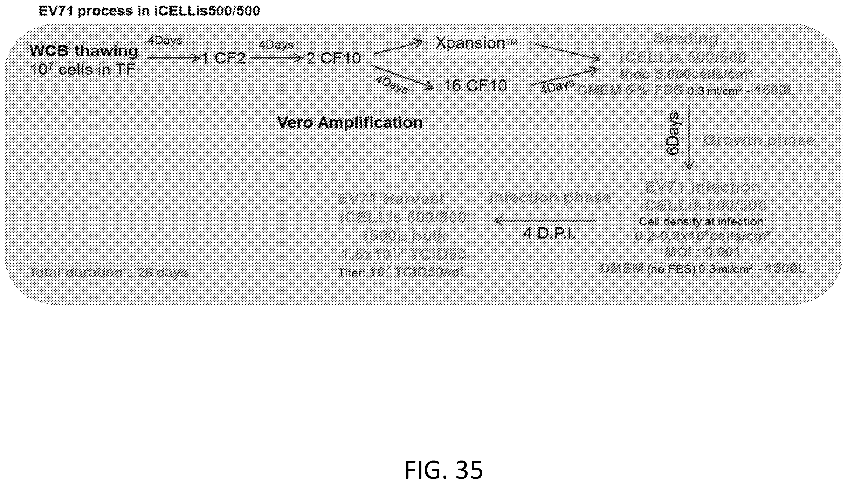

The overall process development strategy for iCELLis EV71 production is shown in **FIG. 34**. The first step (feasibility study) was demonstrated in Examples 1-3. The second step (process optimization in iCELLis NANO 2 cm bed height) was demonstrated in Examples 7-10. The third step, scaling up, is divided into 'horizontal' scaling-up (the bio-reactor's fixed bed height is kept constant, and its diameter is increased) and 'vertical' scaling-up (fixed bed height is increased and diameter is kept constant). Horizontal scale-up (iCELLis500/100, 2 cm bed height) was demonstrated in Example 13. Vertical scale-up is performed in iCELLis NANO 10 cm bed height (200 mL, 4 m$^2$) using the optimized process with 5% FBS. A factory scale EV71 culture is performed in iCELLis500/500 (10 cm bed height) (**FIG. 35**).

**Example 17: CA6 and CA16 infection evaluation in iCELLis NANO at a cellular density of 0.52 E6 cells/cm$^2$**

The kinetics and yields of CA6 and CA16 virus production by Vero cells grown in the iCELLis NANO are evaluated. A 40 ml fixed-bed Compaction 1 X bio-reactor is used and CA6 and CA16 infection is performed at a cellular density of 0.52 E6 cells/cm$^2$.

Methods

The NANO is seeded as described in Example 1 and the total culture volume used is 1,650 mL. At Day 3 the culture medium is fully replaced. Previously characterized Vero-adapted CA6 and CA16 viral strains are used. Nucleic acid and amino acid mutations in the passage 11 (P11) CA6 and passage 3 (P3) CA16 Vero-adapted strains are shown in Tables 19 and 20.

**Table 19:** Nucleic acid and amino acid mutations in Vero-adapted CA6.

|  | Nucleotide sequence | | Amino acid sequence | |
|---|---|---|---|---|
|  | CVA6 P-0 | CVA6 P-11 | CVA6 P-0 | CVA6 P-11 |
| 5'UTR | 1 – T<br>13 – G<br>14 – T<br>15 – G<br>16 – G<br>21 – C<br>23 – C<br>34 – G<br>40 – C | 1 – G<br>13 – A<br>14 – G<br>15 – C<br>16 – A<br>21 – T<br>23 – T<br>34 – T<br>40 – G | – | – |
| VP4 | No Change | No Change | No Change | No Change |
| VP2 | 1216- C<br>1382 - C | 1216 - T<br>1382 - A | 88 – D<br>144 – Q | 88 – D<br>144 – K |
| VP3 | 1753 - C<br>2024 - A<br>2275 - T | 1753 - T<br>2024 - G<br>2275 - C | 11- N<br>92 – I<br>175– D | 11 - N<br>92 – V<br>175 – D |
| VP1 | 2577 - C<br>2708 - G<br>2726 – A<br>3022 - A<br>3242 – G | 2577 - T<br>2708 - A<br>2726 - G<br>3022 - T<br>3242 - A | 46 – A<br>90 – E<br>96 – T<br>184 – S<br>268 – V | 46 – V<br>90 – K<br>96 – A<br>184 – S<br>268 – I |
| 2A | 3691 - C | 3691 - T | 112 – G | 112 – G |
| 3'UTR | 7395 - G | 7395 – T | – | – |

**Table 20:** Nucleic acid and amino acid mutations in Vero-adapted CA16.

|  | Nucleotide sequence | | Amino acid sequence | |
|---|---|---|---|---|
|  | CVA16 P-0 | CVA16 P-3 | CVA16 P-0 | CVA16 P-3 |
| 5' UTR | 6-G<br>33-G | 6-A<br>33-C |  |  |
| VP2 | 1427-T | 1427-C | 161-M | 161-T |
| VP1 | 2729-A<br>2867-T | 2729-G<br>2867-A | 99-D<br>145-V | 99-G<br>145-E |
| 2A | 3328-A | 3328-G | 2-K | 2-E |

The bio- reactor is rinsed twice with DPBS and infection with CA6 and CA16 is started as described in Example 1 using DMEM D1145 supplemented with 2 mM glutamine but devoid of FBS. Given the fact that the cell density in the bio-reactor is twice the cell density of the control Cell-Stack, twice the volume of infection medium is used (3,300 mL, i.e. Vol./surf. ratio of 0.62 mL/cm$^2$).

As a control, a Cell-Stack 10 (6360 cm$^2$) is seeded using the same inoculation density and cells are allowed to grow at 37°C in a CO2 (5%) incubator. After 4 days (i.e. on Day 3), culture medium is discarded and the Cell-Stack is rinsed twice with DPBS and CA6 and CA16 infection is performed using a MOI of 0.024. The infection medium used is identical to the culture medium but devoid of FBS. Composition of the medium used for Vero cell growth and infection is summarized in Table 21. A constant Vol./Surf. ratio of 0.31 mL/cm$^2$ is used for the control experiment.

**Table 21:** Composition of media used during Vero cell growth and CA6 and CA16 infection.

|  | Medium used for Vero cells growth | Medium used for Vero cells infection |
|---|---|---|
| CA6 and CA16 | DMEM #1145 (Sigma) 4.5 g/L glucose 2 mM glutamine 10 % FBS #10099-141 (Gibco) | DMEM #1145 (Sigma) 4.5 g/L glucose 2 mM glutamine |

**Example 18: CA6 and CA16 infection evaluation in iCELLis NANO at a cell density of 0.25 E6 cells/cm$^2$**

The kinetics and yields of CA6 and CA16 virus production by Vero cells grown in the iCELLis NANO are evaluated. A 40 ml fixed-bed Compaction 1.5 X bio-reactor is used and CA6 and CA16 infection is performed at a cell density of 0.25 E6 cells/cm$^2$.

Methods

The NANO is seeded as described in Example 1 and the total culture volume used is 2,500 mL. No medium replacement is performed during the pre-culture phase. The bio-reactor is rinsed with DPBS and infection with CA6 and CA16 is started using a MOI of 0.024. The infection medium used is identical to culture medium but devoid of FBS, as described in Example 17. The culture volume used during infection is 2,500 mL (i.e. vol./surf. ratio of 0.31 mL/cm$^2$). As a control, a Cell-Stack 5 (CS-5) is cultured and infected as described in Example 17. Composition of the medium used for Vero cell growth and infection is summarized in Table 21 of Example 17.

**Example 19: Optimization study: impact of decreasing CA6 and CA16 MOI in iCELLis NANO cultures by 20-fold**

The impact of reducing the CA6 and CA16 MOI in iCELLis NANO cultures by 20-fold, from 0.02 down to 0.001, is evaluated. Reducing the MOI lowers the volume and costs of the corresponding virus seed bank.

Methods

To evaluate the impact of a 20-fold decrease of cell density at inoculation, two iCELLis NANO cultures are performed and infected at densities of either 0.25 or $0.5 \times 10E6$ cells/cm$^2$ using a MOI of 0.001. These cultures are then compared to iCELLis NANO cultures performed under similar conditions but infected with a MOI of 0.02. The viral productivity is evaluated using the TCID50 method.

**Example 20: Optimization study: determination of optimal cell density at CA6 and CA16 infection and productivity peak**

Cellular density at infection critically impacts viral productivity. The optimal cell density at CA6 and CA16 infection and the kinetics of virus release are evaluated in order to determine the productivity peak.

Methods

A series of five iCELLis NANO cultures are infected with CA6 and CA16 (MOI of 0.001) at different cell densities. The following culture conditions are used: cell density at inoculation: 15,000 cells/cm$^2$, growth media: Sigma D1145+10% FBS+ 4 mM glutamine, infection media: growth media without FBS, volume/surface ratio: 0.3 mL/cm$^2$. A full media replacement during the growth phase is made to reach the following densities: 0.55, 0.7 and $0.86 \times 10E6$ cells/cm$^2$. Cultures are fully monitored (glucose consumption, lactate production, cell density, DO, pH) and viral productivity is measured by the TCID50 method at each day after infection.

**Example 21: Optimization study: impact of decreasing cell density at CA6 and CA16 inoculation**

Potential benefits of reducing the volume of inoculum include: (i) less labor time and (ii) less risk of contamination. The impact of reducing the cell density at inoculation by 3-fold is evaluated.

Methods

An iCELLis NANO is inoculated with a low density of 5,000 cells/cm$^2$ and compared to an iCELLis NANO inoculated with a cell density of 15,000 cells/cm$^2$. Both iCELLis NANO are infected with CA6 and CA16 at a cellular density of 350,000 cells/cm$^2$ using an MOI of 0.001. Volume/surface ratios during growth and infections phases are 0.3 mL/cm$^2$.

**Example 22: Optimization study: impact of decreasing FBS concentration and volume/surface ratio during growth phase.**

The impact of reducing the culture volume used (reduction of the volume/surface ratio) or reducing the FBS concentration on cell growth or CA6 and CA16 viral productivity is evaluated.

Methods

A series of experiments is performed in T-flasks and cell density is monitored, together with glucose consumption and lactate production. A culture in iCELLis NANO using 5% FBS at growth phase and a low inoculation density of 5,000 cells/cm$^2$ is also performed. Cells are allowed to grow to a density of ~0.25×10E6 cells/cmand then CA6 and CA16 infected (MOI= 0.001). CA6 and CA16 viral productivity is assayed.

**Example 23: CA6 and CA16 process in iCELLis500/100**

The ability to scale-up CA6 and CA16 production, from iCELLis NANO (2 cm bed height) to iCELLis500/100 (2 cm bed height) is evaluated. The post-optimization cell culture parameters are utilized, with the exception that a 10% FBS concentration is used during the growth phase.

Methods

The run is performed using the iCELLis500/100 bio-reactor (2 cm bed height, 5L fixed-bed developing a surface of 100 m2), equipped with a single-use bio-mass probe and piloted by the iCELLis Skid. As controls, an iCELLis NANO and a Cell-Stack culture are performed using the same raw materials as those used for the iCELLis500/100 culture.

*Inoculum*

5 billion Vero cells (5x109) are used to seed the iCELLis500/100 at a density of 5,000 cells/cm$^2$. The inoculum is made through a series of amplification steps. 4 Cell-Stack 10 are used to produce the desired amount of inoculum for the iCELLis 100 m$^2$.

*Growth phase*

Growth phase is performed using 0.3 mL/cm$^2$ of DMEM media (4.5 g/L glucose) supplemented with 4 mM glutamine and 10 % FBS (no antibiotics are added) for 6 days, until cell density reaches the optimal density of $0.2 \times 10^6$ cells/cm$^2$. No media replacement is performed. Temperature is 37°C and pH is maintained at 7.4 by automated infection of $CO_2$ and NaOH. Density of oxygen is maintained above 50% by automated injection of air and/or oxygen.

The culture is performed in a recirculated batch mode using a total of 300 L which is divided as follow: 65 L in the bio-reactor and the remaining 235 L in a 500 L bag in a palletank. Recirculation between the bio-reactor and the bag is performed using the Skid's peristaltic pump at a rate of 0.25 L/min, which allows a complete recirculation of the culture media every 20 hours. Re-circulation is initiated 40 hours after seeding to (i) allow optimal fixation of the cells to the carriers and (ii) to reduce dilution of secreted growth factors.

A continuous monitoring of the following parameters is automatically performed by the Skid controller: T°, pH, DO before and after fixed-bed, conductivity, and bio-mass (capacitance). The bio-mass probe is used to evaluate the cell density (and therefore time for infection) and to follow the cytopathic effect. The amount of air, oxygen, $CO_2$, and NaOH consumed during the culture is also recorded. Daily samplings are performed to measure pH (off readings), glucose, and lactate concentrations.

*Infection phase*

Infection phase is performed in serum-free infection medium using 0.3 mL/cm$^2$ of DMEM (4.5 g/L glucose) supplemented with 4 mM glutamine, under identical T°, pH, and DO conditions as the growth phase.

Prior to infection, the fixed-bed of the bio-reactor is rinsed twice with 65 L of infection medium to remove FBS and other serum proteins. Infection is performed by mixing the appropriate amount of virus seed in the bio-reactor to reach a MOI of 0.001. During this step, the re-circulation between the bio-reactor and the bag is stopped for 4 hours, to prevent virus losses in the bag, and re-started.

Cell culture monitoring is performed identically as during the growth phase. In addition, samples are taken for QC analysis (TCID50, HCP, DNA quantification) in the reactor and in the re-circulation bag.

*Harvest and clarification*

In order to perform a virus kinetic study over a prolonged period of time, infection is allowed to proceed until 6 days post-infection (6 DPI). An intermediate harvest (40 L) is taken at 3 DPI and the final harvest is made at 6 DPI. Both harvests are clarified on Bio-20 (PALL) deep filters for subsequent DSP studies.

*iCELLis500 handling*

The iCELLis500/100 run is performed under sterile conditions, without antibiotics. All connections are performed aseptically using a Bio-Welder or aseptic connectors. Culture samples are taken under a laminar flow, using appropriate manifolds.

**Example 24: iCELLis500/100 with 5% FBS during growth phase**

CA6 and CA16 are produced in iCELLis500/100 as described in Example 23, with the exception that a reduced 5% FBS concentration is used.

**Example 25: CA6 and CA16 process in serum-free media (SFM)**

A fully serum-free process is used to produce CA6 and CA16. Cell growth and viral productivity (TCID50) are compared between serum-free (SFM, OptiPro) or 10% FBS media used during the growth phase. CA6 and CA16 production in serum-free conditions is evaluated in iCELLis NANO using a GMP Vero SFM-adapted cell line.

**Claims**

1.  A method for producing an Enterovirus A virus, comprising:

    (a) culturing an adherent cell in a fixed bed comprising a macrocarrier, wherein the cell is cultured in a first cell culture medium;
    (b) inoculating the cell with the Enterovirus A virus under conditions in which the Enterovirus A virus infects the cell;
    (c) culturing the infected cell in the fixed bed in a second cell culture medium under conditions in which the infected cell produces the Enterovirus A virus; and
    (d) harvesting the Enterovirus A virus produced by the cell, wherein (i) the cell is inoculated with the Enterovirus A virus at an MOI of between 0.025 and 0.0009, optionally 0.001, and/or (ii) between 200,000 cells/cm$^2$ and 350,000 cells/cm$^2$ are inoculated in step (b).

2.  The method of claim 1, wherein the cell is a mammalian cell, optionally a Vero cell, optionally wherein the Vero cell line is selected from the group consisting of WHO Vero 10-87, ATCC CCL-81, Vero 76 ATCC Accession No. CRL-1587, and Vero C1008 ATCC Accession No. CRL-1586.

3.  The method of claim 1 or 2, wherein the Enterovirus A virus is selected from the group consisting of EV71, CA6, and CA16.

4.  The method of claim 3, wherein the Enterovirus A virus comprises at least one non-human cell adaptation mutation, optionally wherein:

    i) the Enterovirus A virus is EV71, and the at least one non-human cell adaptation mutation comprises a VP1 polypeptide mutation; or
    ii) the Enterovirus A virus is CA6, and the at least one non-human cell adaptation mutation comprises a mutation selected from the group consisting of a VP1 polypeptide mutation, a VP2 polypeptide mutation, a VP3 polypeptide mutation, and a 5' untranslated region (UTR) mutation; or
    iii) the Enterovirus A virus is CA16, and the at least one non-human cell adaptation mutation comprises a mutation selected from the group consisting of a 2A polypeptide mutation, a VP1 polypeptide mutation, a VP2 polypeptide mutation, and a 5' untranslated region (UTR) mutation.

5.  The method of any one of claims 1-4, wherein:
    between 4,000 cells/cm$^2$ and 16,000 cells/cm$^2$ are cultured in step (a), optionally 5,000 cells/cm$^2$.

6.  The method of any one of claims 1-5, wherein the first cell culture medium comprises serum,
    optionally wherein the first cell culture medium comprises less than 10% fetal bovine serum, optionally 5% fetal bovine serum, and the cell is not adapted to serum free medium.

7.  The method of any one of claims 1-6, wherein the first cell culture medium and the second cell culture medium are different and the method optionally further comprises between steps (a) and (b), removing the first cell culture medium and rinsing the cell with the second culture medium.

8.  The method of any one of claims 1-7, wherein the second cell culture medium is a serum-free medium.

9.  The method of any one of claims 1-8, wherein the cell is cultured during steps (a), (b), and/or (c) at a volume/surface ratio of 0.3 mL/cm$^2$.

10. The method of any one of claims 1-9, wherein:

    i) the lactate concentration in the first cell culture medium during steps (a) and/or (b) does not exceed 25 mM and/or wherein the lactate concentration in the second cell culture medium during step (c) does not exceed 25 mM; and/or
    ii) the density of oxygen (DO) in the first cell culture medium during steps (a) and/or (b) is maintained above 50% and/or the density of oxygen (DO) in the second cell culture medium during step (c) is maintained above 50%.

11. The method of any one of claims 1-10, wherein the fixed bed has a bed height of 2 cm or 10 cm.

12. The method of any one of claims 1-11, wherein the macrocarrier is a microfiber matrix, optionally wherein:

    i) the macrocarrier has a porosity between 60% and 99%, optionally between 80% and 90%; and/or
    ii) the macrocarrier has a surface area accessible to the cell of between 150 $cm^2/cm^3$ and 1000 $cm^2/cm^3$.

13. The method of any one of claims 1-12, wherein at least $5.0 \times 10^7$ TCID50/mL of the Enterovirus A virus is harvested in step (d).

14. The method of any one of claims 1 to 13, wherein the Enterovirus A virus is an attenuated virus.

15. A method for producing and inactivating an Enterovirus A virus comprising producing the Enterovirus A virus using the method of any one of claims 1 to 14 and then inactivating the Enterovirus A virus with one or more of beta-propiolactone (BPL), formalin, or binary ethylenimine (BEI).

16. A method for producing an immunogenic composition or a vaccine, comprising producing an Enterovirus A virus according to any one of claims 1-14, or producing and inactivating an Enterovirus A virus according to claim 15, and further comprising formulating the virus into an immunogenic composition or vaccine.

**Patentansprüche**

1. Verfahren zur Herstellung eines Enterovirus-A-Virus, das Folgendes umfasst:

    (a) das Kultivieren einer adhärenten Zelle in einem Festbett, das einen Makroträger umfasst, wobei die Zelle in einem ersten Zellkulturmedium kultiviert wird;
    (b) das Inokulieren der Zelle mit dem Enterovirus-A-Virus unter Bedingungen, bei denen das Entervirus-A-Virus die Zelle infiziert;
    (c) das Kultivieren der infizierten Zelle in dem Festbett in einem zweiten Zellkulturmedium unter Bedingungen, bei denen die infizierte Zelle das Enterovirus-A-Virus produziert; und
    (d) das Abnehmen des von der Zelle produzierten Enterovirus-A-Virus, wobei (i) die Zelle mit einem MOI zwischen 0,025 und 0,0009, gegebenenfalls 0,001, mit dem Enterovirus-A-Virus inokuliert wird und/oder (ii) zwischen 200.000 Zellen/$cm^2$ und 350.000 Zellen/$cm^2$ in Schritt (b) inokuliert werden.

2. Verfahren nach Anspruch 1, wobei die Zelle eine Säugetierzelle, gegebenenfalls eine Vero-Zelle, ist, wobei die Vero-Zelllinie gegebenenfalls aus der aus WHO Vero 10-87, ATCC CCL-81, Vero 76 ATCC-Zugriffsnr. CRL-1587 und Vero C1008 ATCC-Zugriffsnr. CRL-1586 bestehenden Gruppe ausgewählt ist.

3. Verfahren nach Anspruch 1 oder 2, wobei das Enterovirus-A-Virus aus der aus EV71, CA6 und CA16 bestehenden Gruppe ausgewählt ist.

4. Verfahren nach Anspruch 3, wobei das Enterovirus-A-Virus zumindest eine nichtmenschliche Zellanpassungsmutation umfasst, gegebenenfalls wobei:

    i) das Enterovirus-A-Virus EV71 ist und die zumindest eine nichtmenschliche Zellanpassungsmutation eine VP1-Polypeptidmutation umfasst; oder
    ii) das Enterovirus-A-Virus CA6 ist und die zumindest eine nichtmenschliche Zellanpassungsmutation eine Mutation umfasst, die aus der aus einer VP1-Polypeptidmutation, einer VP2-Polypeptidmutation, einer VP3-Polypeptidmutation und der Mutation einer untranslatierten 5'-Region (UTR) bestehenden Gruppe ausgewählt ist; oder
    iii) das Enterovirus-A-Virus CA16 ist und die zumindest eine nichtmenschliche Zellanpassungsmutation eine Mutation umfasst, die aus der aus einer 2A-Polypeptidmutation, einer VP1-Polypeptidmutation, einer VP2-Polypeptidmutation und der Mutation einer untranslatierten 5'-Region (UTR) bestehenden Gruppe ausgewählt ist.

5. Verfahren nach einem der Ansprüche 1 bis 4, wobei in Schritt (a) zwischen 4.000 Zellen/$cm^2$ und 16.000 Zellen/$cm^2$, gegebenenfalls 5.000 Zellen/$cm^2$, kultiviert werden.

6. Verfahren nach einem der Ansprüche 1 bis 5, wobei das erste Zellkulturmedium Serum umfasst, wobei gegebenenfalls das erste Zellkulturmedium weniger als 10 % Rinderfötenserum, gegebenenfalls 5 % Rinderfötenserum, umfasst und die Zelle nicht an serumfreies Medium angepasst ist.

7. Verfahren nach einem der Ansprüche 1 bis 6, wobei sich das erste Zellkulturmedium von dem zweiten Zellkulturmedium unterscheidet und das Verfahren gegebenenfalls zwischen den Schritten (a) und (b) außerdem das Entfernen des ersten Zellkulturmediums und Spülen der Zelle mit dem zweiten Kulturmedium umfasst.

8. Verfahren nach einem der Ansprüche 1 bis 7, wobei das zweite Zellkulturmedium ein serumfreies Zellkulturmedium ist.

9. Verfahren nach einem der Ansprüche 1 bis 8, wobei die Zelle während der Schritte (a), (b) und/oder (c) mit einem Volumen/Oberflächen-Verhältnis von 0,3 ml/cm$^2$ kultiviert wird.

10. Verfahren nach einem der Ansprüche 1 bis 9, wobei:

i) die Lactatkonzentration in dem ersten Zellkulturmedium während der Schritte (a) und/oder (b) 25 mM nicht übersteigt und/oder wobei die Lactatkonzentration in dem zweiten Zellkulturmedium während Schritt (c) 25 mM nicht übersteigt; und/oder
ii) die Sauerstoffdichte (DO) in dem ersten Zellkulturmedium während der Schritte (a) und/oder (b) über 50 % gehalten wird und/oder die Sauerstoffdichte (DO) in dem zweiten Zellkulturmedium während Schritt (c) über 50 % gehalten wird.

11. Verfahren nach einem der Ansprüche 1 bis 10, wobei das Festbett eine Betthöhe von 2 cm oder 10 cm aufweist.

12. Verfahren nach einem der Ansprüche 1 bis 11, wobei der Makroträger eine Mikrofasermatrix ist, gegebenenfalls wobei:

i) der Makroträger eine Porosität zwischen 60 % und 99 %, gegebenenfalls zwischen 80 % und 90 %, aufweist; und/oder
ii) der Makroträger eine der Zelle zugängliche Oberfläche zwischen 150 cm$^2$/cm$^3$ und 1000 cm$^2$/cm$^3$ aufweist.

13. Verfahren nach einem der Ansprüche 1 bis 12, wobei in Schritt (d) zumindest $5,0 \times 10^7$ TCID50/ml des Enterovirus-A-Virus abgenommen werden.

14. Verfahren nach einem der Ansprüche 1 bis 13, wobei das Enterovirus-A-Virus ein attenuiertes Virus ist.

15. Verfahren zur Herstellung und Inaktivierung eines Enterovirus-A-Virus, das das Herstellen des Enterovirus-A-Virus unter Verwendung eines Verfahrens nach einem der Ansprüche 1 bis 14 und dann das Inaktivieren des Enterovirus-A-Virus mit einem oder mehreren aus beta-Propiolacton (BPL), Formalin oder binärem Ethylenimin (BEI) umfasst.

16. Verfahren zur Herstellung einer immunogenen Zusammensetzung oder einer Vakzine, das das Herstellen eines Enterovirus-A-Virus nach einem der Ansprüche 1 bis 14 oder das Herstellen und Inaktivieren eines Enterovirus-A-Virus nach Anspruch 15 und außerdem das Formulieren des Virus zu einer immunogenen Zusammensetzung oder Vakzine umfasst.

**Revendications**

1. Procédé de production d'un virus Entérovirus A, comprenant les étapes consistant à :

(a) cultiver une cellule adhérente dans un lit fixe comprenant un macro-support, dans lequel la cellule est cultivée dans un premier milieu de culture cellulaire ;
(b) inoculer la cellule avec le virus Entérovirus A dans des conditions dans lesquelles le virus Entérovirus A infecte la cellule ;
(c) cultiver la cellule infectée dans le lit fixe dans un second milieu de culture cellulaire dans des conditions dans lesquelles la cellule infectée produit le virus Entérovirus A ; et
(d) récolter le virus Entérovirus A produit par la cellule, dans lequel (i) la cellule est inoculée avec le virus Entérovirus A à une MOI comprise entre 0,025 et 0,0009, facultativement 0,001, et/ou (ii) entre 200 000 cellu-

les/cm$^2$ et 350 000 cellules/cm$^2$ sont inoculées à l'étape (b).

2. Procédé selon la revendication 1, dans lequel la cellule est une cellule de mammifère, facultativement une cellule Vero, facultativement dans lequel la lignée cellulaire Vero est choisie dans le groupe constitué de WHO Vero 10-87, ATCC CCL-81, Vero 76 Numéro d'Accession ATCC CRL-1587, et Vero C1008 Numéro d'Accession ATCC CRL-1586.

3. Procédé selon la revendication 1 ou 2, dans laquelle le virus Entérovirus A est choisi dans le groupe constitué de EV71, CA6, et CA16.

4. Procédé selon la revendication 3, dans lequel le virus Entérovirus A comprend au moins une mutation d'adaptation cellulaire non humaine, dans lequel facultativement :

   i) le virus Entérovirus A est EV71, et la au moins une mutation d'adaptation cellulaire non humaine comprend une mutation de polypeptide VP1 ; ou
   ii) le virus Entérovirus A est CA6, et la au moins une mutation d'adaptation cellulaire non humaine comprend une mutation choisie dans le groupe constitué d'une mutation de polypeptide VP1, d'une mutation de polypeptide VP2, d'une mutation de polypeptide VP3, et d'une mutation de région non traduite (UTR) en 5' ; ou
   iii) le virus Entérovirus A est CA16, et la au moins une mutation d'adaptation cellulaire non humaine comprend une mutation choisie dans le groupe constitué d'une mutation de polypeptide 2A, d'une mutation de polypeptide VP1, une mutation de polypeptide VP2, et d'une mutation de région non traduite (UTR) en 5'.

5. Procédé selon l'une quelconque des revendications 1 à 4, dans lequel :
   entre 4 000 cellules/cm$^2$ et 16 000 cellules/cm$^2$ sont cultivées à l'étape (a), facultativement 5 000 cellules/cm$^2$.

6. Procédé selon l'une quelconque des revendications 1 à 5, dans lequel le premier milieu de culture cellulaire comprend du sérum,
   facultativement dans lequel le premier milieu de culture cellulaire comprend moins de 10 % de sérum bovin fœtal, facultativement 5 % de sérum bovin fœtal, et la cellule n'est pas adaptée à un milieu sans sérum.

7. Procédé selon l'une quelconque des revendications 1 à 6, dans lequel le premier milieu de culture cellulaire et le second milieu de culture cellulaire sont différents et le procédé comprend en outre facultativement entre les étapes (a) et (b), le retrait du premier milieu de culture cellulaire et le rinçage de la cellule avec le second milieu de culture.

8. Procédé selon l'une quelconque des revendications 1 à 7, dans lequel le second milieu de culture cellulaire est un milieu sans sérum.

9. Procédé selon l'une quelconque des revendications 1 à 8, dans lequel la cellule est cultivée au cours des étapes (a), (b), et/ou (c) à un rapport volume/surface de 0,3 ml/cm$^2$.

10. Procédé selon l'une quelconque des revendications 1 à 9, dans lequel :

    i) la concentration en lactate dans le premier milieu de culture cellulaire au cours des étapes (a) et/ou (b) ne dépasse pas 25 mM et/ou dans lequel la concentration en lactate dans le second milieu de culture cellulaire au cours de l'étape (c) ne dépasse pas 25 mM ; et/ou
    ii) la densité d'oxygène (DO) dans le premier milieu de culture cellulaire au cours des étapes (a) et/ou (b) est maintenue au-dessus de 50 % et/ou la densité d'oxygène (DO) dans le second milieu de culture cellulaire au cours de l'étape (c) est maintenue au-dessus de 50 %.

11. Procédé selon l'une quelconque des revendications 1 à 10, dans lequel le lit fixe a une hauteur de lit de 2 cm ou 10 cm.

12. Procédé selon l'une quelconque des revendications 1 à 11, dans lequel le macro-support est une matrice de microfibres, facultativement dans lequel :

    i) le macro-support a une porosité comprise entre 60 % et 99 %, facultativement entre 80 % et 90 % ; et/ou
    ii) le macro-support a une surface superficielle accessible à la cellule comprise entre 150 cm$^2$/cm$^3$ et 1 000 cm$^2$/cm$^3$.

**13.** Procédé selon l'une quelconque des revendications 1 à 12, dans lequel au moins 5,0 × 10$^7$ TCID50/ml du virus Entérovirus A sont récoltés à l'étape (d).

**14.** Procédé selon l'une quelconque des revendications 1 à 13, dans lequel le virus Entérovirus A est un virus atténué.

**15.** Procédé de production et d'inactivation d'un virus Entérovirus A comprenant la production du virus Entérovirus A en utilisant le procédé selon l'une quelconque des revendications 1 à 14, puis l'inactivation du virus Entérovirus A avec un ou plusieurs parmi la bêta-propiolactone (BPL), formol, ou éthylèneimine binaire (BEI).

**16.** Procédé de production d'une composition immunogène ou d'un vaccin, comprenant la production d'un virus Entérovirus A selon l'une quelconque des revendications 1 à 14, ou la production et l'inactivation d'un virus Entérovirus A selon la revendication 15, et comprenant en outre la formulation du virus en un composition immunogène ou vaccin.

FIG. 1

FIG. 2C

Carrier at Day 6

FIG. 2B

Carrier at Day 3

FIG. 2A

Empty carrier

FIG. 3

EP 3 256 571 B1

FIG. 4

FIG. 5

FIG. 6

FIG. 7

FIG. 8

FIG. 9

FIG. 10

FIG. 11

FIG. 12

FIG. 13

NANO 40 compaction 1

0.015 E6/cm2
0.31 ml/cm2
37° C / pH 7.4
DMEM 4.5g/l
FBS10%

0.135 E6/cm2
0.31 ml/cm2
37° C / pH 7.4
DMEM 4.5g/l
FBS10%

Medium replacement

0.55 E6/cm2
0.62 ml/cm2
34° C / pH 7.4
M199 1g/l
No FBS

Polio S2 infection
MOI 0.002

Termination

0       3       6       13

FIG. 14

FIG. 15

FIG. 16

FIG. 17

FIG. 18

FIG. 19

FIG. 20

FIG. 21

FIG. 22

FIG. 23B

FIG. 23A

FIG. 23D

FIG. 23C

FIG. 24

EP 3 256 571 B1

FIG. 25

FIG. 26

FIG. 27

EV71 process in iCELLis500/100

FIG. 28

FIG. 29

FIG. 30A

FIG. 30B

FIG. 30C

**FIG. 30D**

**FIG. 30E**

**FIG. 30F**

FIG. 31A

FIG. 31B

FIG. 31C

FIG. 32

FIG. 33

FIG. 34

EV71 process in iCELLis500/500

WCB thawing
$10^7$ cells in TF

$\xrightarrow{\text{4Days}}$ 1 CF2 $\xrightarrow{\text{4Days}}$ 2 CF10

Xpansion™

16 CF10

Vero Amplification

FIG. 35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120045468 A1
- WO 9737001 A
- WO 0138362 A
- WO 0240665 A
- WO 9737000 A
- WO 2006027698 A
- US 8597939 B
- US 8137959 B
- US 20080248552 A
- WO 2014093444 A
- US 6500419 B, Hone
- WO 0032047 A, Galen
- WO 02083890 A, Galen
- EP 0689454 B1
- US 5057540 A
- EP 0362279 B1
- US 3238190 A, Erbring
- WO 9602555 A
- WO 9933488 A
- US 6008200 A
- US 5856462 A
- US 5666153 A
- US 5278302 A

- WO 9526204 A
- WO 0009159 A
- EP 399843 B
- WO 9517210 A
- WO 9856414 A
- WO 9912565 A
- WO 9911241 A
- US 5422109 A
- EP 0480982 B2
- US 5424067 A
- EP 0480981 B
- EP 0382271 B1
- US 853826
- US 09074720 B
- WO 9952549 A
- WO 9927961 A
- US 4596556 A
- US 5993412 A
- WO 9748440 A
- WO 9828037 A
- WO 9820734 A
- WO 62116361 A

**Non-patent literature cited in the description**

- **HUANG, CC et al.** *N Engl J Med,* 1999, vol. 341, 936-942
- **CHANG, LY et al.** *Lancet,* 1998, vol. 352, 367-368
- **OOI, MH et al.** *BMC Infect Dis,* 2009, vol. 9, 3
- **SOLOMON, T et al.** *Lancet Infect Dis,* 2010, vol. 10 (11), 778-90
- **MCMINN, PC.** *FEMS Microbiol Rev,* 2002, vol. 26, 91-107
- **PALLANSCH ; ROOS.** Fields Virology. Lippincott Williams & Wilkins, 2007, 839-894
- **KOBAYASHI M et al.** *Jpn J Infect Dis,* 2013, vol. 66, 260-1
- **NAGATA, N et al.** *J Med Virol,* 2002, vol. 67, 207-216
- **YAMAYOSHI, S et al.** *Nat Med,* 2009, vol. 15, 798-801
- **NISHIMURA, Y et al.** *Nat Med,* 2009, vol. 15, 794-797
- **OBERSTE, MS et al.** *J Clin Microbiol,* 1999, vol. 37, 1288-1293

- **LEE, MS et al.** *PLoS Negl Trop Dis,* 2012, vol. 6, e1737
- **BROWN, BA et al.** *J Virol,* 1999, vol. 73, 9969-9975
- **CHAN, YF et al.** *Infect Genet Evol,* 2009, vol. 10 (3), 404-12
- **YOKE-FUN ; ABUBAKAR.** *BMC Microbiol,* 2006, vol. 6, 74
- **WU et al.** *Vaccine,* 2004, vol. 22, 3858-2864
- *BMC Infect Dis,* 2009, vol. 9, 3
- **WANG, SM et al.** *Clin Infect Dis,* 1999, vol. 29, 184-190
- **SAMBROOK et al.** Molecular Cloning: A Laboratory Manual. Cold Spring Harbor Laboratory Press, 2001
- Current Protocols in Molecular Biology. 2003
- PCR 2: A Practical Approach. Methods in Enzymology. Academic Press, Inc, 1995
- Antibodies, A Laboratory Manual. 1988
- Animal Cell Culture. 1987
- Oligonucleotide Synthesis. 1984

- Methods in Molecular Biology. Humana Press
- Cell Biology: A Laboratory Notebook. Academic Press, 1998
- **J.P. MATHER ; P.E. ROBERTS.** Introduction to Cell and Tissue Culture. Plenum Press, 1998
- Cell and Tissue Culture: Laboratory Procedures. J. Wiley and Sons, 1993
- Handbook of Experimental Immunology
- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- **C.A. JANEWAY ; P. TRAVERS.** Immunobiology. 1997
- **P. FINCH.** Antibodies. 1997
- Antibodies: A Practical Approach. IRL Press, 1988
- Monoclonal Antibodies: A Practical Approach. Oxford University Press, 2000
- **E. HARLOW ; D. LANE.** Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- **EAGLE.** *Science,* 1959, vol. 130, 432
- **JONES.** The Chemical Synthesis of Peptides. Clarendon Press, 1994
- **GENNARO.** Remington: The Science and Practice of Pharmacy. 2000
- The Theory and Practical Application of Adjuvants. John Wiley & Sons Ltd, 1995
- Vaccines: New Generation Immunological Adjuvants. Plenum Press, 1995
- **NICKLAS.** *Res. Immunol.,* 1992, vol. 143, 489-493
- **MOREIN B et al.** *Clin. Immunother.,* 1995, vol. 3, 461-475
- **BARR I G ; MITCHELL G F.** *Immunol. and Cell Biol.,* 1996, vol. 74, 8-25
- **KENSIL, C. R.** Saponins as vaccine adjuvants. *Crit Rev Ther Drug Carrier Syst,* 1996, vol. 12 (1-2), 1-55
- **FIEDLER.** *Arzneimittel-Forsch,* 1953, vol. 4, 213
- **YOSHIKAWA M et al.** *Chem Pharm Bull (Tokyo),* August 1996, vol. 44 (8), 1454-1464
- **GISVOLD et al.** *J.Am.Pharm.Assoc.,* 1934, vol. 23, 664
- **RUHENSTROTH-BAUER.** *Physiol.Chem.,* 1955, vol. 301, 621
- **GELBER C et al.** Elicitation of Robust Cellular and Humoral Immune Responses to Small Amounts of Immunogens Using a Novel Medical Device Designated the Virtual Lymph Node. *From the Laboratory to the Clinic, Book of Abstracts,* 12 October 1998
- Dorland's Illustrated Medical Dictionary. W.B. Sanders Company, 1974
- **GRANOFF et al.** *Infect Immun.,* 1997, vol. 65 (5), 1710-1715
- **NILSSON ; LARSSON.** *Res. Immunol.,* 1992, vol. 143, 553-557
- **O'HAGAN et al.** *Biomol Eng.,* 2001, vol. 18 (3), 69-85
- Vaccine Adjuvants: Preparation Methods and Research Protocols. Humana Press, 2000

SEQUENCE LISTING

<110> RAO, Raman

<120> METHODS FOR PRODUCING VIRUS FOR VACCINE
PRODUCTION

<130> 606772001140

<140> Not yet assigned
<141> Concurrently Herewith

<150> 62/116,361 <151> 2015-02-13

<160> 14

<170> FastSEQ for Windows Version 4.0

<210> 1
<211> 297
<212> PRT
<213> Enterovirus A

<400> 1

```
Gly Asp Arg Val Ala Asp Val Ile Glu Ser Ser Ile Gly Asp Ser Val
1               5                   10                  15
Ser Arg Ala Leu Thr Gln Ala Leu Pro Ala Pro Thr Gly Gln Asn Thr
            20                  25                  30
Gln Val Ser Ser His Arg Leu Asp Thr Gly Glu Val Pro Ala Leu Gln
            35                  40                  45
Ala Ala Glu Ile Gly Ala Ser Ser Asn Thr Ser Asp Glu Ser Met Ile
            50                  55                  60
Glu Thr Arg Cys Val Leu Asn Ser His Ser Thr Ala Glu Thr Thr Leu
65                  70                  75                  80
Asp Ser Phe Phe Ser Arg Ala Gly Leu Val Gly Glu Ile Asp Leu Pro
                85                  90                  95
Leu Glu Gly Thr Thr Asn Pro Asn Gly Tyr Ala Asn Trp Asp Ile Asp
            100                 105                 110
Ile Thr Gly Tyr Ala Gln Met Arg Arg Lys Val Glu Leu Phe Thr Tyr
            115                 120                 125
Met Arg Phe Asp Ala Glu Phe Thr Phe Val Ala Cys Thr Pro Thr Gly
    130                 135                 140
Glu Val Val Pro Gln Leu Leu Gln Tyr Met Phe Val Pro Pro Gly Ala
145                 150                 155                 160
Pro Lys Pro Glu Ser Arg Glu Ser Leu Ala Trp Gln Thr Ala Thr Asn
            165                 170                 175
Pro Ser Val Phe Val Lys Leu Thr Asp Pro Pro Ala Gln Val Ser Val
            180                 185                 190
Pro Phe Met Ser Pro Ala Ser Ala Tyr Gln Trp Phe Tyr Asp Gly Tyr
            195                 200                 205
Pro Thr Phe Gly Glu His Lys Gln Glu Lys Asp Leu Glu Tyr Gly Ala
    210                 215                 220
Cys Pro Asn Asn Met Met Gly Thr Phe Ser Val Arg Thr Val Gly Ser
225                 230                 235                 240
Ser Lys Ser Lys Tyr Pro Leu Val Val Arg Ile Tyr Met Arg Met Lys
            245                 250                 255
His Val Arg Ala Trp Ile Pro Arg Pro Met Arg Asn Gln Asn Tyr Leu
            260                 265                 270
Phe Lys Ala Asn Pro Asn Tyr Ala Gly Asn Ser Ile Lys Pro Thr Gly
    275                 280                 285
Thr Ser Arg Asn Ala Ile Thr Thr Leu
```

                290                 295


<210> 2
<211> 305
<212> PRT
<213> Coxsackievirus A6

<400> 2

```
Asn Asp Pro Ile Ser Asn Ala Ile Glu Asn Ala Val Ser Thr Leu Ala
1               5               10              15
Asp Thr Thr Ile Ser Arg Val Thr Ala Ala Asn Thr Ala Ala Ser Ser
        20              25              30
His Ser Leu Gly Thr Gly Arg Val Pro Ala Leu Gln Ala Ala Glu Thr
        35              40              45
Gly Ala Ser Ser Asn Ala Ser Asp Glu Asn Leu Ile Glu Thr Arg Cys
    50              55              60
Val Met Asn Arg Asn Gly Val Asn Glu Ala Ser Val Glu His Phe Tyr
65              70              75              80
Ser Arg Ala Gly Leu Val Gly Val Val Glu Val Lys Asp Ser Gly Thr
            85              90              95
Ser Gln Asp Gly Tyr Thr Val Trp Pro Ile Asp Val Met Gly Phe Val
            100             105             110
Gln Gln Arg Arg Lys Leu Glu Leu Ser Thr Tyr Met Arg Phe Asp Ala
    115             120             125
Glu Phe Thr Phe Val Ser Asn Leu Asn Asp Ser Thr Thr Pro Gly Met
    130             135             140
Leu Leu Gln Tyr Met Tyr Val Pro Pro Gly Ala Pro Lys Pro Asp Gly
145             150             155             160
Arg Lys Ser Tyr Gln Trp Gln Thr Ala Thr Asn Pro Ser Ile Phe Ala
            165             170             175
Lys Leu Ser Asp Pro Pro Pro Gln Val Ser Val Pro Phe Met Ser Pro
            180             185             190
Ala Ser Ala Tyr Gln Trp Phe Tyr Asp Gly Tyr Pro Thr Phe Gly Glu
            195             200             205
His Lys Gln Ala Thr Asn Leu Gln Tyr Gly Gln Cys Pro Asn Asn Met
    210             215             220
Met Gly His Phe Ala Ile Arg Thr Val Ser Glu Ser Thr Thr Gly Lys
225             230             235             240
Asn Val His Val Arg Val Tyr Met Arg Ile Lys His Val Arg Ala Trp
            245             250             255
Val Pro Arg Pro Phe Arg Ser Gln Ala Tyr Met Val Lys Asn Tyr Pro
            260             265             270
Thr Tyr Ser Gln Thr Ile Ser Asn Thr Ala Ala Asp Arg Ala Ser Ile
    275             280             285
Thr Thr Thr Asp Tyr Glu Gly Gly Val Pro Ala Asn Pro Gln Arg Thr
    290             295             300
Phe
305
```

<210> 3

<211> 256

<212> PRT

<213> Coxsackievirus A6

<400> 3

```
Ser Pro Ser Val Glu Ala Cys Gly Tyr Ser Asp Arg Val Ala Gln Leu
1               5               10              15
Thr Val Gly Asn Ser Thr Ile Thr Thr Gln Glu Ala Ala Asn Ile Val
        20              25              30
Leu Ser Tyr Gly Glu Trp Pro Gly Tyr Cys Pro Ser Thr Asp Ala Thr
        35              40              45
```

```
Ala Val Asp Lys Pro Thr Arg Pro Asp Val Ser Val Asn Arg Phe Tyr
    50                  55                  60
Thr Leu Ser Thr Lys Ser Trp Lys Thr Glu Ser Thr Gly Trp Tyr Trp
65                  70                  75                  80
Lys Phe Pro Asp Val Leu Asn Asp Thr Gly Val Phe Gly Gln Asn Ala
                85                  90                  95
Gln Phe His Tyr Leu Tyr Arg Ser Gly Phe Cys Met His Val Gln Cys
            100                 105                 110
Asn Ala Ser Lys Phe His Gln Gly Ala Leu Leu Val Val Val Ile Pro
            115                 120                 125
Glu Phe Val Val Ala Ala Ser Ser Pro Ala Met Lys Pro Asn Gly Gln
    130                 135                 140
Gly Leu Tyr Pro Asp Phe Ala His Thr Asn Pro Gly Lys Glu Gly Gln
145                 150                 155                 160
Val Phe Arg Asp Pro Tyr Val Leu Asp Ala Gly Ile Pro Leu Ser Gln
            165                 170                 175
Ala Leu Val Phe Pro His Gln Trp Ile Asn Leu Arg Thr Asn Asn Cys
            180                 185                 190
Ala Thr Ile Ile Met Pro Tyr Val Asn Ala Leu Pro Phe Asp Ser Ala
            195                 200                 205
Leu Asn His Ser Asn Phe Gly Leu Ala Val Ile Pro Ile Ser Pro Leu
    210                 215                 220
Lys Tyr Cys Asn Gly Ala Thr Thr Glu Val Pro Ile Thr Leu Thr Ile
225                 230                 235                 240
Ala Pro Leu Asn Ser Glu Phe Ser Gly Leu Arg Gln Ala Ile Lys Gln
                245                 250                 255
```

<210> 4
<211> 240
<212> PRT
<213> Coxsackievirus A6

<400> 4

```
Gly Leu Pro Thr Glu Leu Lys Pro Gly Thr Asn Gln Phe Leu Thr Thr
1               5                   10                  15
Asp Asp Gly Thr Ser Pro Pro Ile Leu Pro Gly Phe Glu Pro Thr Pro
            20                  25                  30
Leu Ile His Ile Pro Gly Glu Phe Thr Ser Leu Leu Asp Leu Cys Arg
            35                  40                  45
Ile Glu Thr Ile Leu Glu Val Asn Asn Thr Thr Gly Thr Thr Gly Val
            50                  55                  60
Asn Arg Leu Leu Ile Pro Val Arg Ala Gln Asn Asn Val Asp Gln Leu
65                  70                  75                  80
Cys Ala Ser Phe Gln Val Asp Pro Gly Arg Asn Gly Pro Trp Gln Ser
                85                  90                  95
Thr Met Val Gly Gln Ile Cys Arg Tyr Tyr Thr Gln Trp Ser Gly Ser
            100                 105                 110
Leu Lys Val Thr Phe Met Phe Thr Gly Ser Phe Met Ala Thr Gly Lys
            115                 120                 125
Met Leu Ile Ala Tyr Thr Pro Pro Gly Ser Ala Gln Pro Thr Thr Arg
            130                 135                 140
Glu Ala Ala Met Leu Gly Thr His Ile Val Trp Asp Phe Gly Leu Gln
145                 150                 155                 160
Ser Ser Val Thr Leu Val Ile Pro Trp Ile Ser Asn Thr His Phe Arg
                165                 170                 175
Ala Val Lys Thr Gly Gly Val Tyr Asp Tyr Tyr Ala Thr Gly Ile Val
            180                 185                 190
Thr Ile Trp Tyr Gln Thr Asn Phe Val Val Pro Pro Asp Thr Pro Ser
            195                 200                 205
Glu Ala Asn Ile Ile Ala Leu Gly Ala Ala Gln Glu Asn Phe Thr Leu
210                 215                 220
Lys Leu Cys Lys Asp Thr Asp Glu Ile Arg Gln Thr Ala Glu Tyr Gln

225                 230                 235                 240


<210> 5
<211> 150
<212> PRT
<213> Coxsackievirus A16

<400> 5
Gly Lys Phe Gly Gln Gln Ser Gly Ala Ile Tyr Val Gly Asn Tyr Arg
1               5                   10                  15
Val Val Asn Arg His Leu Ala Thr His Asn Asp Trp Ala Asn Leu Val
            20                  25                  30
Trp Glu Asp Ser Ser Arg Asp Leu Leu Val Ser Ser Thr Thr Ala Gln
            35                  40                  45
Gly Cys Asp Thr Ile Ala Arg Cys Asp Cys Gln Thr Gly Ile Tyr Tyr
            50                  55                  60
Cys Ser Ser Lys Arg Lys His Tyr Pro Val Ser Phe Thr Lys Pro Ser
65                  70                  75                  80
Leu Ile Phe Val Glu Ala Ser Glu Tyr Tyr Pro Ala Arg Tyr Gln Ser
                85                  90                  95
His Leu Met Leu Ala Val Gly His Ser Glu Pro Gly Asp Cys Gly Gly
            100                 105                 110
Ile Leu Arg Cys Gln His Gly Val Val Gly Ile Val Ser Thr Gly Gly
            115                 120                 125
Asn Gly Leu Val Gly Phe Ala Asp Val Arg Asp Leu Leu Trp Leu Asp
            130                 135                 140
Glu Glu Ala Met Glu Gln
145                 150
```

<210> 6
<211> 254
<212> PRT
<213> Coxsackievirus A16

<400> 6

```
Ser Pro Ser Ala Glu Ala Cys Gly Tyr Ser Asp Arg Val Ala Gln Leu
1               5                   10                  15
Thr Ile Gly Asn Ser Thr Ile Thr Thr Gln Glu Ala Ala Asn Ile Val
            20                  25                  30
Ile Ala Tyr Gly Glu Trp Pro Glu Tyr Cys Pro Asp Thr Asp Ala Thr
            35                  40                  45
Ala Val Asp Lys Pro Thr Arg Pro Asp Val Ser Val Asn Arg Phe Phe
        50                  55                  60
Thr Leu Asp Thr Lys Ser Trp Ala Lys Asp Ser Lys Gly Trp Tyr Trp
65                  70                  75                  80
Lys Phe Pro Asp Val Leu Thr Glu Val Gly Val Phe Gly Gln Asn Ala
                85                  90                  95
Gln Phe His Tyr Leu Tyr Arg Ser Gly Phe Cys Val His Val Gln Cys
            100                 105                 110
Asn Ala Ser Lys Phe His Gln Gly Ala Leu Leu Val Ala Val Leu Pro
            115                 120                 125
Glu Tyr Val Leu Gly Thr Ile Ala Gly Gly Thr Gly Asn Glu Asn Ser
    130                 135                 140
His Pro Pro Tyr Ala Thr Thr Gln Pro Gly Gln Val Gly Ala Val Leu
145                 150                 155                 160
Met His Pro Tyr Val Leu Asp Ala Gly Ile Pro Leu Ser Gln Leu Thr
                165                 170                 175
Val Cys Pro His Gln Trp Ile Asn Leu Arg Thr Asn Asn Cys Ala Thr
                180                 185                 190
Ile Ile Val Pro Tyr Met Asn Thr Val Pro Phe Asp Ser Ala Leu Asn
            195                 200                 205


His Cys Asn Phe Gly Leu Leu Val Ile Pro Val Val Pro Leu Asp Phe
    210                 215                 220
Asn Ala Gly Ala Thr Ser Glu Ile Pro Ile Thr Val Thr Ile Ala Pro
225                 230                 235                 240
Met Cys Ala Glu Phe Ala Gly Leu Arg Gln Ala Val Lys Gln
                245                 250
```

<210> 7
<211> 297
<212> PRT
<213> Coxsackievirus A16

<400> 7

```
Gly Asp Pro Ile Ala Asp Met Ile Asp Gln Thr Val Asn Asn Gln Val
1               5                   10                  15
Asn Arg Ser Leu Thr Ala Leu Gln Val Leu Pro Thr Ala Ala Asn Thr
            20                  25                  30
Glu Ala Ser Ser His Arg Leu Gly Thr Gly Val Val Pro Ala Leu Gln
            35                  40                  45
Ala Ala Glu Thr Gly Ala Ser Ser Asn Ala Ser Asp Lys Asn Leu Ile
        50                  55                  60
Glu Thr Arg Cys Val Leu Asn His His Ser Thr Gln Glu Thr Ala Ile
65                  70                  75                  80
Gly Asn Phe Phe Ser Arg Ala Gly Leu Val Ser Ile Ile Thr Met Pro
                85                  90                  95
Thr Thr Asp Thr Gln Asn Thr Asp Gly Tyr Val Asn Trp Asp Ile Asp
            100                 105                 110
Leu Met Gly Tyr Ala Gln Leu Arg Arg Lys Cys Glu Leu Phe Thr Tyr
        115                 120                 125
Met Arg Phe Asp Ala Glu Phe Thr Phe Val Val Ala Lys Pro Asn Gly
    130                 135                 140
Val Leu Val Pro Gln Leu Leu Gln Tyr Met Tyr Val Pro Pro Gly Ala
145                 150                 155                 160
Pro Lys Pro Thr Ser Arg Asp Ser Phe Ala Trp Gln Thr Ala Thr Asn
                165                 170                 175
Pro Ser Val Phe Val Lys Met Thr Asp Pro Pro Ala Gln Val Ser Val
                180                 185                 190
Pro Phe Met Ser Pro Ala Ser Ala Tyr Gln Trp Phe Tyr Asp Gly Tyr
        195                 200                 205
Pro Thr Phe Gly Glu His Leu Gln Ala Asn Asp Leu Asp Tyr Gly Gln
    210                 215                 220
Cys Pro Asn Asn Met Met Gly Thr Phe Ser Ile Arg Thr Val Gly Thr
225                 230                 235                 240
Glu Lys Ser Pro His Ser Ile Thr Leu Arg Val Tyr Met Arg Ile Lys
                245                 250                 255
His Val Arg Ala Trp Ile Pro Arg Pro Leu Arg Asn Gln Pro Tyr Leu
            260                 265                 270
Phe Lys Thr Asn Pro Asn Tyr Lys Gly Asn Asp Ile Lys Cys Thr Ser
        275                 280                 285
Thr Ser Arg Asp Lys Ile Thr Thr Leu
    290                 295
```

<210> 8
<211> 739
<212> PRT
<213> Coxsackievirus A6

<400> 8

```
Thr Thr Ala Ala Ala Ala Cys Ala Gly Cys Thr Ala Gly Thr Gly Gly
1               5                   10                  15
Gly Thr Thr Gly Cys Ala Cys Cys Cys Ala Cys Thr Cys Ala Cys Ala
```

58

```
                        20                          25                          30
        Gly Gly Gly Cys Cys Cys Ala Cys Thr Gly Gly Gly Cys Gly Cys Thr
                35                          40                          45
        Ala Gly Cys Ala Cys Ala Cys Thr Gly Ala Thr Thr Thr Cys Cys Cys
                50                          55                          60
        Gly Gly Ala Ala Thr Cys Thr Thr Gly Thr Gly Cys Gly Cys Cys
        65                      70                      75                      80
        Thr Gly Thr Thr Thr Thr Ala Thr Ala Thr Cys Cys Cys Cys Thr Cys
                        85                          90                          95
        Cys Cys Cys Cys Ala Thr Gly Cys Gly Cys Ala Ala Cys Thr Thr Ala
                        100                         105                         110
        Gly Ala Ala Gly Cys Ala Ala Thr Cys Thr Ala Cys Ala Cys Cys Thr
                        115                         120                         125
        Thr Cys Gly Ala Thr Cys Ala Ala Thr Ala Gly Cys Ala Gly Gly Cys
                130                         135                         140
        Gly Thr Gly Gly Cys Gly Cys Gly Cys Cys Ala Gly Cys Cys Ala Thr
        145                         150                         155                         160
        Gly Thr Cys Thr Ala Gly Ala Thr Cys Ala Ala Gly Cys Ala Cys Thr
                        165                         170                         175
        Thr Cys Thr Gly Thr Thr Thr Cys Cys Cys Gly Gly Ala Cys Thr
                180                         185                         190
        Gly Ala Gly Thr Ala Thr Cys Ala Ala Thr Ala Ala Ala Cys Thr Gly
                195                         200                         205
        Cys Thr Cys Ala Cys Gly Cys Gly Gly Thr Thr Gly Ala Ala Gly Gly
                210                         215                         220
        Ala Gly Ala Ala Ala Ala Thr Gly Thr Thr Cys Gly Thr Thr Ala Cys
        225                         230                         235                         240
        Cys Cys Gly Gly Cys Thr Ala Ala Cys Thr Ala Cys Thr Thr Cys Gly
                        245                         250                         255
        Ala Gly Ala Ala Ala Cys Cys Thr Ala Gly Thr Ala Gly Cys Ala Cys
                        260                         265                         270
        Cys Ala Thr Gly Ala Ala Ala Ala Thr Thr Gly Cys Ala Gly Ala Gly
                        275                         280                         285
        Cys Gly Thr Thr Cys Gly Thr Cys Ala Gly Cys Gly Thr Cys Cys Cys
                290                         295                         300
        Cys Gly Cys Gly Ala Gly Ala Thr Cys Ala Gly Gly Cys Thr Gly Ala
        305                         310                         315                         320
        Thr Gly Ala Gly Thr Cys Ala Cys Thr Gly Cys Ala Thr Thr Cys Cys
                        325                         330                         335
        Thr Cys Ala Cys Gly Gly Gly Cys Gly Ala Cys Cys Gly Thr Gly Gly
                340                         345                         350
        Cys Ala Gly Thr Gly Gly Cys Thr Gly Cys Gly Thr Thr Gly Gly Cys
                355                         360                         365
        Gly Gly Cys Cys Thr Gly Cys Cys Cys Ala Thr Gly Gly Gly Gly Thr
                370                         375                         380
        Ala Ala Cys Cys Cys Ala Thr Gly Gly Gly Ala Cys Gly Cys Thr Cys
        385                         390                         395                         400
        Thr Ala Ala Thr Ala Thr Gly Gly Ala Cys Ala Thr Gly Gly Thr Gly
                405                         410                         415
        Thr Gly Ala Ala Gly Ala Gly Thr Cys Thr Ala Thr Thr Gly Ala Gly
                420                         425                         430
        Cys Thr Ala Gly Thr Thr Ala Gly Thr Ala Gly Thr Cys Cys Thr Cys
                435                         440                         445
        Cys Gly Gly Cys Cys Cys Cys Thr Gly Ala Ala Thr Gly Cys Gly Gly
                450                         455                         460
        Cys Thr Ala Ala Thr Cys Cys Thr Ala Ala Cys Thr Gly Cys Gly Gly
        465                         470                         475                         480
        Ala Gly Cys Ala Cys Ala Thr Ala Cys Cys Cys Cys Cys Ala Ala Ala
                        485                         490                         495
        Cys Cys Ala Gly Gly Gly Gly Gly Cys Gly Gly Thr Gly Thr Gly Thr
                500                         505                         510
        Cys Gly Thr Ala Ala Cys Gly Gly Gly Cys Ala Ala Cys Thr Cys Thr
                515                         520                         525
```

```
Gly Cys Ala Gly Cys Gly Gly Ala Ala Cys Cys Gly Ala Cys Thr Ala
    530                 535                 540
Cys Thr Thr Thr Gly Gly Gly Thr Gly Thr Cys Cys Gly Thr Gly Thr
545                 550                 555                 560
Thr Thr Cys Cys Thr Thr Thr Thr Ala Thr Thr Cys Thr Thr Ala Thr
                565                 570                 575
Ala Thr Thr Gly Gly Cys Thr Gly Cys Thr Thr Ala Thr Gly Gly Thr
                580                 585                 590
Gly Ala Cys Ala Ala Thr Thr Gly Ala Ala Ala Gly Ala Thr Thr Gly
                595                 600                 605
Thr Thr Ala Cys Cys Ala Thr Ala Thr Ala Gly Cys Thr Ala Thr Thr
                610                 615                 620
Gly Gly Ala Thr Thr Gly Gly Cys Cys Ala Thr Cys Cys Gly Gly Thr
625                 630                 635                 640
Gly Ala Ala Thr Ala Ala Cys Ala Gly Ala Gly Cys Cys Thr Thr Gly
                645                 650                 655
Ala Thr Ala Thr Ala Cys Cys Thr Thr Thr Thr Thr Gly Thr Ala Gly
                660                 665                 670
Gly Gly Thr Thr Thr Ala Thr Ala Cys Cys Ala Cys Thr Thr Ala Cys
                675                 680                 685
Thr Cys Thr Thr Cys Gly Cys Gly Thr Thr Gly Thr Thr Gly Ala Gly
                690                 695                 700
Ala Cys Thr Cys Thr Ala Ala Ala Gly Thr Ala Cys Ala Thr Thr Cys
705                 710                 715                 720
Thr Ala Ala Thr Cys Thr Thr Gly Ala Ala Cys Ala Cys Thr Ala Gly
                725                 730                 735
Ala Ala Ala
```

<210> 9
<211> 735
<212> PRT
<213> Coxsackievirus A16

<400> 9

```
Ala Gly Cys Cys Thr Gly Thr Gly Gly Gly Thr Thr Gly Thr Thr Cys
1               5                   10                  15
Cys Cys Ala Cys Cys Cys Ala Cys Ala Gly Gly Gly Cys Cys Cys Ala
        20                  25                  30
Gly Thr Gly Gly Gly Cys Gly Cys Thr Ala Gly Cys Ala Cys Ala Cys
        35                  40                  45
Thr Gly Ala Thr Thr Cys Thr Gly Cys Gly Gly Gly Ala Thr Cys Thr
    50                  55                  60
Thr Thr Gly Thr Gly Cys Gly Cys Cys Thr Gly Thr Thr Thr Thr Ala
65                  70                  75                  80
Thr Ala Ala Cys Cys Cys Cys Thr Thr Cys Cys Cys Thr Ala Ala Gly
                85                  90                  95
Cys Ala Gly Cys Ala Ala Cys Thr Thr Ala Gly Ala Ala Gly Thr Thr
        100                 105                 110
Thr Cys Ala Cys Ala Cys Ala Ala Thr Cys Ala Cys Gly Ala Cys Cys
        115                 120                 125
Ala Gly Thr Ala Gly Thr Gly Gly Gly Cys Gly Thr Gly Gly Cys Gly
        130                 135                 140
Cys Gly Cys Cys Ala Gly Thr Cys Ala Cys Gly Thr Cys Thr Thr Gly
145                 150                 155                 160
Gly Thr Cys Ala Ala Gly Cys Ala Cys Thr Thr Cys Thr Gly Thr Thr
        165                 170                 175
Cys Cys Cys Cys Cys Gly Gly Ala Cys Thr Gly Ala Gly Thr Ala Thr
        180                 185                 190
Cys Ala Ala Thr Ala Gly Ala Cys Thr Gly Cys Thr Cys Ala Cys Gly
        195                 200                 205
Cys Gly Gly Thr Thr Gly Ala Ala Gly Gly Ala Gly Ala Ala Ala Ala
```

```
            210                 215                 220
Cys Gly Thr Thr Cys Gly Thr Thr Ala Thr Cys Cys Gly Gly Cys Thr
225                 230                 235                 240
Ala Ala Cys Thr Ala Cys Thr Thr Cys Gly Ala Gly Ala Ala Ala Cys
                245                 250                 255
Cys Thr Ala Gly Ala Gly Cys Ala Cys Cys Gly Thr Gly Ala Ala Ala
                260                 265                 270
Gly Thr Thr Gly Cys Gly Gly Ala Gly Gly Thr Cys Gly Cys Thr Cys
                275                 280                 285
Ala Gly Cys Ala Cys Thr Thr Cys Cys Cys Cys Gly Thr Gly Thr
                290                 295                 300
Ala Gly Ala Thr Cys Ala Gly Gly Thr Cys Gly Ala Thr Gly Ala Gly
305                 310                 315                 320
Thr Cys Ala Cys Thr Gly Thr Ala Ala Ala Cys Cys Cys Cys Ala Cys
                325                 330                 335
Gly Gly Gly Cys Gly Ala Cys Cys Gly Thr Gly Ala Cys Ala Gly Thr
                340                 345                 350
Gly Gly Cys Thr Gly Cys Gly Thr Thr Gly Gly Cys Gly Gly Cys Cys
                355                 360                 365
Thr Gly Cys Cys Cys Ala Thr Gly Gly Gly Gly Thr Ala Ala Cys Cys
370                 375                 380
Cys Ala Thr Gly Gly Gly Ala Cys Gly Cys Thr Cys Thr Ala Ala Thr
385                 390                 395                 400
Ala Cys Ala Gly Ala Cys Ala Thr Gly Gly Thr Gly Thr Gly Ala Ala
                405                 410                 415
Gly Ala Gly Thr Cys Thr Ala Thr Thr Gly Ala Gly Cys Thr Ala Gly
                420                 425                 430
Thr Thr Ala Gly Thr Ala Gly Thr Cys Cys Thr Cys Cys Gly Gly Cys
                435                 440                 445
Cys Cys Cys Thr Gly Ala Ala Thr Gly Cys Gly Gly Cys Thr Ala Ala
                450                 455                 460
Thr Cys Cys Thr Ala Ala Cys Thr Gly Cys Gly Gly Ala Gly Cys Ala
465                 470                 475                 480
Cys Gly Cys Ala Cys Cys Cys Thr Cys Ala Ala Cys Cys Cys Ala Gly
                485                 490                 495
Gly Gly Gly Gly Cys Gly Gly Cys Gly Thr Gly Thr Cys Gly Thr Ala
                500                 505                 510
Ala Thr Gly Gly Gly Thr Ala Ala Cys Thr Cys Thr Gly Cys Ala Gly
                515                 520                 525
Cys Gly Gly Ala Ala Cys Cys Gly Ala Cys Thr Ala Cys Thr Thr Thr
530                 535                 540
Gly Gly Gly Thr Gly Thr Cys Cys Gly Thr Gly Thr Thr Thr Cys Cys
545                 550                 555                 560
Thr Thr Thr Thr Ala Thr Thr Cys Cys Thr Thr Ala Thr Thr Gly Gly
                565                 570                 575
Cys Thr Gly Cys Thr Thr Ala Thr Gly Gly Thr Gly Ala Cys Ala Ala
                580                 585                 590
Thr Thr Gly Ala Ala Ala Ala Gly Thr Thr Gly Thr Thr Ala Cys Cys
                595                 600                 605
Ala Thr Ala Thr Ala Gly Cys Thr Ala Thr Thr Gly Gly Ala Thr Thr
610                 615                 620
Gly Gly Cys Cys Ala Thr Cys Cys Gly Gly Thr Gly Thr Cys Thr Ala
625                 630                 635                 640
Ala Cys Ala Gly Ala Gly Cys Thr Ala Thr Thr Gly Thr Thr Thr Ala
                645                 650                 655
Cys Cys Thr Ala Thr Thr Thr Ala Thr Thr Gly Gly Ala Thr Ala Cys
                660                 665                 670
Gly Thr Cys Cys Cys Thr Cys Thr Thr Ala Ala Thr Cys Thr Cys Ala
                675                 680                 685
Ala Gly Gly Cys Cys Ala Thr Thr Cys Ala Ala Ala Cys Thr Cys Thr
690                 695                 700
Thr Gly Ala Thr Thr Ala Thr Ala Thr Ala Thr Thr Gly Cys Thr Cys
705                 710                 715                 720
```

Cys Thr Thr Ala Ala Cys Thr Gly Thr Ala Ala Gly Ala Ala Ala
725          730          735

<210> 10
<211> 20
<212> DNA
<213> Artificial Sequence

<220>
<223> Sythetic Construct

<400> 10
tccatgacgt tcctgacgtt 20

<210> 11
<211> 18
<212> DNA
<213> Artificial Sequence

<220>
<223> Sythetic Construct

<400> 11
tctcccagcg tgcgccat 18

<210> 12
<211> 30
<212> DNA
<213> Artificial Sequence

<220>
<223> Sythetic Construct

<400> 12
accgatgacg tcgccggtga cggcaccacg 30

<210> 13
<211> 24
<212> DNA
<213> Artificial Sequence

<220>
<223> Sythetic Construct

<400> 13
tcgtcgtttt gtcgttttgt cgtt 24

<210> 14
<211> 20
<212> DNA
<213> Artificial Sequence

<220>
<223> Sythetic Construct

<400> 14
tccatgacgt tcctgatgct 20